# EUROPEAN PATENT APPLICATION

(11) **EP 4 806 905 A1**
(43) Date of publication of application: **16.09.2026**
(21) Application number: 24888811.7
(22) Date of filing: 08.11.2024
(51) Int. Cl.: C07K 7/08, A61K 38/10, A61K 38/12, A61K 47/65, A61P 35/00, A61P 43/00, C07K 1/06, C12N 5/00

(54) **PEPTIDE, PEPTIDE COMPLEX, PHARMACEUTICAL COMPOSITION, COMPOSITION FOR CELL CULTURE, AND COMPOSITION FOR MEDICAL USE, DIAGNOSTIC USE, OR RESEARCH USE**

(30) Priority: 10.11.2023 JP 2023192587
(71) Applicant: PeptiDream Inc., Kawasaki-Shi, Kanagawa 210-0821 (JP)
(72) Inventor: SUZUKI, Yoshinori, Kawasaki-shi, Kanagawa 210-0821 (JP); SHIBATA, Yoshihiro, Kawasaki-shi, Kanagawa 210-0821 (JP); KAMIKUBO, Kenta, Kawasaki-shi, Kanagawa 210-0821 (JP)
(74) Representative: V.O.
(86) International application number: PCT/JP2024/039749
(87) International publication number: WO 2025/100520

(57) **Abstract**

The present invention relates to a peptide complex containing a first peptide and a second peptide. The first peptide contains an amino acid sequence represented by formula A1, or an amino acid sequence in which one or a plurality of amino acid residues have been substituted, deleted, added or inserted within an amino acid sequence represented by formula A1, the second peptide contains an amino acid sequence represented by formula B1, or an amino acid sequence in which one or a plurality of amino acid residues have been substituted, deleted, added or inserted within an amino acid sequence represented by formula B1.

A1: X1-X2-X3-X4-X5-X6-X7-X8-X9-X10-X11-X12-X13-X14

B1: Y1-Y2-Y3-Y4-Y5-Y6-Y7-Y8-Y9-Y10-Y11-Y12-Y13-Y14

## Description

### Technical Field

The present invention relates to a peptide, a peptide complex, a pharmaceutical composition, a composition for cell culture, and a composition for medical use, diagnostic use or research use.

Priority is claimed on Japanese Patent Application No. 2023-192587, filed November 10, 2023, the content of which is incorporated herein by reference.

### Background Art

Wnt is a family of secreted glycoproteins having a molecular weight of approximately 40,000 which control early development and morphogenesis, as well as postnatal proliferation, differentiation, cell motility, and polarity and the like. Nineteen species of Wnt have been identified in humans and mice, and Wnt3a, which represents one species of the Wnt family, binds to the seven-transmembrane Frizzled receptor and the single-transmembrane co-receptor LRP5 or LRP6, thereby activating the Wnt signaling pathway. Chronic activation of this pathway leads to uncontrolled cell proliferation and survival, causing cell proliferation disorders such as cancer, whereas marked inhibition of this pathway leads to bone loss and other bone disorders. Accordingly, many drugs and antibodies that target the Wnt signaling pathway have been reported for treating the aforementioned cancer and bone disorders (see Patent Documents 1 to 9, Non-Patent Documents 1 and 2).

On the other hand, activation of the Wnt signaling pathway has an important role in early development and the maintenance, proliferation, and differentiation of normal tissue stem cells, and Wnt3a in particular is used to create various organoids of the small intestine, large intestine, stomach, pancreas, and lung and the like.

### Citation List

### Patent Documents

Patent Document 1: Japanese Translation of PCT International Application, Publication No. 2022-544308
Patent Document 2: Japanese Translation of PCT International Application, Publication No. 2022-544307
Patent Document 3: Japanese Translation of PCT International Application, Publication No. 2022-551380
Patent Document 4: Japanese Unexamined Patent Application, First Publication No. 2022-137092
Patent Document 5: Japanese Unexamined Patent Application, First Publication No. 2020-178701
Patent Document 6: Japanese Unexamined Patent Application, First Publication No. 2020-063262
Patent Document 7: Japanese Translation of PCT International Application, Publication No. 2013-530929
Patent Document 8: Japanese Patent (Granted) Publication No. 6093692
Patent Document 9: Japanese Unexamined Patent Application, First Publication No. 2009-142274

### Non Patent Documents

Non-Patent Document 1: Shinji Matsumoto et al., "Development of Novel AntiCancer Drugs based on Wnt Signal Research", Ryoiki Yugo Review, 27 December 2018, vol. 7, e009
Non-Patent Document 2: Neiheisel, A. et al., "Wnt pathway modulators in cancer therapeutics: An update on completed and ongoing clinical trials", International Journal of Cancer, March 2022, volume 150, issue 5, pp. 727 to 740

### Summary of Invention

### Problems to be solved by Invention

Wnt3a is a fat-soluble protein, and therefore exhibits low solubility and is unstable in cultures. However, chemically synthesizable Wnt3 surrogate peptides have not yet been proposed.

The present invention has an object of providing a peptide complex that activates the Wnt signaling pathway.

### Means to Solve the Problems

As a result of intensive research aimed at achieving the object described above, the inventors of the present invention discovered that a peptide complex containing a specific peptide was able to activate the Wnt signaling pathway, enabling them to complete the present invention.

In other words, the present invention includes the following aspects.
[1] A peptide containing an amino acid sequence represented by formula A1, or an amino acid sequence in which one or a plurality of amino acid residues have been substituted, deleted, added or inserted within an amino acid sequence represented by formula A1:

   A1: X1-X2-X3-X4-X5-X6-X7-X8-X9-X10-X11-X12-X13-X14

   wherein
   X1 represents any D-amino acid residue,
   X2 and X3 each independently represent any amino acid residue,
   X4 and X5 each independently represent an amino acid residue having an aliphatic hydrocarbon group which may be substituted in the side chain,
   X6 represents an amino acid residue having an aryl group which may be substituted in the side chain,
   X7 represents an N-alkyl amino acid residue having an aryl group which may be substituted or a cycloalkyl group which may be substituted in the side chain,
   X8 represents any amino acid residue,
   X9 represents an L-threonine (T) residue,
   X10 represents any N-alkylated amino acid residue,
   X11 represents any amino acid residue,
   X12 represents an amino acid residue having an aliphatic hydrocarbon group in the side chain,
   X13 represents an amino acid residue having an aliphatic hydrocarbon group which may be substituted in the side chain,
   X14 represents an L-cysteine (C) residue, and
   in formula A1, the amino acid sequence is described from the N-terminal side to the C-terminal side.
[2] The peptide according to [1], wherein in formula A1:
   X1 represents a D-phenylalanine (df) residue, (R)-2-amino-3-(pyridin-4-yl)propanoic acid (d4py) residue, (R)-2-amino-3-(pyridin-3-yl)propanoic acid (d3py) residue, (R)-2-amino-3-(3-methoxyphenyl)propanoic acid (df3OMe) residue, (R)-2-amino-3-(4-methoxyphenyl)propanoic acid (df4OMe) residue, (R)-2-amino-5-ureidopentanoic acid (dicit) residue, D-tyrosine (dy) residue, or (R)-2-amino-3-(4-fluorophenyl)propanoic acid (df4F) residue,
   X2 represents an L-serine (S) residue, L-asparagine (N) residue, L-aspartic acid (D) residue, L-2-aminoadipic acid (Hgl) residue, L-citrulline (Cit) residue, or (S)-2-amino-6-morpholinohexanoic acid (Kmor) residue,
   X3 represents an L-aspartic acid (D) residue, L-arginine (R) residue, L-citrulline (Cit) residue, N6-(4-(carboxymethyl)piperazine-1-carbonyl)-L-lysine (KCOpipzaa) residue, (2*S*)-2-amino-3-(oxan-4-yl)propanoic acid (Atp) residue, L-asparagine (N) residue, or L-serine (S) residue,
   X4 represents an L-isoleucine (I) residue or O-methyl-L-threonine (TMe) residue,
   X5 represents an L-arginine (R) residue, L-glutamic acid (E) residue, (S)-2-aminoheptanoic acid (Ahp) residue, L-serine (S) residue, L-homoserine (Hse) residue, L-citrulline (Cit) residue, N6-carbamoyl-L-lysine (Hcit) residue, (S)-2-amino-4-ureidobutanoic acid (Ncit) residue, or (S)-5-acetamido-2-aminopentanoic acid (OrnAc) residue,
   X6 represents an L-tyrosine (Y) residue, methyl-L-tyrosine (MeY) residue, (S)-2-amino-3-(4-fluorophenyl)propanoic acid (F4F) residue, (S)-2-amino-3-(4-chlorophenyl)propanoic acid (F4C) residue, (S)-2-amino-3-(pyridin-4-yl)propanoic acid (4Py) residue, or (S)-3-(2-amino-2-carboxyethyl)benzoic acid (F3COO) residue,
   X7 represents a methyl-L-phenylalanine (MeF) residue, (S)-2-(methylamino)-3-(pyridin-3-yl)propanoic acid (Me3Py) residue, (S)-2-(methylamino)-3-(pyridin-4-yl)propanoic acid (Me4Py) residue, (S)-3-(2-carboxy-2-(methylamino)ethyl)benzoic acid (MeF3COO) residue, (S)-4-(2-carboxy-2-(methylamino)ethyl)benzoic acid (MeF4COO) residue, or (S)-2-(methylamino)-3-(tetrahydro-2H-pyran-4-yl)propanoic acid (MeAtp) residue,
   X8 represents an L-glutamine (Q) residue, L-valine (V) residue, L-2-aminoadipic acid (Hgl) residue, 1-(carboxymethyl)-L-tryptophan (W1aa) residue, (S)-2-aminoheptanoic acid (Ahp) residue, homo-L-tyrosine (Hty) residue, L-threonine (T) residue, allothreonine (alT) residue, L-citrulline (Cit) residue, N6-(4-(carboxymethyl)piperazine-1-carbonyl) -L-lysine (KCOpipzaa) residue, (S)-2-amino-2-(tetrahydro-2H-pyran-4-yl)acetic acid (Gthp) residue, (S)-2-amino-2-(1-(carboxymethyl)piperidin-4-yl)acetic acid (G4pipaa) residue, (S)-2-amino-2-cyclobutylacetic acid (Cbg) residue, (S)-2-amino-2-cyclohexylacetic acid (Chg) residue, or O-methyl-threonine (TMe) residue,
   X10 represents a methyl-L-phenylalanine (MeF) residue, (S)-2-(methylamino)hexanoic acid (MeNle) residue, (S)-2-(methylamino)-3-(pyridin-3-yl)propanoic acid (Me3Py) residue, (S)-2-(methylamino)-3-(pyridin-4-yl)propanoic acid (Me4Py) residue, (S)-4-(2-carboxy-2-(methylamino)ethyl)benzoic acid (MeF4COO) residue, or (S)-2-(methylamino)-3-(tetrahydro-2H-pyran-4-yl)propanoic acid (MeAtp) residue,
   X11 represents an L-isoleucine (I) residue, L-tyrosine (Y) residue, (S)-2-amino-3-(4-(carboxymethoxy)phenyl)propanoic acid (F4aao) residue, or O-methyl-L-threonine (TMe) residue,
   X12 represents an L-valine (V) residue, methyl-L-valine (MeV) residue, or N-methyl-L-cyclohexylglycine (MeChg) residue, and
   X13 represents a glycine (G) residue, D-alanine (da) residue, or D-serine (ds) residue.
[3] A peptide containing an amino acid sequence represented by formula A2, or an amino acid sequence in which at least one amino acid residue among 1 to 12 amino acid residues selected from the group consisting of the 1st, 2nd, 3rd, 4th, 5th, 6th, 7th, 8th, 10th, 11th, 12th and 13th amino acid residues in an amino acid sequence represented by formula A2 has been substituted, deleted, added or inserted:

   A2: df-S-D-I-R-Y-MeF-Q-T-MeF-I-V-G-C (SEQ ID NO: 164)

   wherein
   df represents a D-phenylalanine residue,
   S represents an L-serine residue,
   D represents an L-aspartic acid residue,
   I represents an L-isoleucine residue,
   R represents an L-arginine residue,
   Y represents an L-tyrosine residue,
   MeF represents a methyl-L-phenylalanine residue,
   Q represents an L-glutamine residue,
   T represents an L-threonine residue,
   V represents an L-valine residue,
   G represents a glycine residue, and
   C represents an L-cysteine residue.
[4] The peptide according to any one of [1] to [3], also having a glycine residue at the C-terminal.
[5] The peptide according to any one of [1] to [3], wherein the peptide is a cyclic peptide.
[6] The peptide according to [4], wherein the peptide is a cyclic peptide
[7] The peptide according to any one of [1] to [6], having a cyclic structure in which an amino acid residue derived from a chloroacetylated amino acid and an L-cysteine residue contained within the peptide are bonded together.
[8] The peptide according to any one of [1] to [7], further containing an additional amino acid residue.
[9] The peptide according to any one of [1] to [8], having binding properties to an Fzd receptor.
[10] The peptide according to [9], having the ability to bind to at least one receptor selected from the group consisting of an Fzd receptor 2, Fzd receptor 7 and Fzd receptor 8.
[11] The peptide according to any one of [1] to [3], wherein the peptide has an amino acid sequence represented by formula A3:

   A3: C1Ac-X1-X2-X3-X4-X5-X6-X7-X8-X9-X10-X11-X12-X13-X14

   wherein
   ClAc represents a chloroacetyl group, X1, X2, X3, X4, X5, X6, X7, X8, X9, X10, X11, X12, X13, and X14 represent the amino acid residues shown in Table 1, and in Table 1,
   df represents a D-phenylalanine residue,
   d4py represents an (R)-2-amino-3-(pyridin-4-yl)propanoic acid residue,
   d3py represents an (R)-2-amino-3-(pyridin-3-yl)propanoic acid residue,
   df3OMe represents an (R)-2-amino-3-(3-methoxyphenyl)propanoic acid residue,
   df4OMe represents an (R)-2-amino-3-(4-methoxyphenyl)propanoic acid residue,
   dcit represents an (R)-2-amino-5-ureidopentanoic acid residue,
   dy represents a D-tyrosine residue,
   df4F represents an (R)-2-amino-3-(4-fluorophenyl)propanoic acid residue,
   S represents an L-serine residue,
   N represents an L-asparagine residue,
   D represents an L-aspartic acid residue,
   Hgl represents an L-2-aminoadipic acid residue,
   Cit represents an L-citrulline residue,
   Kmor represents an (S)-2-amino-6-morpholinohexanoic acid residue,
   R represents an L-arginine residue,
   KCOpipzaa represents an N6-(4-(carboxymethyl)piperazine-1-carbonyl)-L-lysine residue,
   Atp represents a (2S)-2-amino-3-(oxan-4-yl)propanoic acid residue,
   I represents an L-isoleucine residue,
   TMe represents an O-methyl-L-threonine residue,
   E represents an L-glutamic acid residue,
   Ahp represents an (S)-2-aminoheptanoic acid residue,
   Hse represents an L-homoserine residue,
   Hcit represents an N6-carbamoyl-L-lysine residue,
   Ncit represents an (S)-2-amino-4-ureidobutanoic acid residue,
   OrnAc represents an (S)-5-acetamido-2-aminopentanoic acid residue,
   Y represents an L-tyrosine residue,
   MeY represents a methyl-L-tyrosine residue,
   F4F represents an (S)-2-amino-3-(4-fluorophenyl)propanoic acid residue,
   F4C represents an (S)-2-amino-3-(4-chlorophenyl)propanoic acid residue,
   4Py represents an (S)-2-amino-3-(pyridin-4-yl)propanoic acid residue,
   F3COO represents an (S)-3-(2-amino-2-carboxyethyl)benzoic acid residue,
   MeF represents a methyl-L-phenylalanine residue,
   Me3Py represents an (S)-2-(methylamino)-3-(pyridin-3-yl)propanoic acid residue,
   Me4Py represents an (S)-2-(methylamino)-3-(pyridin-4-yl) ) propanoic acid residue,
   MeF3COO represents an (S)-3-(2-carboxy-2-(methylamino)ethyl)benzoic acid residue,
   MeF4COO represents an (S)-4-(2-carboxy-2-(methylamino)ethyl)benzoic acid residue,
   MeAtp represents an (S)-2-(methylamino)-3-(tetrahydro-2H-pyran-4-yl)propanoic acid residue,
   Q represents an L-glutamine residue,
   V represents an L-valine residue,
   W1aa represents a 1-(carboxymethyl)-L-tryptophan residue,
   Hty represents a homo-L-tyrosine residue,
   T represents an L-threonine residue,
   alT represents an allothreonine residue,
   Gthp represents an (S)-2-amino-2-(tetrahydro-2H-pyran-4-yl)acetic acid residue,
   G4pipaa represents an (S)-2-amino-2-(1-(carboxymethyl)piperidin-4-yl)acetic acid residue,
   Cbg represents an (S)-2-amino-2-cyclobutylacetic acid residue,
   Chg represents an (S)-2-amino-2-cyclohexylacetic acid residue,
   MeNle represents an (S)-2-(methylamino)hexanoic acid residue,
   F4aao represents an (S)-2-amino-3-(4-(carboxymethoxy)phenyl)propanoic acid residue,
   MeV represents a methyl-L-valine residue,
   MeChg represents an N-methyl-L-cyclohexylglycine residue,
   G represents a glycine residue, and
   C represents a L-cysteine residue.
[12] A peptide containing an amino acid sequence represented by formula B1, or an amino acid sequence in which one or a plurality of amino acid residues have been substituted, deleted, added or inserted within an amino acid sequence represented by formula B1:

   B1: Y1-Y2-Y3-Y4-Y5-Y6-Y7-Y8-Y9-Y10-Y11-Y12-Y13-Y14

   wherein
   Y1 represents an L-phenylalanine (F) residue which may be substituted,
   Y2 represents an L-arginine (R) residue which may be substituted,
   Y3 represents an L-tryptophan (W) residue which may be substituted or an (S)-2-amino-3-(naphthalen-1-yl)propanoic acid (Nal1) residue which may be substituted,
   Y4 represents an acidic amino acid residue or a neutral amino acid residue,
   Y5 represents an aromatic amino acid residue which may be substituted, an aliphatic amino acid residue, or an L-proline (P) residue,
   Y6 represents an aliphatic amino acid residue which may be substituted,
   Y7 represents an N-alkylated aromatic amino acid residue,
   Y8 represents an amino acid residue derived from an amino acid having a hydroxy group, carboxy group or amide group in the side chain,
   Y9 represents any amino acid residue,
   Y10 represents an L-tryptophan (W) residue which may be substituted,
   Y11 represents an L-asparagine (N) residue which may be substituted or an L-glutamine (Q) residue,
   Y12 represents an (S)-1,2,3,4-tetrahydroisoquinoline-3-carboxylic acid (Tic) residue which may be substituted,
   Y13 represents a neutral amino acid residue or acidic amino acid residue which may be substituted,
   Y14 represents an L-cysteine (C) residue, and
   in formula B1, the amino acid sequence is described from the N-terminal side to the C-terminal side.
[13] The peptide according to [12], wherein in formula B1:
   Y1 represents an L-phenylalanine (F) residue, (S)-2-amino-3-(4-fluorophenyl)propanoic acid (F4F) residue, (S)-2-amino-3-(p-toluyl)propanoic acid (F4Me) residue, or (S)-2-amino-3-(m-toluyl)propanoic acid (F3Me) residue,
   Y2 represents an L-arginine (R) residue or N6-carbamoyl-L-lysine (Heit) residue,
   Y3 represents an L-tryptophan (W) residue, methyl-L-tryptophan (MeW) residue, (S)-2-amino-3-(1H-pyrrolo[2,3-b]pyridin-3-yl)propanoic acid (W7N) residue, (S)-2-amino-3-(1H-pyrrolo[3,2-c]pyridin-3-yl)propanoic acid (W5N) residue, (S)-2-amino-3-(1-methyl-1H-pyrrolo[2,3-b]pyridin-3-yl)propanoic acid (W1Me7N) residue, (S)-2-amino-3-(5-methyl-1H-pyrrolo[2,3-b]pyridin-3-yl)propanoic acid (W7N5Me) residue, (S)-2-amino-3-(naphthalen-1-yl)propanoic acid (Nal1) residue, or (S)-2-amino-3-(quinolin-4-yl)propanoic acid (Nal14N) residue,
   Y4 represents an L-aspartic acid (D) residue, L-glutamic acid (E) residue, L-asparagine (N) residue, or L-glutamine (Q) residue,
   Y5 represents an L-valine (V) residue, methyl-L-valine (MeV) residue, 1-(carboxymethyl)-L-tryptophan (W1aa) residue, (S)-2-amino-3-cyclohexylpropanoic acid (Cha) residue, (S)-2-amino-3-(naphthalen-1-yl)propanoic acid (Nal1) residue, (S)-2-amino-3-(5-phenylpyridin-3-yl)propanoic acid (3Py5Ph) residue, (S)-2-amino-2-cyclobutylacetic acid (Cbg) residue, or L-proline (P) residue,
   Y6 represents an L-arginine (R) residue, methyl-L-arginine (MeR) residue, (S)-2-amino-6-morpholinohexanoic acid (Kmor) residue, L-citrulline (Cit) residue, N6-carbamoyl-L-lysine (Hcit) residue, (S)-2-amino-4-ureidobutanoic acid (Ncit) residue, or (S)-5-acetamido-2-aminopentanoic acid (OrnAc) residue,
   Y7 represents a methyl-L-phenylalanine (MeF) residue, (S)-4-(2-carboxy-2-(methylamino)ethyl)benzoic acid (MeF4COO) residue, methyl-L-tyrosine (MeY) residue, (S)-2-(methylamino)-3-(p-toluyl)propanoic acid (MeF4Me) residue, (S)-3-(4-chlorophenyl)-2-(methylamino)propanoic acid (MeF4C) residue, (S)-3-(2-carboxy-2-(methylamino)ethyl)benzoic acid (MeF3COO) residue, (S)-2-(methylamino)-3-(m-toluyl)propanoic acid (MeF3Me) residue, or (S)-3-(3-chlorophenyl)-2-(methylamino)propanoic acid (MeF3C) residue,
   Y8 represents an L-serine (S) residue, 1-(carboxymethyl)-L-tryptophan (W1aa) residue, homo-L-tyrosine (Hty) residue, allothreonine (alT) residue, L-threonine (T) residue, L-asparagine (N) residue, L-glutamine (Q) residue, L-aspartic acid (D) residue, or L-glutamic acid (E) residue,
   Y9 represents an L-asparagine (N) residue, methyl-L-asparagine (MeN) residue, L-2-aminoadipic acid (Hgl) residue, (S)-2-amino-3-cyclohexylpropanoic acid (Cha) residue, homo-L-tyrosine (Hty) residue, (S)-2-amino-4-(1H-pyrrolo[2,3-b]pyridin-3-yl)butanoic acid (Hw7N) residue, N6-(4-(carboxymethyl)piperazine-1-carbonyl)-L-lysine (KCOpipzaa) residue, L-glutamine (Q) residue, L-aspartic acid (D) residue, or L-glutamic acid (E) residue,
   Y10 represents an L-tryptophan (W) residue, (S)-2-amino-3-(1H-pyrrolo[2,3-b]pyridin-3-yl)propanoic acid (W7N) residue, (S)-2-amino-3-(1H-pyrrolo[2,3-c]pyridin-3-yl)propanoic acid (W6N) residue, (S)-2-amino-3-(1-methyl-1H-pyrrolo[2,3-b]pyridin-3-yl)propanoic acid (W1Me7N) residue, or (S)-2-amino-3-(5-methyl-1H-pyrrolo[2,3-b]pyridin-3-yl)propanoic acid (W7N5Me) residue,
   Y11 represents an L-asparagine (N) residue, L-glutamine (Q) residue, or (2S)-2-amino-4-(methylamino)-4-oxobutanoic acid (Nmm) residue,
   Y12 represents an (S)-1,2,3,4-tetrahydroisoquinoline-3-carboxylic acid (Tic) residue, (S)-6-chloro-1,2,3,4-tetrahydroisoquinoline-3-carboxylic acid (Tic6C) residue, (S)-7-hydroxy-1,2,3,4-tetrahydroisoquinoline-3-carboxylic acid (Tic7H) residue, or (S)-7-chloro-1,2,3,4-tetrahydroisoquinoline-3-carboxylic acid (Tic7C) residue, and
   Y13 represents an L-asparagine (N) residue, L-aspartic acid (D) residue, L-glutamine (Q) residue, (2S)-2-amino-4-(methylamino)-4-oxobutanoic acid (Nmm) residue, or N4,N4-dimethyl-L-asparagine (Ndm) residue.
[14] A peptide containing an amino acid sequence represented by formula B2, or an amino acid sequence in which at least one amino acid residue among 1 to 13 amino acid residues selected from the group consisting of the 1st, 2nd, 3rd, 4th, 5th, 6th, 7th, 8th, 9th, 10th, 11th, 12th and 13th amino acid residues in an amino acid sequence represented by formula B2 has been substituted, added, deleted or inserted:

   B2: F-R-W-D-V-R-MeF-S-N-W-N-Tic-N-C (SEQ ID NO: 165)

   wherein
   F represents an L-phenylalanine residue,
   R represents an L-arginine residue,
   W represents an L-tryptophan residue,
   D represents an L-aspartic acid residue,
   V represents an L-valine residue,
   MeF represents a methyl-L-phenylalanine residue,
   S represents an L-serine residue,
   Tic represents an (S)-1,2,3,4-tetrahydroisoquinoline-3-carboxylic acid residue,
   N represents an L-asparagine residue, and
   C represents an L-cysteine residue.
[15] The peptide according to any one of [12] to [14], further having a glycine residue at the C-terminal.
[16] The peptide according to any one of [12] to [14], wherein the peptide is a cyclic peptide.
[17] The peptide according to [15], wherein the peptide is a cyclic peptide
[18] The peptide according to any one of [12] to [17], having a cyclic structure in which an amino acid residue derived from a chloroacetylated amino acid and an L-cysteine residue contained within the peptide are bonded together.
[19] The peptide according to any one of [12] to [18], further containing an additional amino acid residue.
[20] The peptide according to any one of [12] to [19], having binding properties to LRP.
[21] The peptide according to [20], having the ability to bind to at least one receptor selected from the group consisting of LRP5 and LRP6.
[22] The peptide according to any one of [12] to [19], having DKK1-like activity.
[23] The peptide according to any one of [12] to [14], wherein the peptide has an amino acid sequence represented by formula B3:

   B3: ClAc-Y1-Y2-Y3-Y4-Y5-Y6-Y7-Y8-Y9-Y10-Y11-Y12-Y13-Y14

   wherein
   ClAc represents a chloroacetyl group, Y1, Y2, Y3, Y4, Y5, Y6, Y7, Y8, Y9, Y10, Y11, Y12, Y13 and Y14 represent the amino acid residues shown in Table 2, and in Table 2,
   F represents an L-phenylalanine residue,
   F4F represents an (S)-2-amino-3-(4-fluorophenyl)propanoic acid residue,
   F4Me represents an (S)-2-amino-3-(p-toluyl)propanoic acid residue,
   F3Me represents an (S)-2-amino-3-(m-toluyl)propanoic acid residue,
   R represents an L-arginine residue,
   Hcit represents an N6-carbamoyl-L-lysine residue,
   W represents an L-tryptophan residue,
   MeW represents a methyl-L-tryptophan residue,
   W7N represents an (S)-2-amino-3-(1H-pyrrolo[2,3-b]pyridin-3-yl)propanoic acid residue,
   W5N represents an (S)-2-amino- 3-(1H-pyrrolo[3,2-c]pyridin-3-yl)propanoic acid residue,
   W1Me7N represents an (S)-2-amino-3-(1-methyl-1H-pyrrolo[2,3-b]pyridin-3-yl)propanoic acid residue,
   W7N5Me represents an (S)-2-amino-3-(5-methyl-1H-pyrrolo[2,3-b]pyridin-3-yl)propanoic acid residue
   Nal1 represents an (S)-2-amino-3-(naphthalen-1-yl)propanoic acid residue,
   Nal14N represents an (S)-2-amino-3-(quinolin-4-yl)propanoic acid residue,
   D represents an L-aspartic acid residue,
   E represents an L-glutamic acid residue,
   N represents an L-asparagine residue,
   Q represents an L-glutamine residue,
   V represents an L-valine residue,
   MeV represents a methyl-L-valine residue,
   W1aa represents a 1-(carboxymethyl)-L-tryptophan residue,
   Cha represents an (S)-2-amino-3-cyclohexylpropanoic acid residue,
   3Py5Ph represents an (S)-2-amino-3-(5-phenylpyridin-3-yl)propanoic acid residue,
   Cbg represents an (S)-2-amino-2-cyclobutylacetic acid residue,
   P represents an L-proline residue,
   MeR represents a methyl-L-arginine residue,
   Kmor represents an (S)-2-amino-6-morpholinohexanoic acid residue,
   Cit represents an L-citrulline residue,
   Ncit represents an (S)-2-amino-4-ureidobutanoic acid residue,
   OrnAc represents an (S)-5-acetamido-2-aminopentanoic acid residue,
   MeF represents a methyl-L-phenylalanine residue,
   MeF4COO represents an (S)-4-(2-carboxy-2-(methylamino)ethyl)benzoic acid residue,
   MeY represents a methyl-L-tyrosine residue,
   MeF4Me represents an (S)-2-(methylamino)-3-(p-toluyl)propanoic acid residue,
   MeF4C represents an (S)-3-(4-chlorophenyl)-2-(methylamino)propanoic acid residue,
   MeF3COO represents an (S)-3-(2-carboxy-2-(methylamino)ethyl)benzoic acid residue,
   MeF3Me represents an (S)-2-(methylamino)-3-(m-toluyl)propanoic acid residue,
   MeF3C represents an (S)-3-(3-chlorophenyl)-2-(methylamino)propanoic acid residue,
   S represents an L-serine residue,
   Hty represents a homo-L-tyrosine residue,
   alT represents an allothreonine residue,
   T represents an L-threonine residue,
   MeN represents a methyl-L-asparagine residue,
   Hgl represents an L-2-aminoadipic acid residue,
   Hw7N represents an (S)-2-amino-4-(1H-pyrrolo[2,3-b]pyridin-3-yl)butanoic acid residue,
   KCOpipzaa represents an N6-(4-(carboxymethyl)piperazine-1-carbonyl)-L-lysine residue,
   W6N represents an (S)-2-amino-3-(1H-pyrrolo[2,3-c]pyridin-3-yl)propanoic acid residue,
   Nmm represents a (2S)-2-amino-4-(methylamino)-4-oxobutanoic acid residue,
   Tic represents an (S)-1,2,3,4-tetrahydroisoquinoline-3-carboxylic acid residue,
   Tic6C represents an (S)-6-chloro-1,2,3,4-tetrahydroisoquinoline-3-carboxylic acid residue,
   Tic7H represents an (S)-7-hydroxy-1,2,3,4-tetrahydroisoquinoline-3-carboxylic acid residue,
   Tic7C represents an (S)-7-chloro-1,2,3,4-tetrahydroisoquinoline-3-carboxylic acid residue,
   Ndm represents an N4,N4-dimethyl-L-asparagine residue,
   C represents an L-cysteine residue, and
   G represents a glycine residue.
[24] A peptide complex containing a first peptide and a second peptide, wherein
   the first peptide is the peptide according to any one of [1] to [11], and
   the second peptide is the peptide according to any one of [12] to [23].
[25] The peptide complex according to [24], wherein the peptide complex is composed of the first peptide, the second peptide, and a linker connecting the first peptide and the second peptide.
[26] The peptide complex according to [25], wherein the C-terminal of the first peptide and the C-terminal of the second peptide are bonded via the linker.
[27] The peptide complex according to [25], wherein the linker is selected from among the linkers shown in Table 3, and
   in Table 3, -click- represents a bond formed by click chemistry.
[28] The peptide complex according to any one of [24] to [27], wherein
   the first peptide is a peptide containing an amino acid sequence represented by formula A1, and
   the second peptide is a peptide containing an amino acid sequence represented by formula B1:

      A1: X1-X2-X3-X4-X5-X6-X7-X8-X9-X10-X11-X12-X13-X14

      B1: Y1-Y2-Y3-Y4-Y5-Y6-Y7-Y8-Y9-Y10-Y11-Y12-Y13-Y14
   wherein in formula A1:
   X1 represents a D-phenylalanine (df) residue,
   X2 represents an L-serine (S) residue or an L-2-aminoadipic acid (Hgl) residue,
   X3 represents an L-aspartic acid (D) residue, L-citrulline (Cit) residue, or (2S)-2-amino-3-(oxan-4-yl)propanoic acid (Atp) residue,
   X4 represents an L-isoleucine (I) residue,
   X5 represents an L-arginine (R) residue or an L-citrulline (Cit) residue,
   X6 represents an L-tyrosine (Y) residue,
   X7 represents a methyl-L-phenylalanine (MeF) residue or an (S)-3-(2-carboxy-2-(methylamino)ethyl)benzoic acid (MeF3COO) residue,
   X8 represents an L-glutamine (Q) residue, L-valine (V) residue, homo-L-tyrosine (Hty) residue, L-threonine (T) residue, L-citrulline (Cit) residue, or O-methyl-L-threonine (TMe) residue,
   X10 represents a methyl-L-phenylalanine (MeF) residue or an (S)-2-(methylamino)-3-(pyridin-4-yl)propanoic acid (Me4Py) residue,
   X11 represents an L-isoleucine (I) residue or an L-tyrosine (Y) residue,
   X12 represents an L-valine (V) residue or a methyl-L-valine (MeV) residue, and
   X13 represents a glycine (G) residue or a D-serine (ds) residue, and
   in formula B1:
   Y1 represents an L-phenylalanine (F) residue,
   Y2 represents an L-arginine (R) residue,
   Y3 represents an L-tryptophan (W) residue or an (S)-2-amino-3-(1H-pyrrolo[2,3-b]pyridin-3-yl)propanoic acid (W7N) residue,
   Y4 represents an L-aspartic acid (D) residue,
   Y5 represents an L-valine (V) residue,
   Y6 represents an L-arginine (R) residue or an (S)-2-amino-6-morpholinohexanoic acid (Kmor) residue,
   Y7 represents a methyl-L-phenylalanine (MeF) residue or an (S)-3-(2-carboxy-2-(methylamino)ethyl)benzoic acid (MeF3COO) residue,
   Y8 represents an L-serine (S) residue or an L-glutamic acid (E) residue,
   Y9 represents an L-asparagine (N) residue, N6-(4-(carboxymethyl)piperazine-1-carbonyl)-L-lysine (KCOpipzaa) residue, or L-glutamic acid (E) residue,
   Y10 represents an L-tryptophan (W) residue or an (S)-2-amino-3-(1H-pyrrolo[2,3-b]pyridin-3-yl)propanoic acid (W7N) residue,
   Y11 represents an L-asparagine (N) residue,
   Y12 represents an (S)-1,2,3,4-tetrahydroisoquinoline-3-carboxylic acid (Tic) residue, and
   Y13 represents an L-asparagine (N) residue or an L-aspartic acid (D) residue.
[29] The peptide complex according to any one of [24] to [28], wherein the peptide complex activates Wnt signaling.
[30] A pharmaceutical composition, containing the peptide according to any one of [1] to [23].
[31] A pharmaceutical composition, containing the peptide complex according to any one of [24] to [29].
[32] A composition for cell culture, containing the peptide complex according to any one of [24] to [29], and used for cell culture.
[33] A composition for medical use, diagnostic use or research use, containing the peptide complex according to any one of [24] to [29].
[34] A composition for medical use, diagnostic use or research use, containing the peptide according to any one of [1] to [11] and [12] to [23].
[35] The peptide according to any one of [1] to [23], for use in the treatment or prevention of a cancer disease such as breast cancer, colon cancer, lung cancer or stomach cancer, or inflammatory bowel disease.
[36] The peptide complex according to any one of [24] to [29], for use in the treatment or prevention of a bone metabolic disease such as osteoporosis.
[37] Use of the peptide according to any one of [1] to [23] for producing a pharmaceutical composition for the treatment or prevention of a cancer disease such as breast cancer, colon cancer, lung cancer or stomach cancer, or inflammatory bowel disease.
[38] Use of the peptide complex according to any one of [24] to [29] for producing a pharmaceutical composition for the treatment or prevention of a bone metabolic disease such as osteoporosis.
[39] A method for treating or preventing a cancer disease such as breast cancer, colon cancer, lung cancer or stomach cancer, or inflammatory bowel disease, the method including administering an effective amount of the peptide according to any one of [1] to [23] to a subject in need thereof.
[40] A method for treating or preventing a bone metabolic disease such as osteoporosis, the method including administering an effective amount of the peptide complex according to any one of [24] to [29] to a subject in need thereof.
[41] Use of the peptide complex according to any one of [24] to [29] for creating an organoid of the small intestine, large intestine, stomach, pancreas, or lung or the like.

### Effects of Invention

The present invention provides a peptide complex that activates the Wnt signaling pathway.

### Description of Embodiments

Embodiments of the peptide, the peptide complex, the pharmaceutical composition, the composition for cell culture, and the composition for medical use, diagnostic use or research use according to the present invention are described below in detail. However, the present invention is not limited to the following embodiments, and various modifications are possible without departing from the scope of the present invention.

### [Abbreviations (General)]

Å: angstrom (unit, 10⁻¹⁰ m)
AcOH: acetic acid
BSA: bovine serum albumin
Boc: tert-butoxycarbonyl group
ClAc: chloroacetyl
ClAcOSu: (2,5-dioxopyrrolidin-1-yl) 2-chloroacetate (CAS RN: 27243-15-8)
[Cu(CH₃CN)_{4]}PF₆: tetrakis(acetonitrile)copper(I) hexafluorophosphate (CAS RN: 64443-05-6)
DCM: dichloromethane or methylene chloride
DIPCI or DIC: N,N'-diisopropylcarbodiimide
DIPEA or DIEA: N,N-diisopropylethylamine
DMA: N,N-dimethylacetamide
Dmb: dimethoxybenzyl group
DMEM: Dulbecco's modified Eagle medium
DMF: N,N-dimethylformamide
DMSO: dimethyl sulfoxide
DODT: 3,6-dioxa-1,8-octanedithiol
EC50: 50% effective concentration
FBS: fetal bovine serum
Fmoc: 9-fluorenylmethyloxycarbonyl
Fmoc-Lys(Fmoc)-OH: N²,N⁶-bis(((9H-fluoren-9-yl)methoxy)carbonyl)-L-lysine
g: gram (unit)
HATU: O-(7-azabenzotriazol-1-yl)-N,N,N',N'-tetramethyluronium hexafluorophosphate
HPLC: high-performance liquid chromatography
LC-MS or LC/MS: liquid chromatography mass spectrometry
M: molar (unit)
MeCN: acetonitrile
mg: milligram (unit)
min: minute (unit)
mL: milliliter (unit)
mM: millimolar (unit)
mm: millimeter (unit)
Mpe group: 3-methyl-pent-3-yl group
NHS: N-hydroxysuccinimide
nm: nanometer (unit)
µL: microliter (unit)
OSu: oxysuccinimide
Pbf: 2,2,4,6,7-pentamethyldihydrobenzofuran-5-sulfonyl group
PEG: polyethylene glycol
rpm: revolutions per minute (unit)
Sub: dibenzosuberyl group
TBTA: tris[(1-benzyl-1H-1,2,3-triazol-4-yl)methyl]amine (CAS RN: 510758-28-8)
tBu: tert-butyl group
TEAA: triethylamine acetate
TFA: trifluoroacetic acid
TIS: triisopropylsilane
Trt or Tr: trityl group

### [Abbreviations (Unnatural Amino Acids)]

3Py5Ph: (S)-2-amino-3-(5-phenylpyridin-3-yl)propanoic acid (CAS RN: 2973752-28-0)
3Py6NH2: (S)-2-amino-3-(6-aminopyridin-3-yl)propanoic acid (CAS RN: 1269968-61-7)
4Py: (S)-2-amino-3-(pyridin-4-yl)propanoic acid (CAS RN: 37535-49-2)
Ahp: (S)-2-aminoheptanoic acid (CAS RN: 44902-02-5)
alT: allothreonine (CAS RN: 28954-12-3)
Atb: (S)-2-amino-4,4-dimethylpentanoic acid (CAS RN: 57224-50-7)
Atp: (2S)-2-amino-3-(oxan-4-yl)propanoic acid (CAS RN: 1344910-91-3)
AZ(L08)-P12-PS: 2,5-dioxopyrrolidin-1-yl 40-(33-azido-29-oxo-4,7,10,13,16,19,22,25-octaoxa-28-azatritriacontanamide)-40-(2,5,8,11,14,17,20,23,26,29,32,35,38-tridecaoxanonatriacontyl)-43-oxo-2,5,8,11,14,17,20,23,26, 29,32,35,38,42-tetradecaoxa-44-azaheptatetracontane-47-oate (NOF Corporation)
Cbg: (S)-2-amino-2-cyclobutylacetic acid (CAS RN: 49607-08-1)
Cha: (S)-2-amino-3-cyclohexylpropanoic acid (CAS RN: 27527-05-5)
Chg: (S)-2-amino-2-cyclohexylacetic acid (CAS RN: 14328-51-9)
Cit: L-citrulline (CAS RN: 372-75-8)
cPEG9c: 4,7,10,13,16,19,22,25,28-nonaoxahentriacontanedioic acid (CAS RN: 1268488-70-5)
cPEG13c: 4,7,10,13,16,19,22,25,28,31,34,37,40-tridecaoxatritetracontanedioic acid (CAS RN: 2225903-66-0)
cPEG17c: 4,7,10,13,16,19,22,25,28,31,34,37,40,43,46,49,52-heptadecaoxapentapentacontanedioic acid (CAS RN: 2226897-74-9)
d3py: (R)-2-amino-3-(pyridin-3-yl)propanoic acid (CAS RN: 70702-47-5)
d4py: (R)-2-amino-3-(pyridin-4-yl)propanoic acid (CAS RN: 37535-50-5)
da: D-alanine (CAS RN: 338-69-2)
dcit: (R)-2-amino-5-ureidopentanoic acid (CAS RN: 13594-51-9)
df: D-phenylalanine (CAS RN: 673-06-3)
df3OMe: (R)-2-amino-3-(3-methoxyphenyl)propanoic acid (CAS RN: 145306-65-6)
df4F: (R)-2-amino-3-(4-fluorophenyl)propanoic acid (CAS RN: 18125-46-7)
df4OMe: (R)-2-amino-3-(4-methoxyphenyl)propanoic acid (CAS RN: 39878-65-4)
dkCOpipzaa: N6-(4-(carboxymethyl)piperazine-1-carbonyl)-D-lysine (CAS RN: 2973757-42-3)
ds: D-serine (CAS RN: 312-84-5)
dy: D-tyrosine (CAS RN: 556-02-5)
F3COO: (S)-3-(2-amino-2-carboxyethyl)benzoic acid (CAS RN: 13861-02-4)
F3Me: (S)-2-amino-3-(m-toluyl)propanoic acid (CAS RN: 114926-37-3)
F4aao: (S)-2-amino-3-(4-(carboxymethoxy)phenyl)propanoic acid (CAS RN: 24558-63-2)
F4C: (S)-2-amino-3-(4-chlorophenyl)propanoic acid (CAS RN: 14173-39-8)
F4F: (S)-2-amino-3-(4-fluorophenyl)propanoic acid (CAS RN: 1132-68-9)
F4Me: (S)-2-amino-3-(p-toluyl)propanoic acid (CAS RN: 1991-87-3)
G4pipaa: (S)-2-amino-2-(1-(carboxymethyl)piperidin-4-yl)acetic acid (CAS RN: 2973756-12-4)
Gpra: (S)-2-aminopent-4-ynoic acid (CAS RN: 23235-01-0)
Gthp: (S)-2-amino-2-(tetrahydro-2H-pyran-4-yl)acetic acid (CAS RN: 811842-25-8)
Hcit: N6-carbamoyl-L-lysine (CAS RN: 1190-49-4)
Hgl: L-2-aminoadipic acid (CAS RN: 1118-90-7)
Hse: L-homoserine (CAS RN: 672-15-1)
Hty: Homo-L-tyrosine (CAS RN: 221243-01-2)
Hw7N: (S)-2-amino-4-(1H-pyrrolo[2,3-b]pyridin-3-yl)butanoic acid (CAS RN: 2349860-05-3)
KCOpipzaa: N6-(4-(carboxymethyl)piperazine-1-carbonyl)-L-lysine (CAS RN: 2913141-21-4)
Kmor: (S)-2-amino-6-morpholinohexanoic acid (CAS RN: 960135-14-2)
KN3: N6-diazo-L-lysine (CAS RN: 159610-92-1)
Me3Py: (S)-2-(methylamino)-3-(pyridin-3-yl)propanoic acid (CAS RN: 2651172-69-7)
Me4Py: (S)-2-(methylamino)-3-(pyridin-4-yl)propanoic acid (CAS RN: 2913141-25-8)
MeAtp: (S)-2-(methylamino)-3-(tetrahydro-2H-pyran-4-yl)propanoic acid (CAS RN: 2973752-56-4)
MeChg: (S)-2-cyclohexyl-2-(methylamino)acetic acid (CAS RN: 145963-18-4)
MeF: methyl-L-phenylalanine (CAS RN: 2566-30-5)
MeF3C: (S)-3-(3-chlorophenyl)-2-(methylamino)propanoic acid (CAS RN: 2255324-91-3)
MeF3COO: (S)-3-(2-carboxy-2-(methylamino)ethyl)benzoic acid (CAS RN: 2913141-26-9)
MeF3Me: (S)-2-(methylamino)-3-(m-toluyl)propanoic acid
MeF4C: (S)-3-(4-chlorophenyl)-2-(methylamino)propanoic acid (CAS RN: 347851-70-1)
MeF4COO: (S)-4-(2-carboxy-2-(methylamino)ethyl)benzoic acid (CAS RN: 2973752-03-1)
MeF4Me: (S)-2-(methylamino)-3-(p-toluyl)propanoic acid (CAS RN: 2307782-25-6)
MeN: methyl-L-asparagine (CAS RN: 19026-58-5)
MeNle: (S)-2-(methylamino)hexanoic acid (CAS RN: 17343-27-0)
MeR: methyl-L-arginine (CAS RN: 2480-28-6)
MeV: methyl-L-valine (CAS RN: 2480-23-1)
MeW: methyl-L-tryptophan (CAS RN: 526-31-8)
MeY: methyl-L-tyrosine (CAS RN: 537-49-5)
Nal1: (S)-2-amino-3-(naphthalen-1-yl)propanoic acid (CAS RN: 55516-54-6)
Nal14N: (S)-2-amino-3-(quinolin-4-yl)propanoic acid (CAS RN: 137855-78-8)
Ncit: (S)-2-amino-4-ureidobutanoic acid (CAS RN: 1190-47-2)
Nmm: (2S)-2-amino-4-(methylamino)-4-oxobutanoic acid (CAS RN: 7175-34-0)
Ndm: (2S)-2-amino-4-(dimethylamino)-4-oxobutanoic acid (CAS RN: 62937-43-3)
OrnAc: (S)-5-acetamido-2-aminopentanoic acid (CAS RN: 2185-16-2)
PEG4c: 1-amino-3,6,9,12-tetraoxapentadecan-15-oic acid (CAS RN: 663921-15-1)
PEG8c: 1-amino-3,6,9,12,15,18,21,24-octaoxaheptacosan-27-oic acid (CAS RN: 756526-04-2)
PEG12c: 1-amino-3,6 ,9,12,15,18,21,24,27,30,33,36-dodecaoxanonatriacontan-39-oic acid (CAS RN: 1415408-69-3)
SMe: O-Methyl-L-serine (CAS RN: 32620-11-4)
Tbg: (S)-2-amino-3,3-dimethylbutanoic acid (CAS RN: 20859-02-3)
Tic: (S)-1,2,3,4-tetrahydroisoquinoline-3-carboxylic acid (CAS RN: 74163-81-8)
Tic6C: (S)-6-chloro-1,2,3,4-tetrahydroisoquinoline-3-carboxylic acid (CAS RN: 1344407-69-7)
Tic7C: (S)-7-chloro-1,2,3,4-tetrahydroisoquinoline-3-carboxylic acid (CAS RN: 1344492-44-9)
Tic7H: (S)-7-hydroxy-1,2,3,4-tetrahydroisoquinoline-3-carboxylic acid (CAS RN: 128502-56-7)
TMe: O-methyl-L-threonine (CAS RN: 4144-02-9)
W1aa: 1-(carboxymethyl)-L-tryptophan (CAS RN: 773823-50-0)
W1Me7N: (S)-2-amino-3-(1-methyl-1H-pyrrolo[2,3-b]pyridin-3-yl)propanoic acid (CAS RN: 1632971-18-6)
W5N: (S)-2-amino-3-(1H-pyrrolo[3,2-c]pyridin-3-yl)propanoic acid (CAS RN: 149704-62-1)
W6N: (S)-2-amino-3-(1H-pyrrolo[2,3-c]pyridin-3-yl)propanoic acid (CAS RN: 149704-63-2)
W7N: (S)-2-amino-3-(1H-pyrrolo[2,3-b]pyridin-3-yl)propanoic acid (CAS RN: 49758-35-2)
W7N5Me: (S)-2-amino-3-(5-methyl-1H-pyrrolo[2,3-b]pyridin-3-yl)propanoic acid (CAS RN: 1270158-20-7)

### [Wnt]

Wnt is a family of secreted glycoproteins having a molecular weight of approximately 40,000 which control early development and morphogenesis, as well as postnatal proliferation, differentiation, cell motility, and polarity and the like. Nineteen species of Wnt (Wnt1 to 19) have been identified in humans and mice.

Wnt binds to members of the Frizzled (Fzd) receptor family and to the co-receptors LRP5 or LRP6, ROR or RYK, thereby activating the Wnt signaling pathway.

The peptide complex of the present invention binds to Frizzled receptors (Fzd2, Fzd7, Fzd8) and/or LRPs (LRP5, LRP6), thereby activating the Wnt signaling pathway, and can therefore be used as an alternative to the naturally occurring Wnt family. Examples of Wnt family members for which the peptide complex of the present invention can function as a substitute include Wnt3 and Wnt1, and Wnt3 is preferred.

### [Wnt Signal Activation Capability]

The expression "Wnt signal activation capability" refers to a capability to induce an effect similar to the effect generated by naturally occurring Wnt. It also refers to a capability to specifically activate the Wnt signaling pathway.

Wnt signal activation capability may be evaluated, for example, using activation of the Wnt/β-catenin pathway. Examples of evaluations that may be conducted include, but are not limited to, measuring the nuclear translocation of β-catenin in cells, and TCF/LEF reporter assays. Examples of evaluation methods include, but are not limited to, evaluations using PathHunter eXpress β-Catenin Nuclear Translocation Assay (from DiscoverX Corporation), and the TCF/LEF Reporter-HEK 293 cell line (from BPS Bioscience Inc.). In any evaluation system, a substance is said to have Wnt signal activation capability if that Wnt signal activation capability can be detected using the peptide or peptide complex at an optimal concentration under optimal conditions according to standard procedures.

### [Wnt Inhibition Activity]

Wnt inhibition activity means activity that inhibits the Wnt signaling pathway. For example, the peptide of the present invention binds to the Wnt co-receptor LRP5 and/or LRP6 and inhibits Wnt binding to these co-receptors, thereby inhibiting the Wnt signaling pathway.

Wnt inhibition activity may be evaluated, for example, using activation of the Wnt/β-catenin pathway. For example, evaluation may be conducted by, but is not limited to, nuclear translocation of beta-catenin in cells or TCF/LEF reporter assay. Examples of evaluation methods include, but are not limited to, evaluations using PathHunter eXpress β-Catenin Nuclear Translocation Assay (from DiscoverX Corporation), and the TCF/LEF Reporter-HEK 293 cell line (from BPS Bioscience Inc.). In any evaluation system, a substance is said to have Wnt inhibition capability if that Wnt inhibition capability can be detected using the peptide or peptide complex at an optimal concentration under optimal conditions according to standard procedures.

### [Canonical Wnt Signaling Pathway and Non-Canonical Wnt Signaling Pathway]

Refer to Neiheisel, A., et al., "Wnt pathway modulators in cancer therapeutics: An update on completed and ongoing clinical trials", International Journal of Cancer, March 2022, volume 150, issue 5, pp. 727 to 740.

A signaling pathway generally refers to a biochemical causal relationship that is initiated by a protein-protein interaction, such as the binding of a growth factor to a receptor, and causes the transmission of a signal from one part of a cell to another part of the cell. The Wnt signaling pathway refers to the biochemical causal relationship that occurs when the above growth factor is Wnt.

The Wnt signaling pathway is classified into the canonical Wnt signaling pathway and the non-canonical Wnt signaling pathway.

### <Canonical Wnt Signaling Pathway>

In the absence of Wnt ligands, a degradation complex composed of APC, Axin, CK1 and GSK3β causes β-catenin phosphorylation, ubiquitination, and degradation in the proteasome. As a result, β-catenin levels in the cytoplasm are kept low, suppressing the transcription of Wnt target genes. On the other hand, when Wnt ligands bind to a Frizzled receptor and the co-receptor LRP5 or LRP6, Dvl proteins are attracted to the plasma membrane, destabilizing the β-catenin degradation complex. Binding of Wnt ligands to the above receptors induces accumulation of unphosphorylated β-catenin in the cytoplasm, and the β-catenin migrates into the nucleus and forms a complex with TCF/LEF, resulting in the transcription of Wnt target genes. It has also been reported that binding of R-spondin to LGR5 activates the canonical Wnt signaling pathway described above.

The Wnt signaling pathway refers to the canonical Wnt signaling pathway in which members of the Wnt family of secreted protein ligands bind to a receptor complex of LRP and Frizzled (Fzd), thereby enabling β-catenin to migrate into the nucleus, interact with TCF/LEF transcription factors, and activate the expression of target genes.

### <Non-Canonical Wnt Signaling Pathway>

At least two types of non-canonical Wnt signaling pathways exist: the PCP pathway which controls planar cell polarity (PCP), and the Ca2+ pathway which promotes the mobilization of calcium ions within cells.

### (PCP Pathway)

When Wnt ligands bind to the Frizzled receptor and the co-receptor ROR or RYK, Dvl proteins are attracted and activate ROCK, which controls the cytoskeleton, or JNK, which causes activation of Jun, a transcription factor that mediates cellular response.

### (Ca2+ Pathway)

When Wnt ligands interact with the Frizzled receptor and the co-receptor ROR, the membrane-bound enzyme PLC is activated, which converts the membrane-bound phospholipid PIP2 into IP3 and DAG. IP3 causes the release of calcium ions from the endoplasmic reticulum into the cytoplasm. The calcium ions activate calmodulin and PKC. PKC activation causes cellular response.

### [Fzd Receptor]

The Fzd receptors are seven-transmembrane domain proteins of the G protein-coupled receptor (GPCR) superfamily, and contain a large extracellular N-terminal ligand-binding domain having 10 conserved cysteines known as the cysteine-rich domain (CRD) or the Fri domain. Ten types of Fzd receptors (Fzd1 to Fzd10) have been identified in humans, and participate in many important biological processes, such as development, cell proliferation, survival, migration, and stem cell maintenance.

In adult animals, interaction of Wnt ligands with Fzd receptor family members activates the Wnt signaling pathway, regulating embryonic development, stem cell and progenitor cell renewal during tissue homeostasis, and cell differentiation.

### [Fzd Binding Activity]

Fzd (Frizzled) binding activity means the activity of specifically binding to an Fzd (Frizzled) receptor, and preferably the activity of binding to the Fzd2, Fzd7, and/or Fzd8 receptor.

### [LRP5/6]

LRP is an abbreviation for low-density lipoprotein receptor-related protein, and includes LRP1 to LRP6, LRP1B, LRP8, and LRP10 to LRP12. LRP members LRP5 and LRP6 are single-pass transmembrane receptors that exist on the cell surface, and form part of the group of co-receptors LRP5/LRP6/Fzd that participate in the canonical Wnt signaling pathway.

LRP5 and LRP6 exhibit high homology, and have identity of 73% and 64% in the extracellular and intracellular domains, respectively. They are widely co-expressed during embryogenesis and in adult tissues, and are also functionally redundant.

### [LRP Binding Activity]

LRP binding activity means the activity of specifically binding to LRP, and preferably the activity of binding to LRP5 and/or LRP6.

### [Dkk]

Dkk (Dickkopf) refers to nucleic acids and proteins of Dkk family members, including Dkk1 to Dkk4, Soggy, and related Dkk proteins. In this description, one preferred example is Dkk1, although this is not a limitation of the present invention.

Dkk1 binds to the Wnt co-receptors LRP5 and LRP6, and inhibits the binding of Wnt to these co-receptors, thereby inhibiting the Wnt signaling pathway. Dkk1 has been clearly shown to inhibit both Wnt1- and Wnt3a-mediated signaling. In this description, the expression "DKK1 -like activity" refers to the activity of inhibiting the Wnt signaling pathway via the above mechanism.

### [Conservative Amino Acid Substitution]

In the peptide and peptide complex of the present invention, in those cases where one, two or three amino acid residues are substituted, deleted, added, or inserted within a specific amino acid sequence, conservative amino acid substitution is preferred.

The expression "conservative amino acid substitution" means substitution with a functionally equivalent or similar amino acid.

Conservative amino acid substitution in a peptide causes a static change in the amino acid sequence of the peptide. For example, one amino acid or two or more amino acids of similar polarity act in a functionally equivalent manner, resulting in a static change in the amino acid sequence of the peptide. In general, it is thought that substitutions within a group can be considered conservative in both structure and function.

However, as will be evident to those skilled in the art, the role played by a specific amino acid residue can be determined in terms of the significance of the residue in the three-dimensional structure of a molecule containing that amino acid.

Cysteine residues can adopt an oxidized (disulfide) form that has lower polarity compared with the reduced (thiol) form.

The long aliphatic portion of an arginine side chain may constitute an important feature, both structurally and functionally.

Further, side chains containing an aromatic ring (tryptophan, tyrosine, phenylalanine) can contribute to ion-aromatic interactions or cation-pi interactions. In such cases, even if amino acids with these side chains are substituted with amino acids belonging to acidic or nonpolar groups, structural and functional conservatism can still be achieved.

Residues such as proline, glycine, and cysteine (the disulfide form) can have direct effects on the three-dimensional structure of the main chain, and can often not be substituted without structural distortion.

As described below, conservative amino acid substitutions include specific substitutions based on side chain similarity (L. Lehninger, Biochemistry, 2nd edition, pp. 73 to 75, Worth Publisher, New York (1975)) and typical substitutions.

Further, as outlined below, conservative amino acid substitution is, for example, preferably substitution with an amino acid that belongs to the same group as the amino acid, wherein natural amino acids are divided into groups based on the properties of their common side chains.

Hydrophobic (also referred to as non-polar) amino acids: Amino acids that exhibit hydrophobicity (non-polarity), examples of which include L-alanine (A), glycine (G), L-valine (V), L-leucine (L), L-isoleucine (I), L-proline (P), L-phenylalanine (F), L-tryptophan (W), L-tyrosine (Y), and L-methionine (M).

Hydrophobic amino acids can also be further divided into the following groups.

Aliphatic amino acids: Amino acids having an aliphatic hydrocarbon group or a hydrogen atom in the side chain, including, for example, L-alanine (A or Ala), glycine (G or Gly), L-valine (V or Val), L-isoleucine (I or Ile), and L-leucine (L or Leu).

Aliphatic branched-chain amino acids: Amino acids having a branched aliphatic hydrocarbon group in the side chain, including, for example, L-valine (V), L-isoleucine (I), and L-leucine (L).

Aromatic amino acids: Amino acids having an aromatic ring in the side chain, including, for example, L-tryptophan (W), L-tyrosine (Y), and L-phenylalanine (F).

Hydrophilic (also referred to as polar) amino acids: Amino acids that exhibit hydrophilicity (polarity), including, for example, L-serine (S), L-threonine (T), L-cysteine (C), L-asparagine (N), L-glutamine (Q), L-aspartic acid (D), L-glutamic acid (E), L-lysine (K), L-arginine (R), and L-histidine (H).

Hydrophilic amino acids can also be further divided into the following groups.

Acidic amino acids: Amino acids in which the side chain exhibits acidity, including, for example, L-aspartic acid (D) and L-glutamic acid (E).

Basic amino acids: Amino acids in which the side chain exhibits basicity, including, for example, L-lysine (K), arginine (R), and L-histidine (H).

Neutral amino acids: Amino acids in which the side chain is neutral, including, for example, L-serine (S), L-threonine (T), L-asparagine (N), L-glutamine (Q), and L-cysteine (C).

Glycine (G) and L-proline (P) can also be classified as "amino acids that influence the orientation of the main chain."

L-cysteine (C) and L-methionine (M), which contain a sulfur atom in the side chain, can also be classified as "sulfur-containing amino acids."

In this description, the term "amino acid" includes not only natural amino acids but also unnatural amino acids. Unnatural amino acids include, for example, N-alkyl amino acids in which the natural amino acids described above have been N-alkylated, and amino acids in which the nitrogen atom that forms the peptide bond has been modified with a branched or unbranched lower (for example, C1 to C5, preferably C1 to C3, and more preferably C1) alkyl group. N-alkyl amino acids are preferably N-ethyl amino acids, N-butyl amino acids, or N-methyl amino acids, and are more preferably N-methyl amino acids.

Furthermore, unnatural amino acids include D-type amino acids (also referred to as D-amino acids), β-amino acids, γ-amino acids, amino acid variants, chemically modified amino acids such as amino acid derivatives, and amino acids that do not become structural components of proteins in vivo, such as norleucine and ornithine.

Moreover, the unnatural amino acids also include amino acids in which a functional group has been added to the side chain of a natural amino acid or substituted with another functional group (for example, amino acids having a substitution or addition in a portion of the side chain such as an arylene group or alkylene group, amino acids having an increased C number in the arylene group, alkylene group or alkyl group of the side chain, amino acids having a substitution in an aromatic ring of the side chain, and amino acids that have undergone heterocyclization or condensed ring cyclization).

Further, by adding or substituting a structure such as a functional group in the side chain of a natural amino acid, the amino acid can be imparted with different properties from the natural amino acid. For example, (S)-2-amino-3-(pyridin-4-yl)propanoic acid (4Py) is an alanine with an added pyridyl group (pyridine ring) in the side chain, and the addition of the pyridyl group means the amino acid is basic and exhibits polarity, unlike alanine which belongs to the group of nonpolar amino acids.

In other words, unnatural amino acids having similar side chain properties may be included in the above groups dividing natural amino acids based on the properties of their common side chains. For example, methyl-L-arginine (MeR), an N-methylated amino acid of the basic amino acid arginine, is an unnatural amino acid, but exhibits basicity and can therefore be classified as a basic amino acid. In this manner, unnatural amino acids with similar side chain properties to certain amino acids may also be included as targets for conservative amino acid substitution.

Unnatural amino acids include, but are not limited to, N-methyl amino acids, (S)-2-amino-3-(pyridin-4-yl)propanoic acid (4Py), allothreonine (alT), L-citrulline (Cit), O-methyl-L-serine (SMe), (2S)-2-amino-3-(oxan-4-yl)propanoic acid (Atp), L-2-aminoadipic acid (Hgl), (S)-2-amino-3-(naphthalen-1-yl)propanoic acid (Nal1), (S)-2-amino-3-(1H-pyrrolo[2,3-c]pyridin-3-yl)propanoic acid (W6N), and (S)-2-amino-3-(1H-pyrrolo[2,3-b]pyridin-3-yl)propanoic acid (W7N). N-methyl amino acids can also be classified as N-alkyl amino acids, or can be classified in accordance with the properties of the side chain of the non-N-methylated original amino acid.

### [Peptides and Pharmaceutically Acceptable Salts, Esters, or Solvates thereof]

Examples of pharmaceutically acceptable salts include, but are not limited to, inorganic acid salts, organic acid salts, inorganic base salts, organic base salts, and acidic or basic amino acid salts.

Examples of the inorganic acid salts include, but are not limited to, hydrochlorides, hydrobromides, sulfates, nitrates, and phosphates.

Examples of the organic acid salts include, but are not limited to, acetates, succinates, fumarates, maleates, tartrates, citrates, lactates, stearates, benzoates, methanesulfonates, and p-toluenesulfonates.

Examples of the inorganic base salts include, but are not limited to, alkali metal salts such as sodium salts and potassium salts, alkaline earth metal salts such as calcium salts and magnesium salts, as well as aluminum salts and ammonium salts.

Examples of the organic base salts include, but are not limited to, diethylamine salts, diethanolamine salts, meglumine salts, and N,N'-dibenzylethylenediamine salts.

Examples of the acidic amino acid salts include aspartates and glutamates, and examples of the basic amino acid salts include arginine salts, lysine salts, and ornithine salts, but the invention is not limited to these salts.

Examples of pharmaceutically acceptable esters include, but are not limited to, acetate esters and sulfate esters.

Examples of pharmaceutically acceptable solvates includes, but is not limited to, hydrates.

### [Peptide]

The peptide of the present invention may be a pharmaceutically acceptable salt, ester or solvate. Examples of the solvate include hydrates.

### (First Embodiment)

A first embodiment of the peptide of the present invention contains an amino acid sequence represented by formula A1, or an amino acid sequence in which one or a plurality of amino acid residues have been substituted, deleted, added or inserted within an amino acid sequence represented by formula A1. In formula A1, the amino acid sequence is described from the N-terminal side to the C-terminal side.

A1: X1-X2-X3-X4-X5-X6-X7-X8-X9-X10-X11-X12-X13-X14

In formula A1:
X1 represents any D-amino acid residue,
X2 and X3 each independently represent any amino acid residue,
X4 and X5 each independently represent an amino acid residue having an aliphatic hydrocarbon group which may be substituted in the side chain,
X6 represents an amino acid residue having an aryl group which may be substituted in the side chain,
X7 represents an N-alkyl amino acid residue having an aryl group which may be substituted or a cycloalkyl group which may be substituted in the side chain,
X8 represents any amino acid residue,
X9 represents an L-threonine (T) residue,
X10 represents any N-alkylated amino acid residue,
X11 represents any amino acid residue,
X12 represents an amino acid residue having an aliphatic hydrocarbon group in the side chain,
X13 represents an amino acid residue having an aliphatic hydrocarbon group which may be substituted in the side chain, and
X14 represents an L-cysteine (C) residue, and

In the peptide of the first embodiment, X1 preferably represents a D-phenylalanine (df) residue, (R)-2-amino-3-(pyridin-4-yl)propanoic acid (d4py) residue, (R)-2-amino-3-(pyridin-3-yl)propanoic acid (d3py) residue, (R)-2-amino-3-(3-methoxyphenyl)propanoic acid (df3OMe) residue, (R)-2-amino-3-(4-methoxyphenyl)propanoic acid (df4OMe) residue, (R)-2-amino-5-ureidopentanoic acid (dicit) residue, D-tyrosine (dy) residue, or (R)-2-amino-3-(4-fluorophenyl)propanoic acid (df4F) residue.

The structures of d4py, d3py, df4F, df3OMe, df4OMe, dcit, df, and dy are shown below.

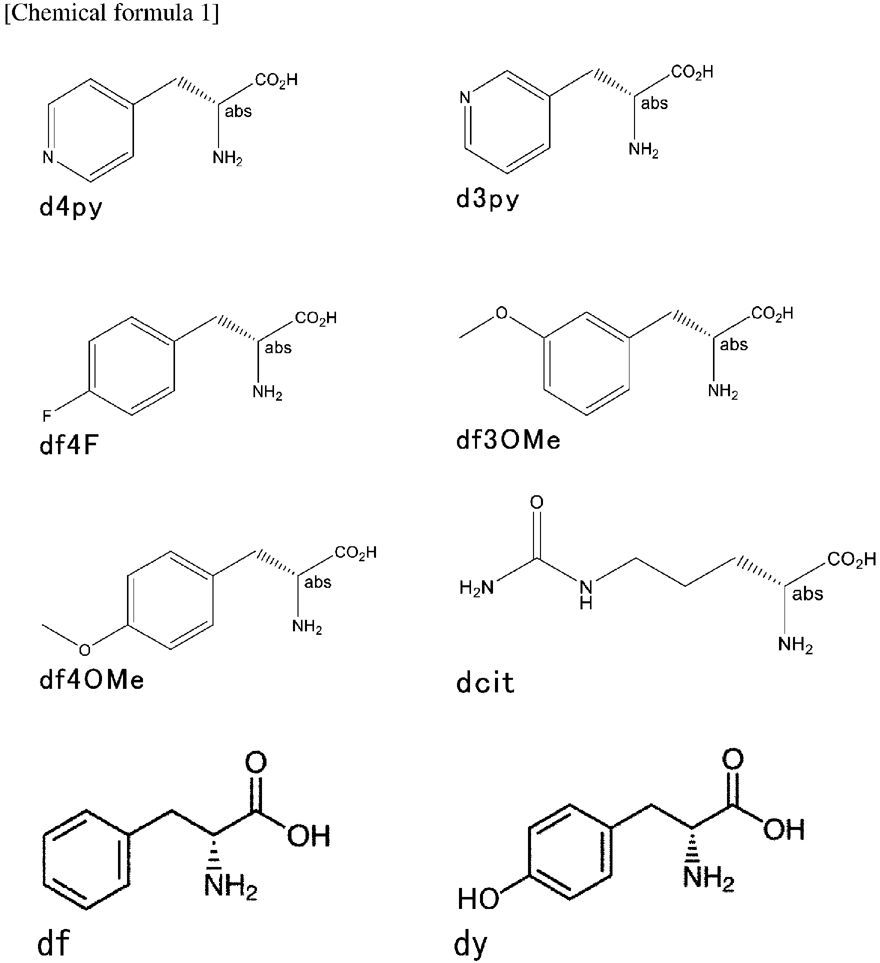

In the peptide of the first embodiment, X2 preferably represents an L-serine (S) residue, L-asparagine (N) residue, L-aspartic acid (D) residue, L-2-aminoadipic acid (Hgl) residue, L-citrulline (Cit) residue, or (S)-2-amino-6-morpholinohexanoic acid (Kmor) residue.

The structures of Kmor, Hgl, and Cit are shown below.

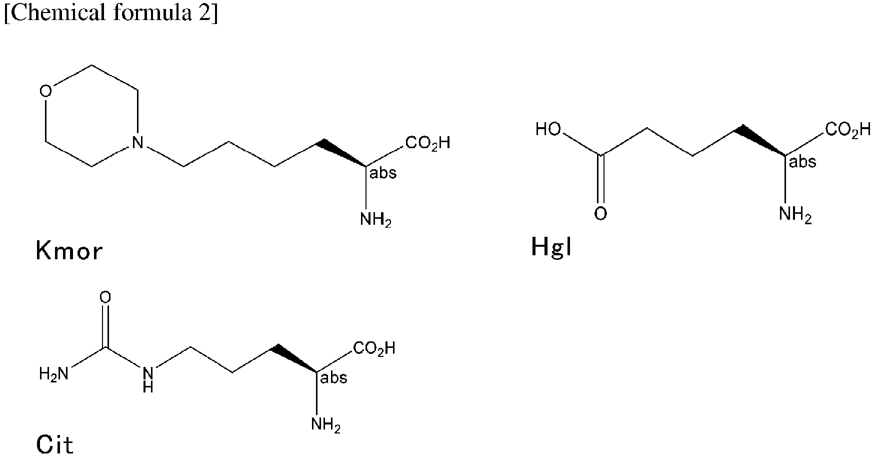

In the peptide of the first embodiment, X3 preferably represents an L-aspartic acid (D) residue, L-arginine (R) residue, L-citrulline (Cit) residue, N6-(4-(carboxymethyl)piperazine-1-carbonyl)-L-lysine (KCOpipzaa) residue, (2S)-2-amino-3-(oxan-4-yl)propanoic acid (Atp) residue, L-asparagine (N) residue, or L-serine (S) residue.

The structures of Cit, Atp, and KCOpipzaa are shown below.

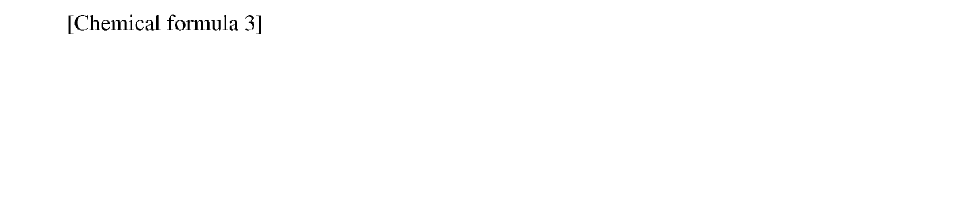

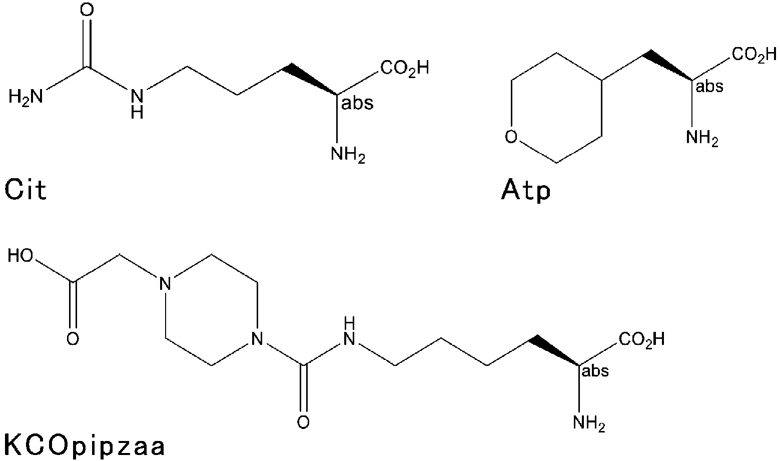

In the peptide of the first embodiment, X4 preferably represents an L-isoleucine (I) residue or an O-methyl-L-threonine (TMe) residue.

The structure of TMe is shown below.

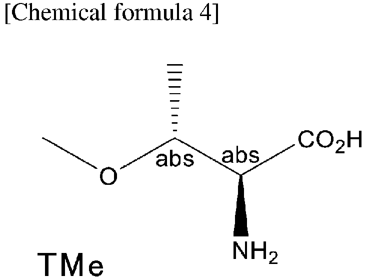

In the peptide of the first embodiment, X5 preferably represents an L-arginine (R) residue, L-glutamic acid (E) residue, (S)-2-aminoheptanoic acid (Ahp) residue, L-serine (S) residue, L-homoserine (Hse) residue, L-citrulline (Cit) residue, an N6-carbamoyl-L-lysine (Hcit) residue, (S)-2-amino-4-ureidobutanoic acid (Ncit) residue, or (S)-5-acetamido-2-aminopentanoic acid (OrnAc) residue.

The structures of Ahp, Hse, Ncit, Hcit, Cit, and OrnAc are shown below.

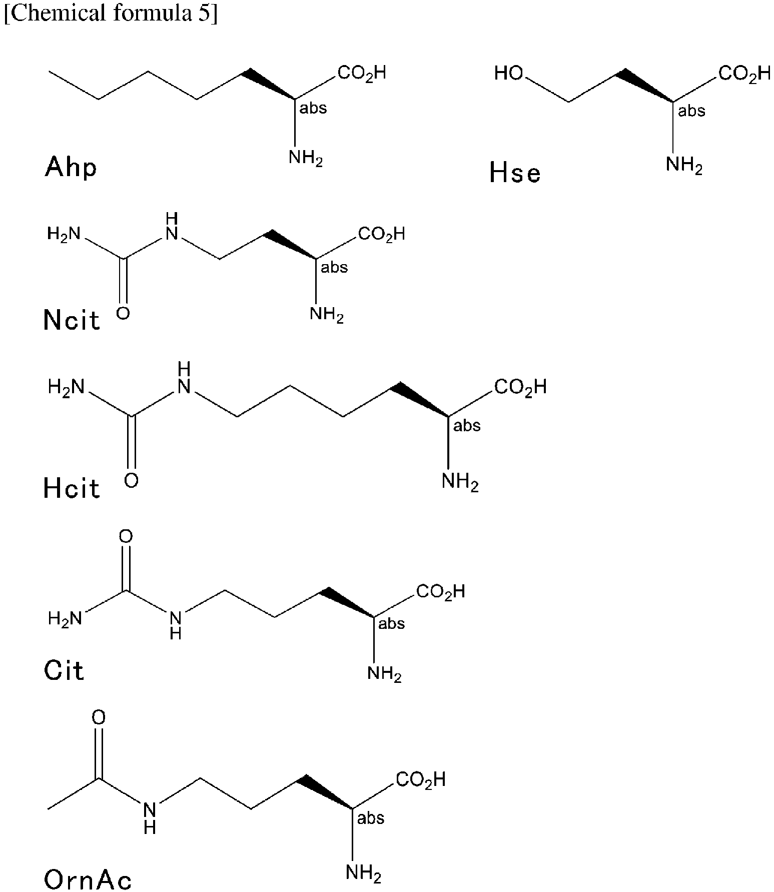

In the peptide of the first embodiment, X6 preferably represents an L-tyrosine (Y) residue, methyl-L-tyrosine (MeY) residue, (S)-2-amino-3-(4-fluorophenyl)propanoic acid (F4F) residue, (S)-2-amino-3-(4-chlorophenyl)propanoic acid (F4C) residue, (S)-2-amino-3-(pyridin-4-yl)propanoic acid (4Py) residue, or (S)-3-(2-amino-2-carboxyethyl)benzoic acid (F3COO) residue.

The structures of F4F, F4C, F3COO, and 4Py are shown below.

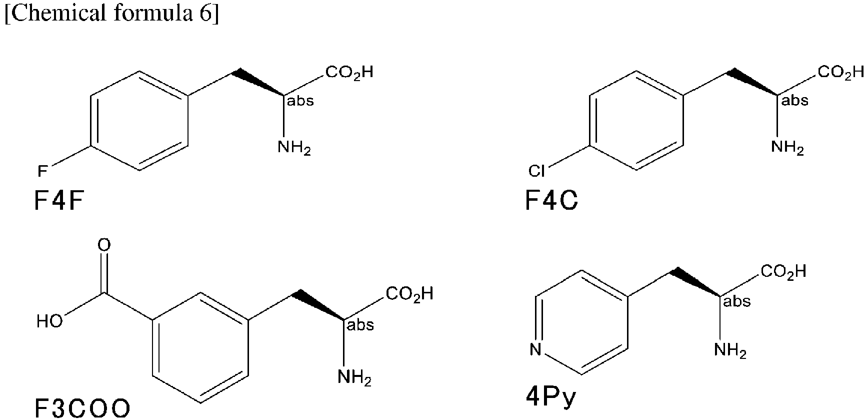

In the peptide of the first embodiment, X7 preferably represents a methyl-L-phenylalanine (MeF) residue, (S)-2-(methylamino)-3-(pyridin-3-yl)propanoic acid (Me3Py) residue, (S)-2-(methylamino)-3-(pyridin-4-yl)propanoic acid (Me4Py) residue, (S)-3-(2-carboxy-2-(methylamino)ethyl)benzoic acid (MeF3COO) residue, (S)-4-(2-carboxy-2-(methylamino)ethyl)benzoic acid (MeF4COO) residue, or (S)-2-(methylamino)-3-(tetrahydro-2H-pyran-4-yl)propanoic acid (MeAtp) residue.

The structures of Me3Py, Me4Py, MeF3COO, MeF4COO, and MeAtp are shown below.

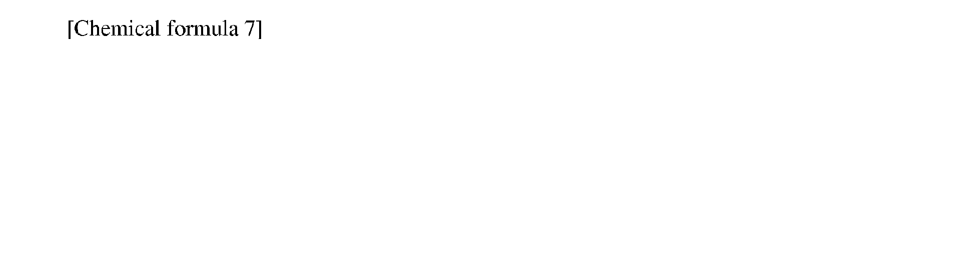

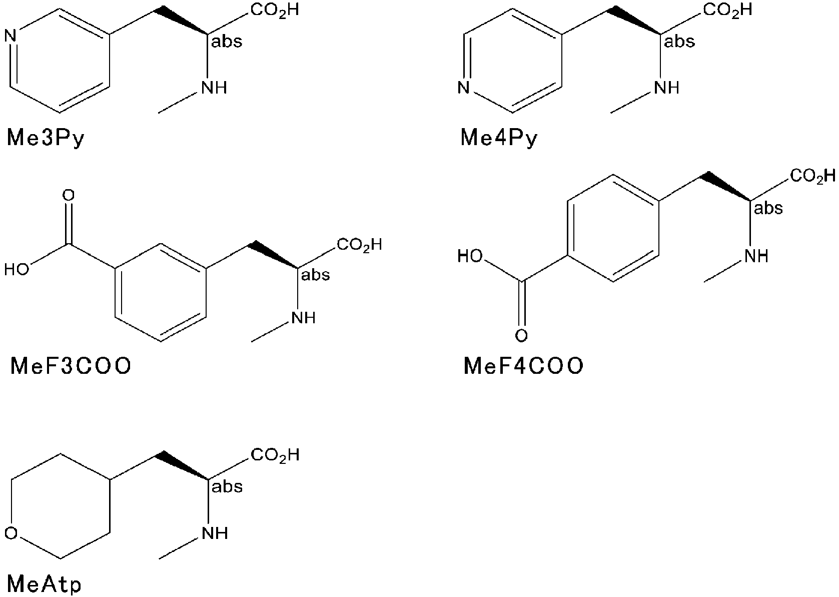

In the peptide of the first embodiment, X8 preferably represents an L-glutamine (Q) residue, L-valine (V) residue, L-2-aminoadipic acid (Hgl) residue, 1-(carboxymethyl)-L-tryptophan (W1aa) residue, (S)-2-aminoheptanoic acid (Ahp) residue, homo-L-tyrosine (Hty) residue, L-threonine (T) residue, allothreonine (alT) residue, L-citrulline (Cit) residue, N6-(4-(carboxymethyl)piperazine-1-carbonyl)-L-lysine (KCOpipzaa) residue, (S)-2-amino-2-(tetrahydro-2H-pyran-4-yl)acetic acid (Gthp) residue, (S)-2-amino-2-(1-(carboxymethyl)piperidin-4-yl)acetic acid (G4pipaa) residue, (S)-2-amino-2-cyclobutylacetic acid (Cbg) residue, (S)-2-amino-2-cyclohexylacetic acid (Chg) residue, or O-methyl-threonine (TMe) residue

The structures of Hgl, Ahp, Hty, Cit, W1aa, KCOpipzaa, TMe, alT, Cbg, Gthp, G4pipaa, and Chg are shown below.

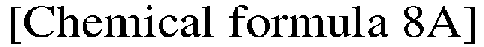

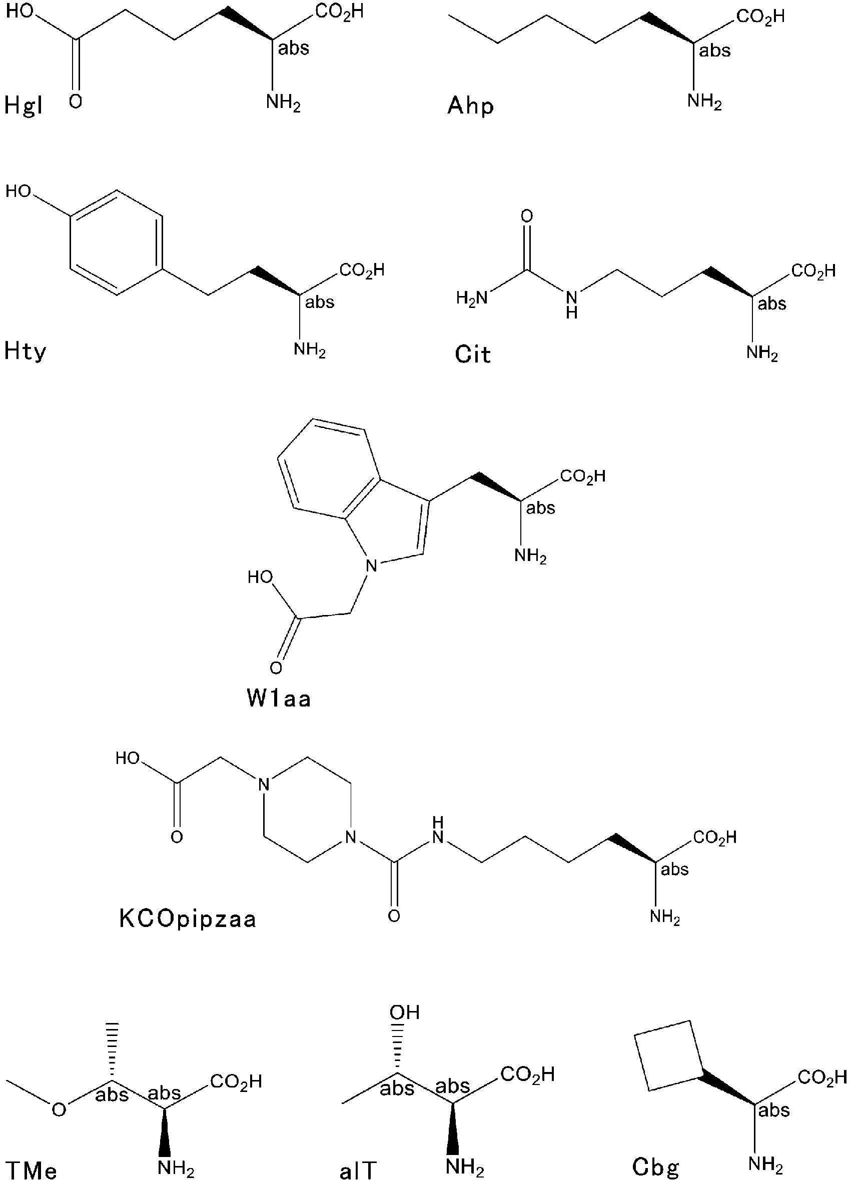

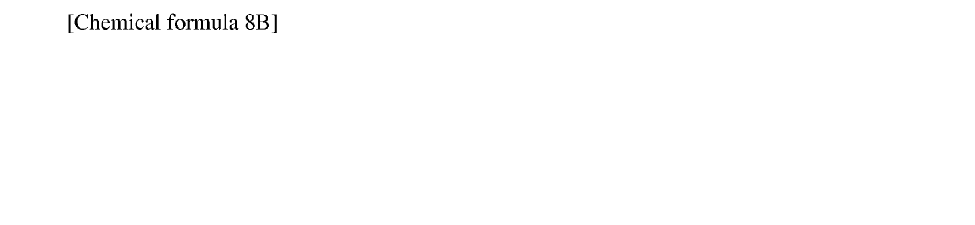

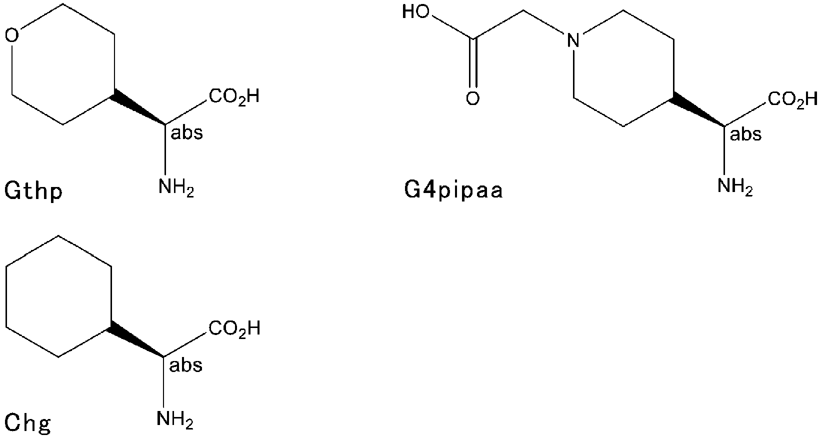

In the peptide of the first embodiment, X10 preferably represents a methyl-L-phenylalanine (MeF) residue, (S)-2-(methylamino)hexanoic acid (MeNle) residue, (S)-2-(methylamino)-3-(pyridin-3-yl)propanoic acid (Me3Py) residue, (S)-2-(methylamino)-3-(pyridin-4-yl)propanoic acid (Me4Py) residue, (S)-4-(2-carboxy-2-(methylamino)ethyl)benzoic acid (MeF4COO) residue, or (S)-2-(methylamino)-3-(tetrahydro-2H-pyran-4-yl)propanoic acid (MeAtp) residue.

The structures of Me3Py, Me4Py, MeF4COO, MeAtp, and MeNle are shown below.

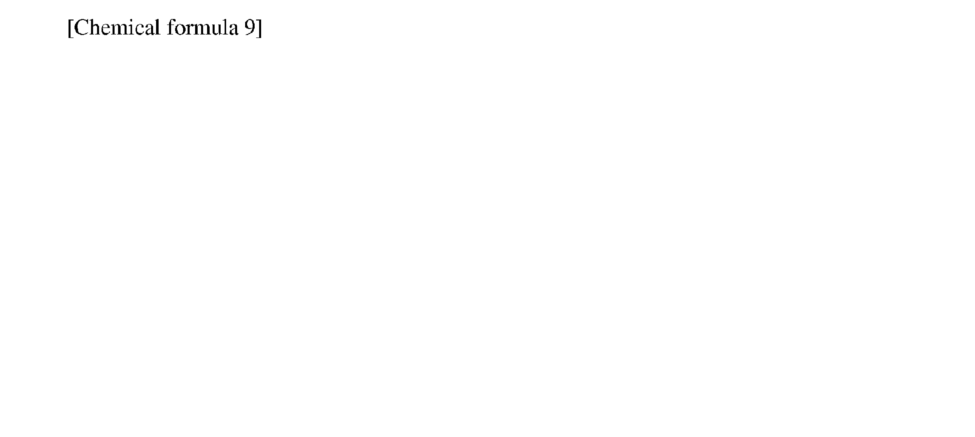

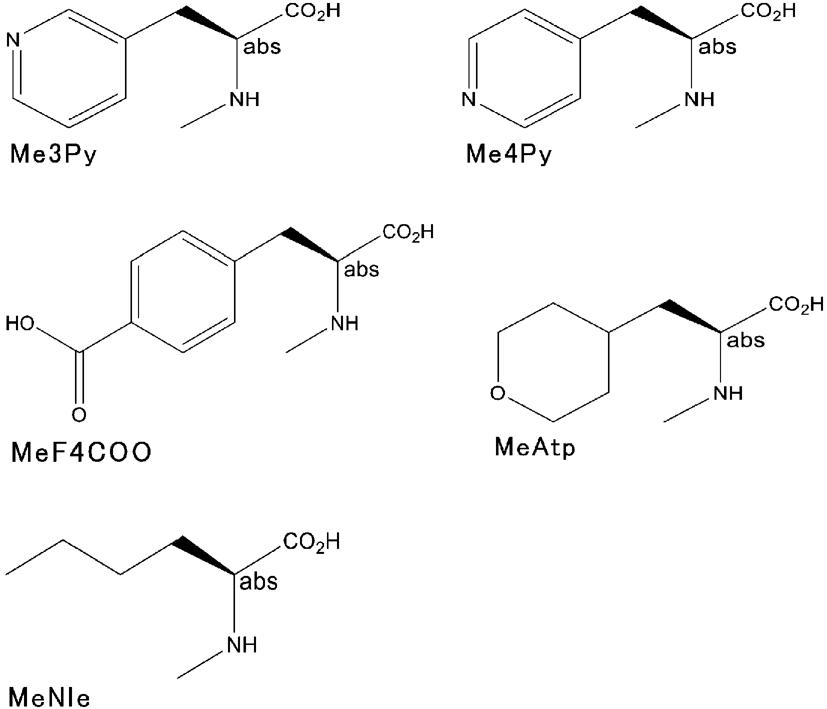

In the peptide of the first embodiment, X11 preferably represents an L-isoleucine (I) residue, L-tyrosine (Y) residue, (S)-2-amino-3-(4-(carboxymethoxy)phenyl)propanoic acid (F4aao) residue, or O-methyl-L-threonine (TMe) residue.

The structures of F4aao and TMe are shown below.

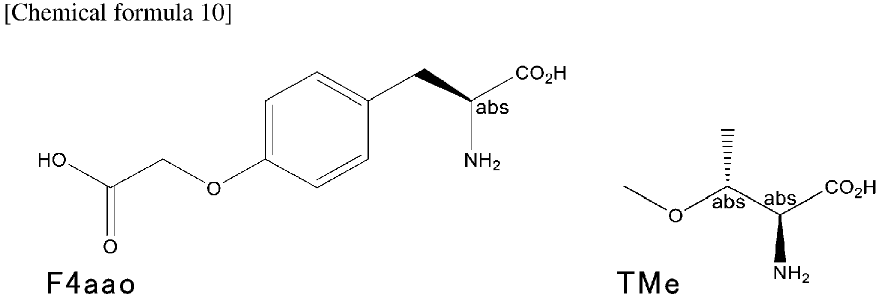

In the peptide of the first embodiment, X12 preferably represents an L-valine (V) residue, methyl-L-valine (MeV) residue, or N-methyl-L-cyclohexylglycine (MeChg) residue.

The structure of MeChg is shown below.

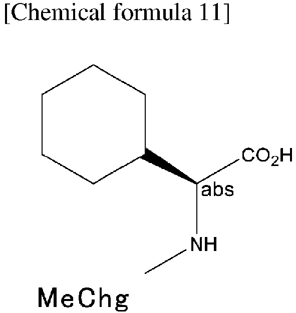

In the peptide of the first embodiment, X13 preferably represents a glycine (G) residue, D-alanine (da) residue, or D-serine (ds) residue.

The peptide of the first embodiment is preferably a peptide containing an amino acid sequence represented by formula A2, or an amino acid sequence in which at least one amino acid residue among 1 to 12 amino acid residues selected from the group consisting of the 1st, 2nd, 3rd, 4th, 5th, 6th, 7th, 8th, 10th, 11th, 12th and 13th amino acid residues in an amino acid sequence represented by formula A2 has been substituted, deleted, added or inserted. In formula A2, the amino acid sequence is described from the N-terminal side to the C-terminal side.

A2: df-S-D-I-R-Y-MeF-Q-T-MeF-I-V-G-C (SEQ ID NO: 164)

In formula A2:
df represents a D-phenylalanine residue,
S represents an L-serine residue,
D represents an L-aspartic acid residue,
I represents an L-isoleucine residue,
R represents an L-arginine residue,
Y represents an L-tyrosine residue,
MeF represents a methyl-L-phenylalanine residue,
Q represents an L-glutamine residue,
T represents an L-threonine residue,
V represents an L-valine residue,
G represents a glycine residue, and
C represents an L-cysteine residue.

The peptide of the first embodiment preferably also has a glycine residue at the C-terminal.

The peptide of the first embodiment is preferably a cyclic peptide.

In those cases where the peptide of the first embodiment is a cyclic peptide, the peptide preferably has a cyclic structure in which an amino acid residue derived from a chloroacetylated amino acid and an L-cysteine residue contained within the peptide are bonded together.

A cyclic peptide describes a peptide in which two amino acids within the peptide are bonded together, with all or part of the resulting structure forming a ring. In this description, the term "cyclic peptide" also includes peptides in which amino acids in the peptide form a crosslinked structure, peptides in which a cyclic structure is formed by lactam ring formation or ring formation resulting from a macrocyclization reaction, and peptides having a lasso peptide-like structure and the like. In other words, in this description, a cyclic peptide may be a peptide in which a portion of the peptide forms a cyclic structure, wherein the peptide may also have a linear portion.

In order to achieve cyclization of the peptide of an embodiment of the present invention, a portion of the amino acids may sometimes be modified. The peptide of an embodiment of the present invention also includes these types of peptides containing partially modified amino acids. One example of a modification used for achieving cyclization involves adding a chloroacetyl group to the amino acid located at the N-terminal, and binding this chloroacetyl group to a cysteine residue in the peptide to achieve cyclization. Peptides containing various (natural/unnatural) amino acids to which a chloroacetyl group has been added are also included within the definition of a peptide in this description.

Peptides generally have poor metabolic stability in vivo and, because of their large size, also suffer from poor permeation through cell membranes. Peptide cyclization has been used as a method to address these issues. It has been suggested that cyclization of a peptide improves protease resistance, thereby improving metabolic stability, and also limits conformational changes, thereby increasing rigidity and improving membrane permeability and affinity for target proteins.

Peptide cyclization can be conducted using conventional methods. For example, by designing a peptide to contain two or more cysteine residues, a cyclic structure can be formed via a disulfide bond following translation, although the present invention is not limited to this method. Cyclization can also be achieved using the method of Goto et al. (Goto, Y. et al., ACS Chem. Biol., 2008, Vol. 3, pp. 120 to 129), by synthesizing a peptide having a chloroacetyl group at the N-terminal and positioning a cysteine residue in the peptide using genetic code reprogramming technology. This enables spontaneous nucleophilic attack by the mercapto group on the chloroacetyl group after translation, causing cyclization of the peptide via a thioether bond. Using genetic code reprogramming technology, other combinations of amino acids that bond to form a cyclic structure can also be positioned in the peptide for cyclization. Alternatively, by synthesizing a peptide having a cycloamide at the N-terminal, and positioning an L-2-aminoadipic acid residue in the peptide, cyclization can be achieved by bonding these two moieties. In this manner, any known cyclization method can be used without any particular limitations.

The peptide of the first embodiment preferably also contains one or more additional amino acid residues. In such cases, there are no particular limitations on these additional amino acid residues.

In the peptide of the first embodiment, additional amino acid residues may be included in a peptide having a cyclic structure, or these additional amino acid residues may be added to the cyclic peptide in the form of a linker. There are no particular limitations on the number of amide bonds (the number of amino acids, or the length) in the peptide or peptide moiety, but the total number of amino acid residues (referring to the number of amino acid residues contained in the peptide that constitutes the cyclic structure, and not including any additional amino acid residues that may be added to the cyclic peptide in the form of a linker) is preferably 20 residues or fewer. The peptide length is preferably at least 6, at least 7, at least 8, at least 9, at least 10, or 11 or more amino acid residues, and is preferably not more than 19, or 18 or fewer amino acid residues. A more preferred peptide length has at least 13 but not more than 16 amino acid residues, and most preferably 14 or 15 amino acids.

The peptide of the first embodiment preferably has binding properties to an Fzd receptor.

The Fzd receptor is preferably at least one receptor selected from the group consisting of the Fzd receptor 2, Fzd receptor 7 and Fzd receptor 8.

The peptide of the first embodiment is preferably a peptide having an amino acid sequence represented by formula A3:

A3: ClAc-X1-X2-X3-X4-X5-X6-X7-X8-X9-X10-X11-X12-X13-X14

wherein ClAc represents a chloroacetyl group, X1, X2, X3, X4, X5, X6, X7, X8, X9, X10, X11, X12, X13, and X14 represent the amino acid residues shown in Table 1. The amino acid residue abbreviations in Table 1 are as described above.

**[Table 1A]**

| Table 1 (1 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 1 | df | S | D | I | R | Y | MeF | Q | T | MeF | I | V | G | C | G |
| 2 | dcit | S | D | I | R | Y | MeF | Q | T | MeF | I | V | G | C | G |
| 3 | dy | S | D | I | R | Y | MeF | Q | T | MeF | I | V | G | C | G |
| 4 | df4F | S | D | I | R | Y | MeF | Q | T | MeF | I | V | G | C | G |
| 5 | df | S | D | I | R | MeY | MeF | Q | T | MeF | I | V | G | C | G |
| 6 | df | S | D | I | R | Y | MeF | Q | T | MeF | I | MeV | G | C | G |
| 7 | df | S | D | I | R | Y | MeF | V | T | MeF | I | MeV | G | C | G |
| 8 | df | Kmor | D | I | R | Y | MeF | Q | T | MeF | I | V | G | C | G |
| 9 | df | S | R | I | R | Y | MeF | Q | T | MeF | I | V | G | C | G |
| 10 | df | S | Cit | I | R | Y | MeF | Q | T | MeF | I | V | G | C | G |
| 11 | df | S | KCOpipzaa | I | R | Y | MeF | Q | T | MeF | I | V | G | C | G |
| 12 | df | S | Atp | I | R | Y | MeF | Q | T | MeF | I | V | G | C | G |
| 13 | df | S | D | I | E | Y | MeF | Q | T | MeF | I | V | G | C | G |
| 14 | df | S | D | I | Ahp | Y | MeF | Q | T | MeF | I | V | G | C | G |
| 15 | df | S | D | I | R | Y | MeF | Hgl | T | MeF | I | V | G | C | G |
| 16 | df | S | D | I | R | Y | MeF | W1aa | T | MeF | I | V | G | C | G |
| 17 | df | S | D | I | R | Y | MeF | V | T | MeF | I | V | G | C | G |
| 18 | df | S | D | I | R | Y | MeF | Ahp | T | MeF | I | V | G | C | G |
| 19 | df | S | D | I | R | Y | MeF | Hty | T | MeF | I | V | G | C | G |
| 20 | df | S | D | I | R | Y | MeF | T | T | MeF | I | V | G | C | G |

**[Table 1B]**

| Table 1 (2 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 21 | df | S | D | I | R | Y | MeF | alT | T | MeF | I | V | G | C | G |
| 22 | df | S | D | I | R | Y | MeF | Cit | T | MeF | I | V | G | C | G |
| 23 | df | S | D | I | R | Y | MeF | KCOpipzaa | T | MeF | I | V | G | C | G |
| 24 | df | S | D | I | R | Y | MeF | Q | T | MeNle | I | V | G | C | G |
| 25 | df | S | D | I | R | Y | MeF | Q | T | MeF | Y | v | G | C | G |
| 26 | df | S | D | I | R | Y | MeF | Q | T | MeF | F4aao | V | G | C | G |
| 27 | d4py | S | D | I | R | Y | MeF | v | T | MeF | I | MeV | G | C | G |
| 28 | d3py | S | D | I | R | Y | MeF | V | T | MeF | I | MeV | G | C | G |
| 29 | df3OMe | S | D | I | R | Y | MeF | v | T | MeF | I | MeV | G | C | G |
| 30 | df4OMe | S | D | I | R | Y | MeF | V | T | MeF | I | MeV | G | C | G |
| 31 | df | N | D | I | R | Y | MeF | V | T | MeF | I | MeV | G | C | G |
| 32 | df | D | D | I | R | Y | MeF | v | T | MeF | I | MeV | G | C | G |
| 33 | df | Hgl | D | I | R | Y | MeF | V | T | MeF | I | MeV | G | C | G |
| 34 | df | Cit | D | I | R | Y | MeF | v | T | MeF | I | MeV | G | C | G |
| 35 | df | S | Atp | I | R | Y | MeF | V | T | MeF | I | MeV | G | C | G |
| 36 | df | S | N | I | R | Y | MeF | V | T | MeF | I | MeV | G | C | G |
| 37 | df | S | S | I | R | Y | MeF | V | T | MeF | I | MeV | G | C | G |
| 38 | df | S | D | TMe | R | Y | MeF | V | T | MeF | I | MeV | G | C | G |
| 39 | df | S | D | I | S | Y | MeF | V | T | MeF | I | MeV | G | C | G |
| 40 | df | S | D | I | Hse | Y | MeF | V | T | MeF | I | MeV | G | C | G |

**[Table 1C]**

| Table 1 (3 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 41 | df | S | D | I | Cit | Y | MeF | V | T | MeF | I | MeV | G | C | G |
| 42 | df | S | D | I | Hcit | Y | MeF | V | T | MeF | I | MeV | G | C | G |
| 43 | df | S | D | I | Ncit | Y | MeF | V | T | MeF | I | MeV | G | C | G |
| 44 | df | S | D | I | OrnAc | Y | MeF | V | T | MeF | I | MeV | G | C | G |
| 45 | df | S | D | I | R | F4F | MeF | v | T | MeF | I | MeV | G | C | G |
| 46 | df | S | D | I | R | F4C | MeF | V | T | MeF | I | MeV | G | C | G |
| 47 | df | S | D | I | R | 4Py | MeF | v | T | MeF | I | MeV | G | C | G |
| 48 | df | S | D | I | R | F3COO | MeF | V | T | MeF | I | MeV | G | C | G |
| 49 | df | S | D | I | R | Y | Me3Py | v | T | MeF | I | MeV | G | C | G |
| 50 | df | S | D | I | R | Y | Me4Py | V | T | MeF | I | MeV | G | C | G |
| 51 | df | S | D | I | R | Y | MeF3COO | V | T | MeF | I | MeV | G | C | G |
| 52 | df | S | D | I | R | Y | MeF4COO | v | T | MeF | I | MeV | G | C | G |
| 53 | df | S | D | I | R | Y | MeAtp | V | T | MeF | I | MeV | G | C | G |
| 54 | df | S | D | I | R | Y | MeF | Gthp | T | MeF | I | MeV | G | C | G |
| 55 | df | S | D | I | R | Y | MeF | G4pipaa | T | MeF | I | MeV | G | C | G |
| 56 | df | S | D | I | R | Y | MeF | Cbg | T | MeF | I | MeV | G | C | G |
| 57 | df | S | D | I | R | Y | MeF | Chg | T | MeF | I | MeV | G | C | G |
| 58 | df | S | D | I | R | Y | MeF | TMe | T | MeF | I | MeV | G | C | G |
| 59 | df | S | D | I | R | Y | MeF | V | T | Me3Py | I | MeV | G | C | G |
| 60 | df | S | D | I | R | Y | MeF | V | T | Me4Py | I | MeV | G | C | G |

**[Table 1D]**

| Table 1 (4 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 61 | df | S | D | I | R | Y | MeF | V | T | MeF4COO | I | MeV | G | C | G |
| 62 | df | S | D | I | R | Y | MeF | V | T | MeAtp | I | MeV | G | C | G |
| 63 | df | S | D | I | R | Y | MeF | V | T | MeF | TMe | MeV | G | C | G |
| 64 | df | S | D | I | R | Y | MeF | v | T | MeF | I | MeChg | G | C | G |
| 65 | df | S | D | I | R | Y | MeF | v | T | MeF | I | MeV | da | C | G |
| 66 | df | S | D | I | R | Y | MeF | V | T | MeF | I | MeV | ds | C | G |
| 67 | df | S | Atp | I | R | Y | MeF | V | T | MeF | I | V | G | C | G |
| 68 | df | S | Cit | I | R | Y | MeF | V | T | MeF | I | MeV | G | C | G |
| 69 | df | S | Atp | I | R | Y | MeF | V | T | MeF | Y | MeV | G | C | G |
| 70 | df | Hgl | D | I | R | Y | MeF | v | T | Me4Py | I | MeV | G | C | G |
| 71 | df | Hgl | D | I | R | Y | MeF3COO | V | T | Me4Py | I | MeV | G | C | G |
| 72 | df | Hgl | D | I | Cit | Y | MeF3COO | V | T | Me4Py | I | MeV | G | C | G |
| 73 | df | Hgl | D | I | Cit | Y | MeF3COO | TMe | T | Me4Py | I | MeV | ds | C | G |
| 74 | df | S | D | I | R | Y | MeF | V | T | Me4Py | I | MeV | ds | C | G |
| 75 | df | Hgl | D | I | R | Y | MeF | V | T | Me4Py | I | MeV | ds | C | G |
| 76 | df | S | Cit | I | R | Y | MeF | V | T | Me4Py | I | MeV | ds | C | G |
| 77 | df | Hgl | D | I | R | Y | MeF3COO | V | T | Me4Py | I | MeV | ds | C | G |
| 78 | df | Hgl | D | I | Cit | Y | MeF3COO | V | T | Me4Py | I | MeV | ds | C | G |

### (Second Embodiment)

A second embodiment of the peptide of the present invention contains an amino acid sequence represented by formula B1, or an amino acid sequence in which one or a plurality of amino acid residues have been substituted, deleted, added or inserted within an amino acid sequence represented by formula B1:

B1: Y1-Y2-Y3-Y4-Y5-Y6-Y7-Y8-Y9-Y10-Y11-Y12-Y13-Y14

In formula B1:
Y1 represents an L-phenylalanine (F) residue which may be substituted,
Y2 represents an L-arginine (R) residue which may be substituted,
Y3 represents an L-tryptophan (W) residue which may be substituted or an (S)-2-amino-3-(naphthalen-1-yl)propanoic acid (Nal1) residue which may be substituted,
Y4 represents an acidic amino acid residue or a neutral amino acid residue,
Y5 represents an aromatic amino acid residue which may be substituted, an aliphatic amino acid residue, or an L-proline (P) residue,
Y6 represents an aliphatic amino acid residue which may be substituted,
Y7 represents an N-alkylated aromatic amino acid residue,
Y8 represents an amino acid residue derived from an amino acid having a hydroxy group, carboxy group or amide group in the side chain,
Y9 represents any amino acid residue,
Y10 represents an L-tryptophan (W) residue which may be substituted,
Y11 represents an L-asparagine (N) residue which may be substituted or a glutamine (Q) residue,
Y12 represents an (S)-1,2,3,4-tetrahydroisoquinoline-3-carboxylic acid (Tic) residue which may be substituted,
Y13 represents a neutral amino acid residue or acidic amino acid residue which may be substituted, and
Y14 represents an L-cysteine (C) residue.

In the peptide of the second embodiment, Y1 preferably represents an L-phenylalanine (F) residue, (S)-2-amino-3-(4-fluorophenyl)propanoic acid (F4F) residue, (S)-2-amino-3-(p-toluyl)propanoic acid (F4Me) residue, or (S)-2-amino-3-(m-toluyl)propanoic acid (F3Me) residue.

The structures of F3Me, F4F, and F4Me are shown below.

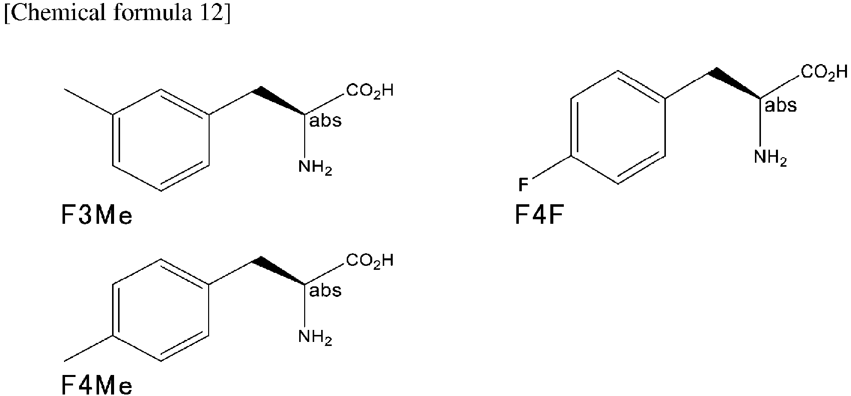

In the peptide of the second embodiment, Y2 preferably represents an L-arginine (R) residue or N6-carbamoyl-L-lysine (Hcit) residue.

The structure of Hcit is shown below.

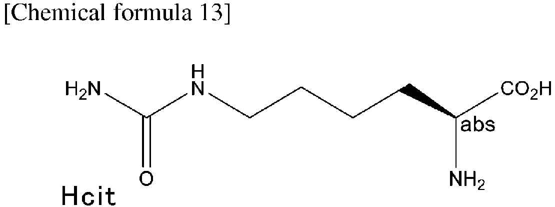

In the peptide of the second embodiment, Y3 preferably represents an L-tryptophan (W) residue, methyl-L-tryptophan (MeW) residue, (S)-2-amino-3-(1H-pyrrolo[2,3-b]pyridin-3-yl)propanoic acid (W7N) residue, (S)-2-amino-3-(1H-pyrrolo[3,2-c]pyridin-3-yl)propanoic acid (W5N) residue, (S)-2-amino-3-(1-methyl-1H-pyrrolo[2,3-b]pyridin-3-yl)propanoic acid (W1Me7N) residue, (S)-2-amino-3-(5-methyl-1H-pyrrolo[2,3-b]pyridin-3-yl)propanoic acid (W7N5Me) residue, (S)-2-amino-3-(naphthalen-1-yl)propanoic acid (Nal1) residue, or (S)-2-amino-3-(quinolin-4-yl)propanoic acid (Nal14N) residue.

The formulas of W7N, W5N, W1Me7N, W7N5Me, Nal1, and Nal14N are shown below.

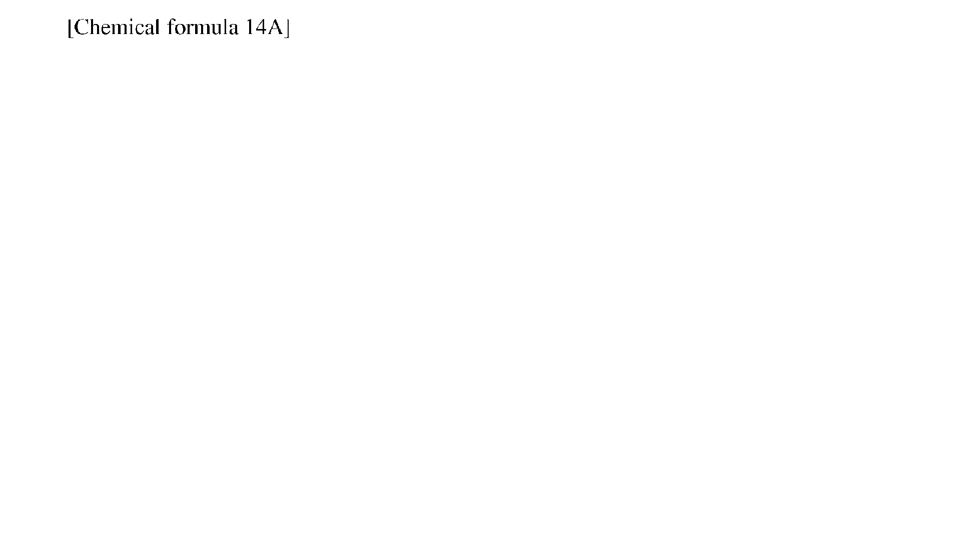

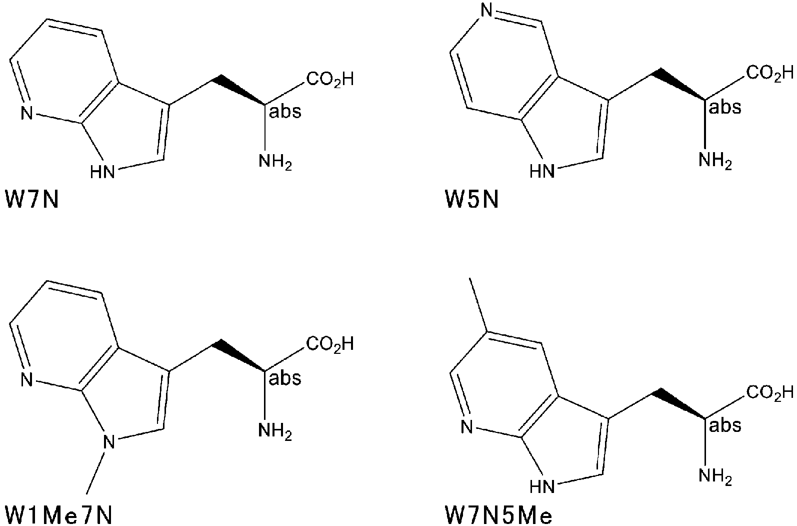

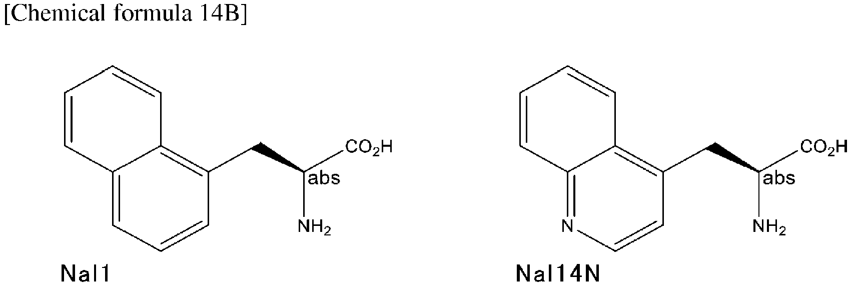

In the peptide of the second embodiment, Y4 preferably represents an L-aspartic acid (D) residue, L-glutamic acid (E) residue, L-asparagine (N) residue, or L-glutamine (Q) residue.

In the peptide of the second embodiment, Y5 preferably represents an L-valine (V) residue, methyl-L-valine (MeV) residue, 1-(carboxymethyl)-L-tryptophan (W1aa) residue, (S)-2-amino-3-cyclohexylpropanoic acid (Cha) residue, (S)-2-amino-3-(naphthalen-1-yl)propanoic acid (Nal1) residue, (S)-2-amino-3-(5-phenylpyridin-3-yl)propanoic acid (3Py5Ph) residue, (S)-2-amino-2-cyclobutylacetic acid (Cbg) residue, or L-proline (P) residue.

The structures of Nal1, 3Py5Ph, W1aa, Cha, and Cbg are shown below.

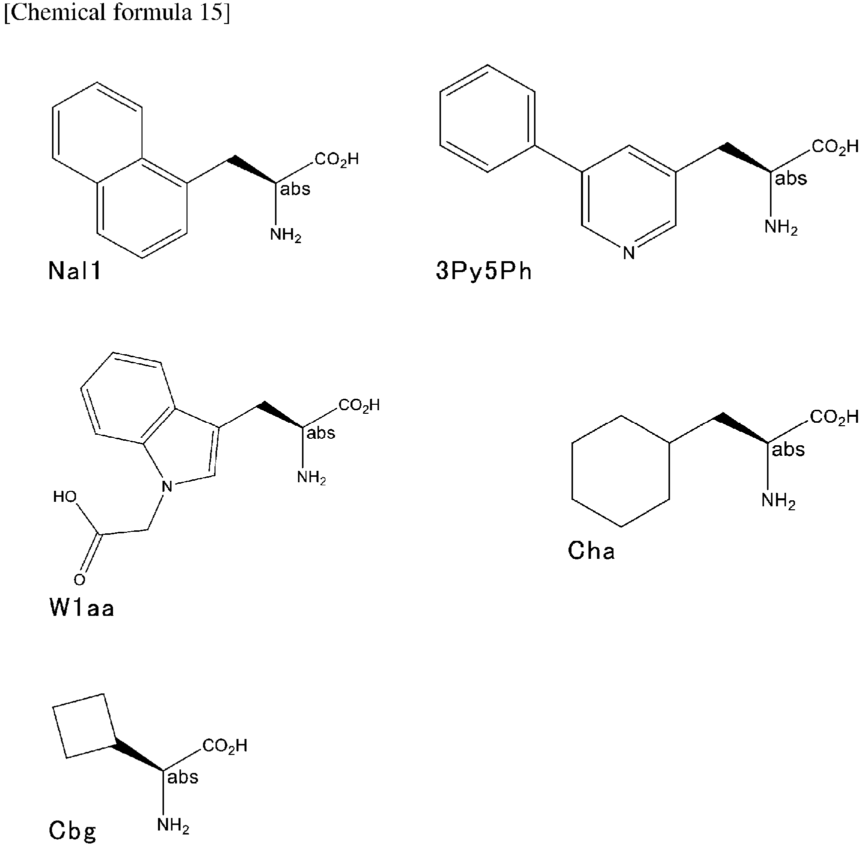

In the peptide of the second embodiment, Y6 preferably represents an L-arginine (R) residue, methyl-L-arginine (MeR) residue, (S)-2-amino-6-morpholinohexanoic acid (Kmor) residue, L-citrulline (Cit) residue, N6-carbamoyl-L-lysine (Hcit) residue, (S)-2-amino-4-ureidobutanoic acid (Ncit) residue, or (S)-5-acetamido-2-aminopentanoic acid (OrnAc) residue.

The structures of Kmor, Cit, Heit, Ncit, and OrnAc are shown below.

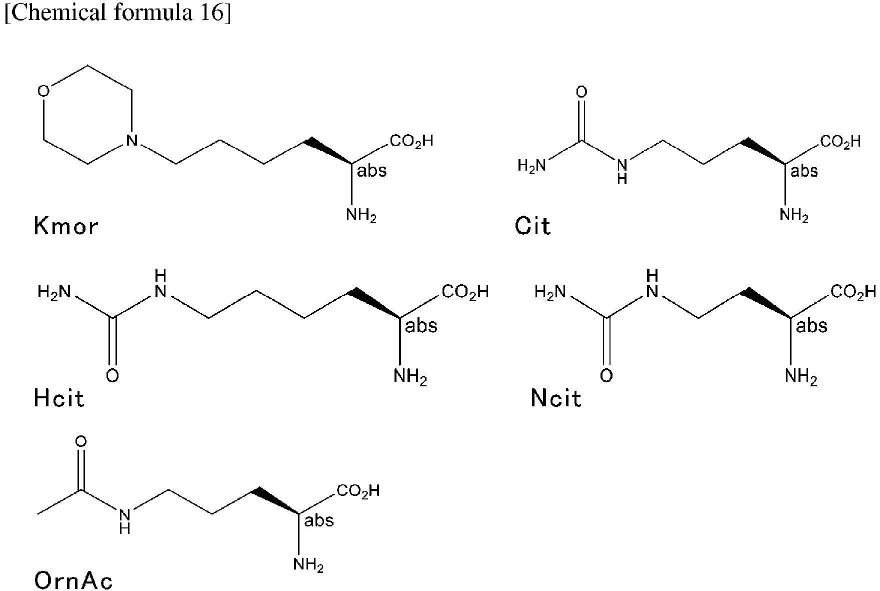

In the peptide of the second embodiment, Y7 preferably represents a methyl-L-phenylalanine (MeF) residue, (S)-4-(2-carboxy-2-(methylamino)ethyl)benzoic acid (MeF4COO) residue, methyl-L-tyrosine (MeY) residue, (S)-2-(methylamino)-3-(p-toluyl)propanoic acid (MeF4Me) residue, (S)-3-(4-chlorophenyl)-2-(methylamino)propanoic acid (MeF4C) residue, (S)-3-(2-carboxy-2-(methylamino)ethyl)benzoic acid (MeF3COO) residue, (S)-2-(methylamino)-3-(m-toluyl)propanoic acid (MeF3Me) residue, or (S)-3-(3-chlorophenyl)-2-(methylamino)propanoic acid (MeF3C) residue.

The structures of MeF4COO, MeF4C, MeF4Me, MeF3Me, MeF3COO, and MeF3C are shown below.

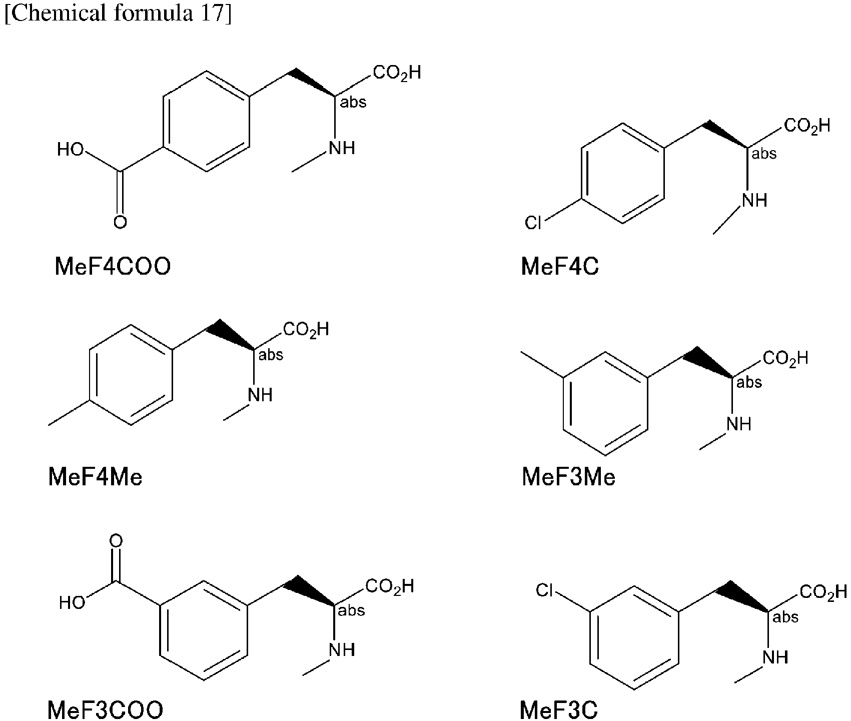

In the peptide of the second embodiment, Y8 preferably represents an L-serine (S) residue, 1-(carboxymethyl)-L-tryptophan (W1aa) residue, homo-L-tyrosine (Hty) residue, allothreonine (alT) residue, L-threonine (T) residue, L-asparagine (N) residue, L-glutamine (Q) residue, L-aspartic acid (D) residue, or L-glutamic acid (E) residue.

The structures of alT, Hty, and W1aa are shown below.

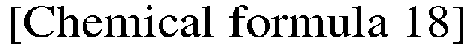

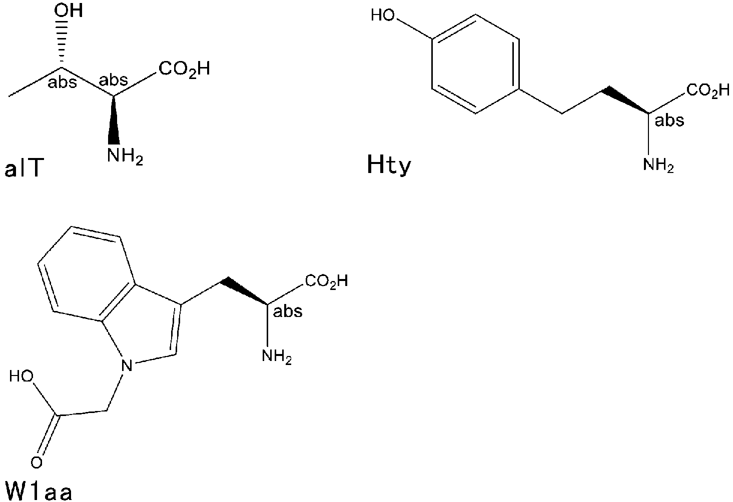

In the peptide of the second embodiment, Y9 preferably represents an L-asparagine (N) residue, methyl-L-asparagine (MeN) residue, L-2-aminoadipic acid (Hgl) residue, (S)-2-amino-3-cyclohexylpropanoic acid (Cha) residue, homo-L-tyrosine (Hty) residue, (S)-2-amino-4-(1H-pyrrolo[2,3-b]pyridin-3-yl)butanoic acid (Hw7N) residue, N6-(4-(carboxymethyl)piperazine-1-carbonyl)-L-lysine (KCOpipzaa) residue, L-glutamine (Q) residue, L-aspartic acid (D) residue, or L-glutamic acid (E) residue.

The structures of Cha, Hty, Hgl, Hw7N, and KCOpipzaa are shown below.

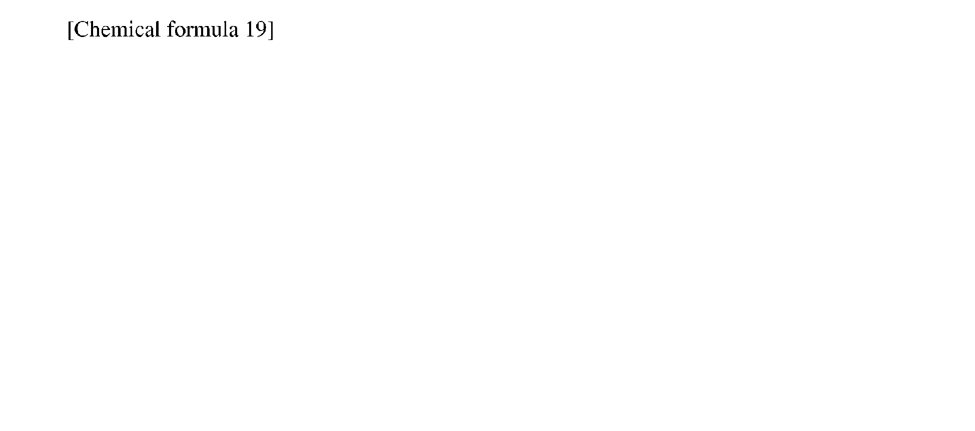

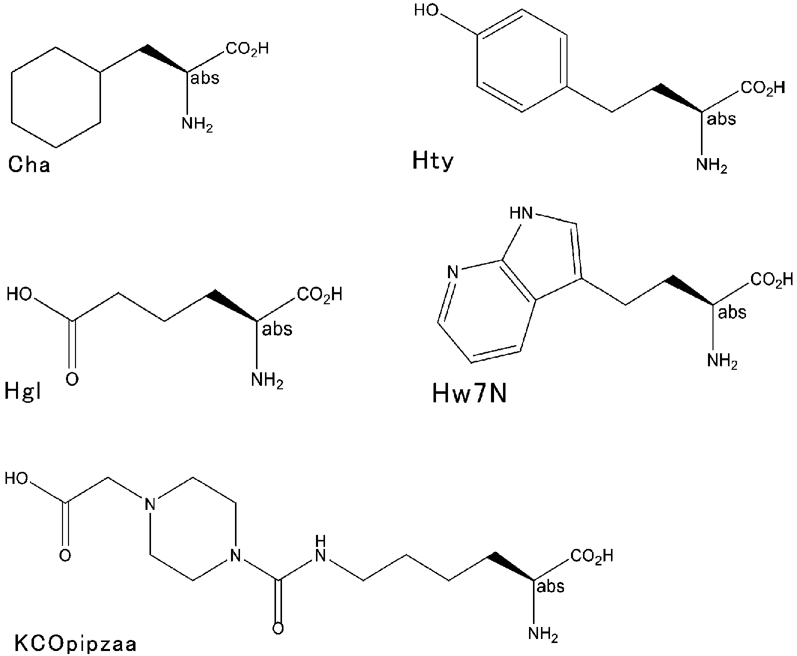

In the peptide of the second embodiment, Y10 preferably represents an L-tryptophan (W) residue, (S)-2-amino-3-(1H-pyrrolo[2,3-b]pyridin-3-yl)propanoic acid (W7N) residue, (S)-2-amino-3-(1H-pyrrolo[2,3-c]pyridin-3-yl)propanoic acid (W6N) residue, (S)-2-amino-3-(1-methyl-1H-pyrrolo[2,3-b]pyridin-3-yl)propanoic acid (W1Me7N) residue, or (S)-2-amino-3-(5-methyl-1H-pyrrolo[2,3-b]pyridin-3-yl)propanoic acid (W7N5Me) residue.

The structures of W1Me7N, W7N, W7N5Me, and W6N are shown below.

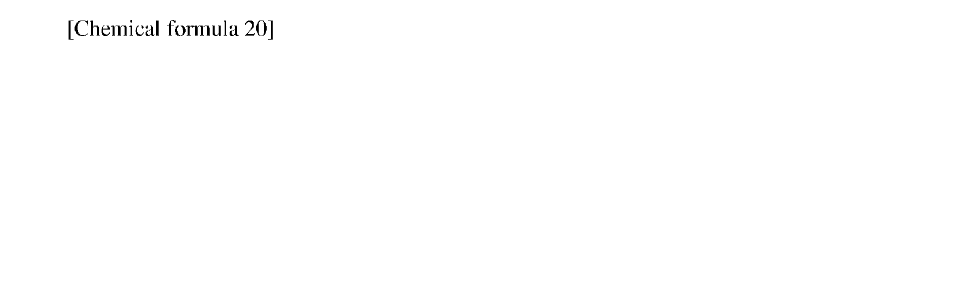

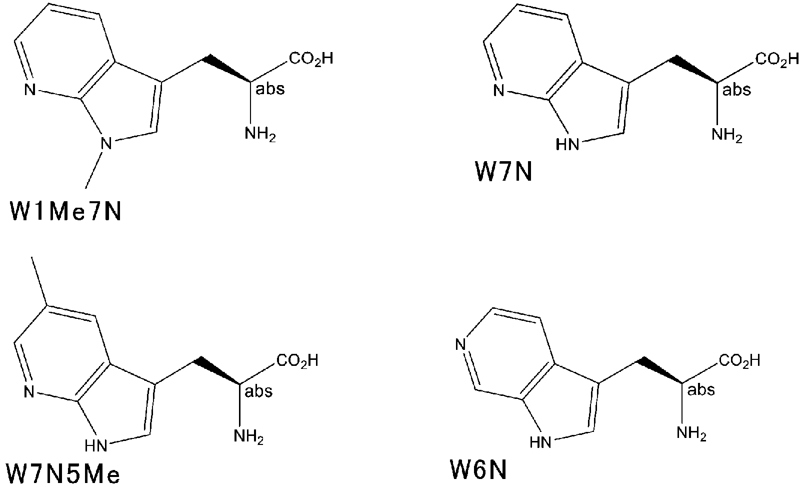

In the peptide of the second embodiment, Y11 preferably represents an L-asparagine (N) residue, L-glutamine (Q) residue, or (2S)-2-amino-4-(methylamino)-4-oxobutanoic acid (Nmm) residue.

The structure of Nmm is shown below.

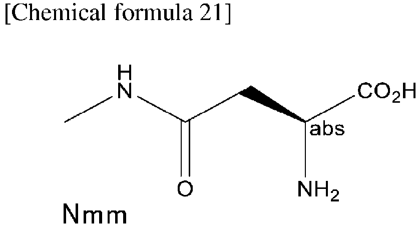

In the peptide of the second embodiment, Y12 preferably represents an (S)-1,2,3,4-tetrahydroisoquinoline-3-carboxylic acid (Tic) residue, (S)-6-chloro-1,2,3,4-tetrahydroisoquinoline-3-carboxylic acid (Tic6C) residue, (S)-7-hydroxy-1,2,3,4-tetrahydroisoquinoline-3-carboxylic acid (Tic7H) residue, or (S)-7-chloro-1,2,3,4-tetrahydroisoquinoline-3-carboxylic acid (Tic7C) residue.

The structures of Tic, Tic6C, Tic7C, and Tic7H are shown below.

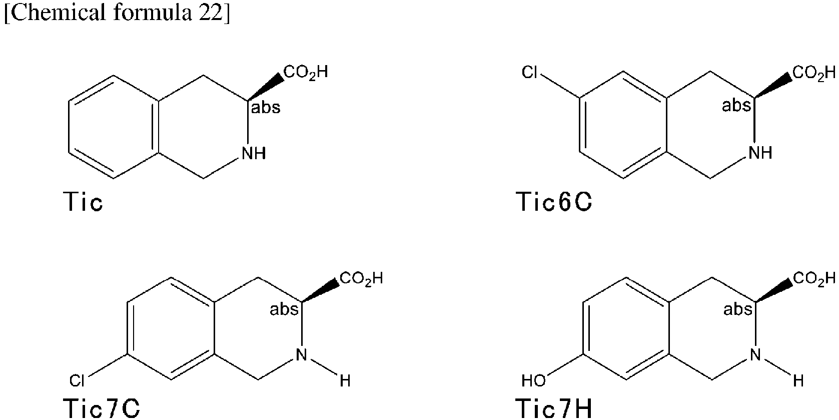

In the peptide of the second embodiment, Y13 preferably represents an L-asparagine (N) residue, L-aspartic acid (D) residue, L-glutamine (Q) residue, (2S)-2-amino-4-(methylamino)-4-oxobutanoic acid (Nmm) residue, or N4,N4-dimethyl-L-asparagine (Ndm) residue.

The structures of Nmm and Ndm are shown below.

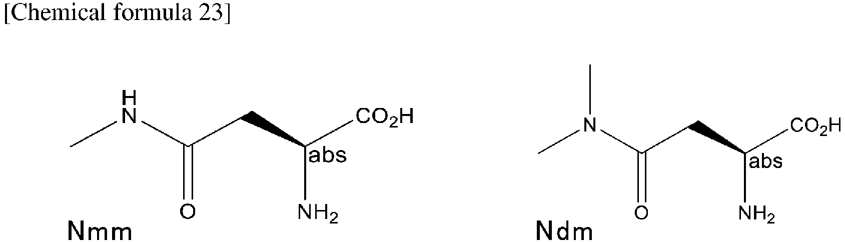

The peptide of the second embodiment is preferably a peptide containing an amino acid sequence represented by formula B2, or an amino acid sequence in which at least one amino acid residue among 1 to 13 amino acid residues selected from the group consisting of the 1st, 2nd, 3rd, 4th, 5th, 6th, 7th, 8th, 9th, 10th, 11th, 12th and 13th amino acid residues in an amino acid sequence represented by formula B2 has been substituted, added, deleted or inserted. In formula B2, the amino acid sequence is described from the N-terminal side to the C-terminal side.

B2: F-R-W-D-V-R-MeF-S-N-W-N-Tic-N-C (SEQ ID NO: 165)

wherein:
F represents an L-phenylalanine residue,
R represents an L-arginine residue,
W represents an L-tryptophan residue,
D represents an L-aspartic acid residue,
V represents an L-valine residue,
MeF represents a methyl-L-phenylalanine residue,
S represents an L-serine residue,
Tic represents an (S)-1,2,3,4-tetrahydroisoquinoline-3-carboxylic acid residue,
N represents an L-asparagine residue, and
C represents an L-cysteine residue.

The peptide of the second embodiment preferably also has a glycine residue at the C-terminal. There are no particular limitations on the number of glycine residues, which may be any number of one or greater.

The peptide of the second embodiment is preferably a cyclic peptide.

In those cases where the peptide of the second embodiment is a cyclic peptide, the peptide preferably has a cyclic structure in which an amino acid residue derived from a chloroacetylated amino acid and an L-cysteine residue contained within the peptide are bonded together.

The peptide of the second embodiment preferably also contains one or more additional amino acid residues.

There are no particular limitations on these additional amino acid residues.

The peptide of the second embodiment preferably has binding properties to LRP.

The above LPR is preferably at least one receptor selected from the group consisting of LRP5 and LRP6.

The peptide of the second embodiment preferably has DKK1-like activity.

The peptide of the second embodiment preferably has an amino acid sequence represented by formula B3.

B3: ClAc-Y1-Y2-Y3-Y4-Y5-Y6-Y7-Y8-Y9-Y10-Y11-Y12-Y13-Y14

In formula B3, ClAc represents a chloroacetyl group, and Y1, Y2, Y3, Y4, Y5, Y6, Y7, Y8, Y9, Y10, Y11, Y12, Y13 and Y14 represent the amino acid residues shown in Table 2. The amino acid residue abbreviations in Table 2 are as described above.

**[Table 2A]**

| Table 2 (1 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 79 | F | R | W | D | V | R | MeF | S | N | W | N | Tic | N | C | G |
| 80 | F4F | R | W | D | V | R | MeF | S | N | W | N | Tic | N | C | G |
| 81 | F | R | MeW | D | V | R | MeF | S | N | W | N | Tic | N | C | G |
| 82 | F | R | W | D | MeV | R | MeF | S | N | W | N | Tic | N | C | G |
| 83 | F | R | W | D | V | MeR | MeF | S | N | W | N | Tic | N | C | G |
| 84 | F | R | W | D | V | R | MeF | S | MeN | W | N | Tic | N | C | G |
| 85 | F | R | W7N | D | V | R | MeF | S | N | W | N | Tic | N | C | G |
| 86 | F | R | W | D | W1aa | R | MeF | S | N | W | N | Tic | N | C | G |
| 87 | F | R | W | D | Cha | R | MeF | S | N | W | N | Tic | N | C | G |
| 88 | F | R | W | D | Nal1 | R | MeF | S | N | W | N | Tic | N | C | G |
| 89 | F | R | W | D | 3Py5Ph | R | MeF | S | N | W | N | Tic | N | C | G |
| 90 | F | R | W | D | V | Kmor | MeF | S | N | W | N | Tic | N | C | G |
| 91 | F | R | W | D | V | R | MeF | W1aa | N | W | N | Tic | N | C | G |
| 92 | F | R | W | D | V | R | MeF | Hty | N | W | N | Tic | N | C | G |
| 93 | F | R | W | D | V | R | MeF | alT | N | W | N | Tic | N | C | G |
| 94 | F | R | W | D | V | R | MeF | S | Hgl | W | N | Tic | N | C | G |
| 95 | F | R | W | D | V | R | MeF | S | Cha | W | N | Tic | N | C | G |
| 96 | F | R | W | D | V | R | MeF | S | Hty | W | N | Tic | N | C | G |
| 97 | F | R | W | D | V | R | MeF | S | Hw7N | W | N | Tic | N | C | G |
| 98 | F | R | W | D | V | R | MeF | S | KCOpipzaa | W | N | Tic | N | C | G |

**[Table 2B]**

| Table 2 (2 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 99 | F | R | W | D | V | R | MeF | S | N | W7N | N | Tic | N | C | G |
| 100 | F | R | W | D | V | R | MeF | S | N | W | N | Tic | D | C | G |
| 101 | F4Me | R | W | D | V | R | MeF | S | N | W | N | Tic | N | C | G |
| 102 | F3Me | R | W | D | V | R | MeF | S | N | W | N | Tic | N | C | G |
| 103 | F | Hcit | W | D | V | R | MeF | S | N | W | N | Tic | N | C | G |
| 104 | F | R | W5N | D | V | R | MeF | S | N | W | N | Tic | N | C | G |
| 105 | F | R | W7N | D | V | R | MeF | S | N | W | N | Tic | N | C | G |
| 106 | F | R | W1Me7N | D | v | R | MeF | S | N | W | N | Tic | N | C | G |
| 107 | F | R | W7N5Me | D | v | R | MeF | S | N | W | N | Tic | N | C | G |
| 108 | F | R | Nal1 | D | V | R | MeF | S | N | W | N | Tic | N | C | G |
| 109 | F | R | Nal14N | D | V | R | MeF | S | N | W | N | Tic | N | C | G |
| 110 | F | R | W | E | v | R | MeF | S | N | W | N | Tic | N | C | G |
| 111 | F | R | W | N | v | R | MeF | S | N | W | N | Tic | N | C | G |
| 112 | F | R | W | Q | V | R | MeF | S | N | W | N | Tic | N | C | G |
| 113 | F | R | W | D | Cbg | R | MeF | S | N | W | N | Tic | N | C | G |
| 114 | F | R | W | D | P | R | MeF | S | N | W | N | Tic | N | C | G |
| 115 | F | R | W | D | V | Cit | MeF | S | N | W | N | Tic | N | C | G |
| 116 | F | R | W | D | V | Hcit | MeF | S | N | W | N | Tic | N | C | G |
| 117 | F | R | W | D | V | Ncit | MeF | S | N | W | N | Tic | N | C | G |
| 118 | F | R | W | D | V | OrnAc | MeF | S | N | W | N | Tic | N | C | G |

**[Table 2C]**

| Table 2 (3 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 119 | F | R | W | D | V | R | MeF4COO | S | N | W | N | Tic | N | C | G |
| 120 | F | R | W | D | V | R | MeY | S | N | W | N | Tic | N | C | G |
| 121 | F | R | W | D | V | R | MeF4Me | S | N | W | N | Tic | N | C | G |
| 122 | F | R | W | D | V | R | MeF4C | S | N | W | N | Tic | N | C | G |
| 123 | F | R | W | D | V | R | MeF3COO | S | N | W | N | Tic | N | C | G |
| 124 | F | R | W | D | V | R | MeF3Me | S | N | W | N | Tic | N | C | G |
| 125 | F | R | W | D | V | R | MeF3C | S | N | W | N | Tic | N | C | G |
| 126 | F | R | W | D | V | R | MeF | T | N | W | N | Tic | N | C | G |
| 127 | F | R | W | D | V | R | MeF | N | N | W | N | Tic | N | C | G |
| 128 | F | R | W | D | V | R | MeF | Q | N | W | N | Tic | N | C | G |
| 129 | F | R | W | D | V | R | MeF | D | N | W | N | Tic | N | C | G |
| 130 | F | R | W | D | V | R | MeF | E | N | W | N | Tic | N | C | G |
| 131 | F | R | W | D | V | R | MeF | S | Q | W | N | Tic | N | C | G |
| 132 | F | R | W | D | V | R | MeF | S | D | W | N | Tic | N | C | G |
| 133 | F | R | W | D | V | R | MeF | S | E | W | N | Tic | N | C | G |
| 134 | F | R | W | D | V | R | MeF | S | N | W6N | N | Tic | N | C | G |
| 135 | F | R | W | D | V | R | MeF | S | N | W1Me7N | N | Tic | N | C | G |
| 136 | F | R | W | D | V | R | MeF | S | N | W7N5Me | N | Tic | N | C | G |
| 137 | F | R | W | D | V | R | MeF | S | N | W | Q | Tic | N | C | G |
| 138 | F | R | W | D | V | R | MeF | S | N | W | Nmm | Tic | N | C | G |

**[Table 2D]**

| Table 2 (4 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 139 | F | R | W | D | V | R | MeF | S | N | W | N | Tic6C | N | C | G |
| 140 | F | R | W | D | V | R | MeF | S | N | W | N | Tic7H | N | C | G |
| 141 | F | R | W | D | V | R | MeF | S | N | W | N | Tic7C | N | C | G |
| 142 | F | R | W | D | V | R | MeF | S | N | W | N | Tic | Q | C | G |
| 143 | F | R | W | D | V | R | MeF | S | N | W | N | Tic | Nmm | C | G |
| 144 | F | R | W | D | V | R | MeF | S | N | W | N | Tic | Ndm | C | G |
| 145 | F | R | W7N | D | V | R | MeF | S | N | W7N | N | Tic | N | C | G |
| 146 | F | R | W7N5Me | D | v | R | MeF | S | N | W7N | N | Tic | N | C | G |
| 147 | F | R | Nal14N | D | v | R | MeF | S | N | W7N | N | Tic | N | C | G |
| 148 | F | R | W7N | D | Cbg | R | MeF | S | N | W7N | N | Tic | N | C | G |
| 149 | F | R | W7N | D | P | R | MeF | S | N | W7N | N | Tic | N | C | G |
| 150 | F | R | W7N | D | v | Kmor | MeF | S | N | W7N | N | Tic | N | C | G |
| 151 | F | R | W7N | D | v | R | MeF4COO | S | N | W7N | N | Tic | N | C | G |
| 152 | F | R | W7N | D | V | R | MeF3COO | S | N | W7N | N | Tic | N | C | G |
| 153 | F | R | W7N | D | V | R | MeF | E | N | W7N | N | Tic | N | C | G |
| 154 | F | R | W7N | D | V | R | MeF | S | E | W7N | N | Tic | N | C | G |
| 155 | F | R | W7N | D | V | R | MeF | S | N | W7N5Me | N | Tic | N | C | G |
| 156 | F | R | W7N | D | V | R | MeF | S | N | W7N | N | Tic7C | N | C | G |
| 157 | F | R | W7N | D | V | R | MeF | S | N | W7N | N | Tic | Nmm | C | G |
| 158 | F | R | W7N | D | V | R | MeF | S | N | W7N | N | Tic | D | C | G |

**[Table 2E]**

| Table 2 (5 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 159 | F | R | W7N | D | V | R | MeF | E | E | W7N | N | Tic | N | C | G |
| 160 | F | R | W7N | D | V | R | MeF | E | E | W7N | N | Tic | D | C | G |
| 161 | F | R | W7N | D | V | R | MeF3COO | E | E | W7N | N | Tic | D | C | G |
| 162 | F | R | W7N | D | V | Kmor | MeF3COO | E | E | W7N | N | Tic | D | C | G |
| 163 | F | R | W7N | D | V | R | MeF3COO | S | E | W7N | N | Tic | D | C | G |

### [Peptide Complex]

The peptide complex of the present invention contains a first peptide and has Wnt signal activation capability. Further, the peptide complex of the present invention may also have a cell proliferation capability derived from the Wnt signal activation capability.

The peptide complex is a peptide containing the first peptide and a peptide containing another peptide or compound, a peptide-containing compound, or a pharmaceutically acceptable salt thereof. The peptide complex may contain a single first peptide, or two or more (three or more or four or more) first peptides.

The peptide complex may also contain one or more partial peptides different from the first peptide. The peptide complex preferably has the first peptide and the partial peptides bonded linked via a linker. The peptide complex may be a homomultimer containing only peptides having the same amino acid sequence. The peptide complex may also be a heteromultimer containing peptides having different amino acid sequences. The peptide complex is preferably a heterodimer having a first peptide and a second peptide with different amino acid sequences, wherein the first peptide and the second peptide are bonded via a linker. As shown in the examples, by employing a peptide complex structure in which the first peptide and the second peptide are bonded via a linker, Wnt signal activation capability can be achieved.

The peptide complex of the present invention may be composed of a first peptide, a second peptide, and a linker that connects the first peptide and the second peptide. In such cases, the second peptide may be the same as, or different from, the first peptide.

One embodiment of the peptide complex of the present invention is a complex containing a first peptide and a second peptide, wherein the first peptide is the peptide of the first embodiment described above, and the second peptide is the peptide of the second embodiment described above.

The peptide complex of the present invention is preferably composed of the first peptide described above, the second peptide described above, and a linker that connects the first peptide and the second peptide.

The linker may be a structure that connects multiple peptides to each other in the peptide complex. Examples of the linker include an amino acid linkers (peptide linkers), chemical linkers, fatty acid linkers, nucleic acid linkers, and sugar chain linkers, and may also be, for example, a complex of a chemical linker and a peptide linker.

Examples of the chemical linkers include PEG (polyethylene glycol) linkers.

The PEG linker may be a linker composed of 1 to 36 ethylene glycol units.

Furthermore, the linker may also be a fatty acid linker containing a divalent chemical portion derived from a fatty acid. The amino acid (peptide) linker is a linker containing at least one amino acid, and examples include glycine-rich peptides such as peptides having the sequence [G-G-G-G-S]ₙ (wherein n is 1, 2, 3, 4, 5, or 6) as described in U.S. Patent No. 7,271,149, or serine-rich peptide linkers such as those described in U.S. Patent No. 5,525,491.

Addition of a linker may, without any particular limitations, change the physical properties (such as the solubility) of the peptide. Further, the linker may be a combination of the linkers described above. For example, the linker may have a structure in which glycine (G)-L-lysine (K) is bound as an amino acid linker, and a PEG linker is additionally bound to the side chain terminal of the Lys. Furthermore, the linker may have a structure in which an amino acid and PEG are bound alternately, such as PEG-amino acid-PEG. Here, PEG means a PEG linker.

Other examples of the linker include linkers in which 1 to 6 amino acids are added to a PEG linker portion. This type of linker may have a structure in which an amino acid is added to one end of the PEG linker portion, or may have a structure in which amino acids are added to both ends of the PEG linker portion. A preferred example of the amino acid added to the PEG moiety is L-lysine (K), but the present invention is not limited to L-lysine (K), and another amino acid may also be used.

The linker may be added to any position in the peptide. For example, the linker may be bonded to the L-cysteine (C) positioned at the C-terminal of the peptide, which is bonded to the 1st amino acid to form a cyclic structure, or may be bonded to an amino acid contained in the cyclic peptide. Although not a particular limitation, it is preferable that the linker is bonded to the L-cysteine (C) positioned at the C-terminal, or to the side chain of an amino acid included in the cyclic peptide.

For example, in the amino acid sequences represented by SEQ ID NOs: 1 to 163, the glycine (G) at position 15 can also be considered to be included in the linker. It is preferable that the glycine (G) at position 15 is included in the linker. In the case of cyclic peptides in which the 1st amino acid in each of the amino acid sequences represented by SEQ ID NOs: 1 to 163 is bonded to the L-cysteine (C) that represents the 14th amino acid, and which adopt a dimer structure via each of the structures represented by the formulas shown below (wherein these formulas include the 15th glycine added to the L-cysteine (C) that represents the 14th amino acid), the linker structures are the structures shown in the following formulas. The dimer is preferably a dimer in which the C-terminal of the first peptide and the C-terminal of the second peptide are bound via the linker.

### [Chemical formula 25A]

| Linker Structure Number | Linker Name | Structure |
|---|---|---|
| 1 | P08K_d | 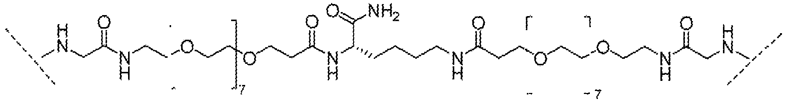 |
| 2 | click_ick_P04K/c_P04K | 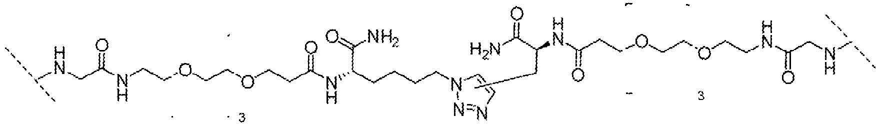 |
| 3 | click_P04K_OH | 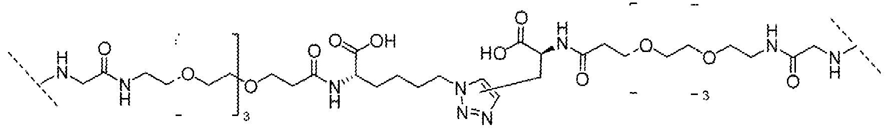 |
| 4 | click_P08K | 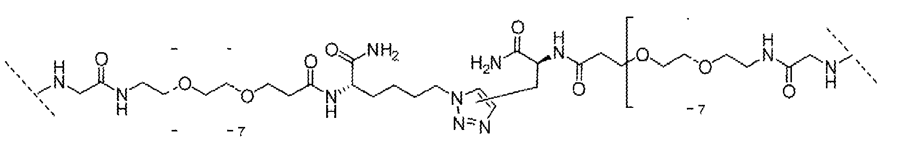 |

### [Chemical formula 25B]

| Linker Structure Number | Linker Name | Structure |
|---|---|---|
| 5 | click _P12K | 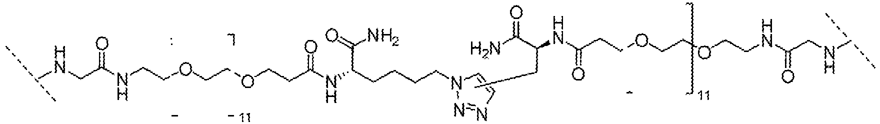 |
| 6 | click_P04K_branch | 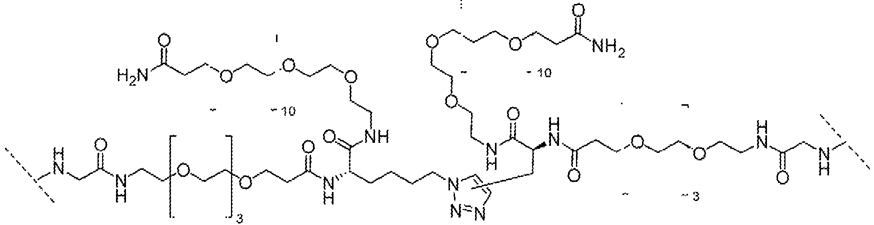 |
| 7 | click_P08K_branch | 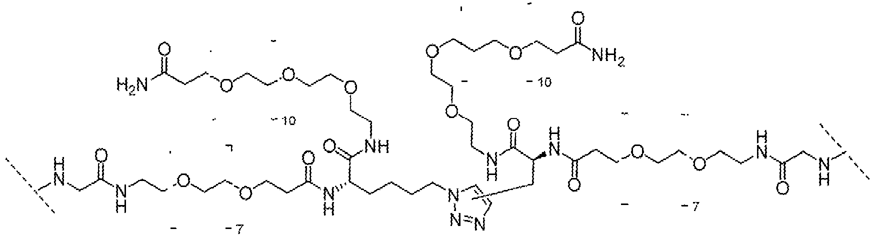 |

### [Chemical formula 25C]

| Linker Structure Number | Linker Name | Structure |
|---|---|---|
| 8 | NOF | 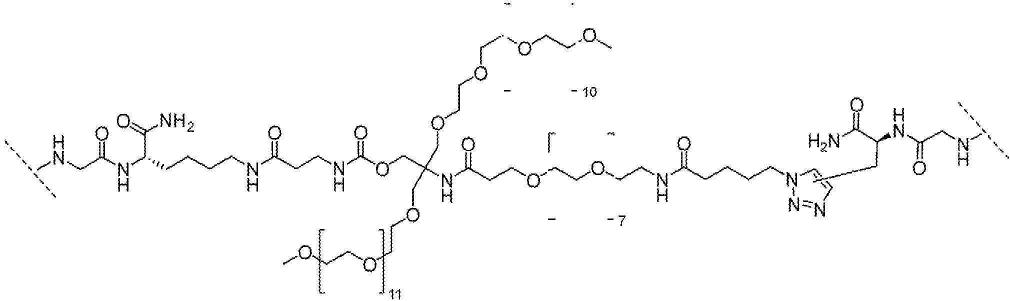 |
| 9 | tag | 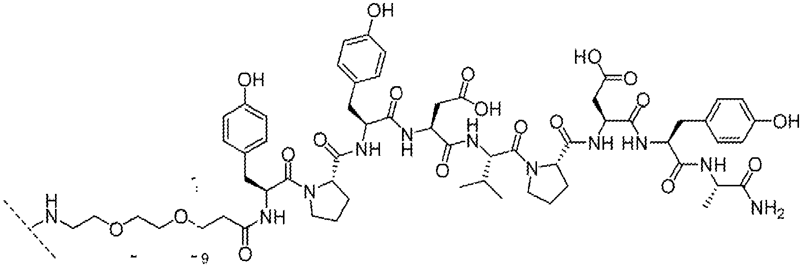 |

The linker is preferably selected from among the linkers shown in Table 3. In Table 3, -click- represents a bond formed by click chemistry.

### [Table 3]

**Table 3**

| |
|---|
| P08K_d |
| click_P04K / c_P04K |
| click_P04K_OH |
| click_P08K |
| click_P12K |
| click_P04K_branch |
| click_P08K_branch |
| NOF |
| tag |

One example of click chemistry is the Fusgen reaction (equation shown below), in which an alkyne and an azide compound undergo a cycloaddition reaction to form a 1,2,3-triazole ring.

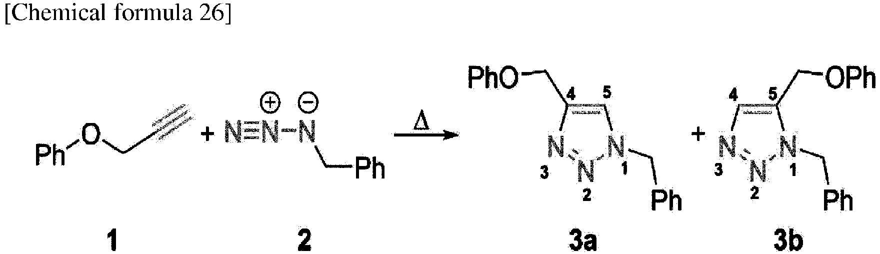

In the peptide complex of an embodiment of the present invention, it is preferable that the first peptide is a peptide having an amino acid sequence represented by formula A1 and the second peptide is a peptide having an amino acid sequence represented by formula B1.

A1: X1-X2-X3-X4-X5-X6-X7-X8-X9-X10-X11-X12-X13-X14

In formula A1:
X1 represents a D-phenylalanine (df) residue,
X2 represents an L-serine (S) residue or an L-2-aminoadipic acid (Hgl) residue,
X3 represents an L-aspartic acid (D) residue, L-citrulline (Cit) residue, or (2S)-2-amino-3-(oxan-4-yl)propanoic acid (Atp) residue,
X4 represents an L-isoleucine (I) residue,
X5 represents an L-arginine (R) residue or an L-citrulline (Cit) residue,
X6 represents an L-tyrosine (Y) residue,
X7 represents a methyl-L-phenylalanine (MeF) residue or an (S)-3-(2-carboxy-2-(methylamino)ethyl)benzoic acid (MeF3COO) residue,
X8 represents an L-glutamine (Q) residue, L-valine (V) residue, homo-L-tyrosine (Hty) residue, L-threonine (T) residue, L-citrulline (Cit) residue, or O-methyl-L-threonine (TMe) residue,
X9 represents an L-threonine residue (T),
X10 represents a methyl-L-phenylalanine (MeF) residue or an (S)-2-(methylamino)-3-(pyridin-4-yl)propanoic acid (Me4Py) residue,
X11 represents an L-isoleucine (I) residue or an L-tyrosine (Y) residue,
X12 represents an L-valine (V) residue or a methyl-L-valine (MeV) residue,
X13 represents a glycine (G) residue or a D-serine (ds) residue, and
X14 represents an L-cysteine (C) residue.

B1: Y1-Y2-Y3-Y4-Y5-Y6-Y7-Y8-Y9-Y10-Y11-Y12-Y13-Y14

In formula B1:
Y1 represents an L-phenylalanine (F) residue,
Y2 represents an L-arginine (R) residue,
Y3 represents an L-tryptophan (W) residue or an (S)-2-amino-3-(1H-pyrrolo[2,3-b]pyridin-3-yl)propanoic acid (W7N) residue,
Y4 represents an L-aspartic acid (D) residue,
Y5 represents an L-valine (V) residue,
Y6 represents an L-arginine (R) residue or an (S)-2-amino-6-morpholinohexanoic acid (Kmor) residue,
Y7 represents a methyl-L-phenylalanine (MeF) residue or an (S)-3-(2-carboxy-2-(methylamino)ethyl)benzoic acid (MeF3COO) residue,
Y8 represents an L-serine (S) residue or an L-glutamic acid (E) residue,
Y9 represents an L-asparagine (N) residue, N6-(4-(carboxymethyl)piperazine-1-carbonyl)-L-lysine (KCOpipzaa) residue, or L-glutamic acid (E) residue,
Y10 represents an L-tryptophan (W) residue or an (S)-2-amino-3-(1H-pyrrolo[2,3-b]pyridin-3-yl)propanoic acid (W7N) residue,
Y11 represents an L-asparagine (N) residue,
Y12 represents an (S)-1,2,3,4-tetrahydroisoquinoline-3-carboxylic acid (Tic) residue,
Y13 represents an L-asparagine (N) residue or an L-aspartic acid (D) residue, and
Y14 represents an L-cysteine (C) residue.

The peptide complex of an embodiment of the present invention preferably activates Wnt signaling.

### [Pharmaceutical Composition]

Yet another embodiment of the present invention relates to a pharmaceutical composition.

One aspect of the pharmaceutical composition of an embodiment of the present invention is a pharmaceutical composition containing at least one type of substance selected from the group consisting of the peptide described above, and pharmaceutically acceptable salts, esters and solvates thereof (hereinafter, for the sake of simplicity, also referred to as simply "the peptide of the present invention"), another aspect is a pharmaceutical composition containing at least one type of substance selected from the group consisting of the peptide complex described above, and pharmaceutically acceptable salts, esters and solvates thereof (hereinafter, for the sake of simplicity, also referred to as simply "the peptide complex of the present invention"), and yet another aspect is a pharmaceutical composition containing at least one type of substance selected from the group consisting of the peptide and peptide complex described above, and pharmaceutically acceptable salts, esters and solvates thereof.

The pharmaceutical composition preferably contains the peptide of the present invention or the peptide complex of the present invention in an effective amount as an active ingredient.

Target diseases for the above pharmaceutical composition include any disease caused by, exacerbated by, or otherwise associated with increases or decreases in the expression or activity of Frizzled receptors (Fzd2, Fzd7, Fzd8) and/or LRPs (LRP5, LRP6), or any disease caused by, exacerbated by, or otherwise associated with increases or decreases in Wnt signaling or any other intracellular signaling cascade activated via the above Frizzled receptors and/or the LRP. Examples of these diseases include, but are not limited to, bone metabolic diseases such as osteoporosis (see Manolagas, S.C., "Wnt signaling and osteoporosis," Maturitas, 2014, Vol. 78, No. 3, pp. 233 to 237), inflammatory bowel disease (see Moparthi, L. et al., "Wnt signaling in intestinal inflammation," Differentiation, 2019, Vol. 108, pp. 24 to 32), and cancer diseases such as breast cancer, colon cancer, lung cancer, and stomach cancer (see Park, W.-J. et al., "A New Wave of Targeting 'Undruggable' Wnt Signaling for Cancer Therapy: Challenges and Opportunities", Cells, 2023, Vol. 12, p. 1110).

There are no particular limitations on the administration form of the pharmaceutical composition, and either oral administration or parenteral administration may be used. Examples of parenteral administration include administration by injection such as intramuscular injection, intravenous injection and subcutaneous injection, transdermal administration, and transmucosal administration (including nasal, oral, ocular, pulmonary, vaginal and rectal administration).

The peptide in the pharmaceutical composition may be subjected to various modifications, with due consideration of the susceptibility of the peptide to metabolism and excretion. For example, polyethylene glycol (PEG) or sugar chains may be added to the polypeptide to lengthen the blood residence time and reduce antigenicity. Further, the polypeptide may be encapsulated in a biodegradable polymer such as polylactic acid glycol (PLGA), porous hydroxyapatite, liposomes, surface-modified liposomes, emulsions prepared with unsaturated fatty acids, nanoparticles, or nanospheres or the like, with this polymer functioning as a sustained-release base. In the case of transdermal administration, a weak electric current can be applied to the skin surface to enable permeation of the stratum corneum (the iontophoresis method).

The pharmaceutical composition may simply employ the active ingredient as is, or may be a formulation produced by adding pharmaceutically acceptable carriers, excipients, and additives and the like. Examples of possible forms for the composition include liquids (such as injections), dispersions, suspensions, tablets, pills, powders, suppositories, powders, fine granules, granules, capsules, syrups, lozenges, inhalants, ointments, eye drops, nasal drops, ear drops, and poultices.

The formulation mentioned above can be prepared by conventional methods, using, for example, appropriate amounts of excipients, binders, disintegrants, lubricants, solubilizers, dissolution assistants, colorants, flavoring agents, stabilizers, emulsifiers, absorption enhancers, surfactants, pH adjusters, preservatives, and antioxidants and the like.

Examples of components that may be used in the above formulation include, but are not limited to, purified water, saline, phosphate buffer, dextrose, glycerol, pharmaceutically acceptable organic solvents such as ethanol, animal and vegetable oils, lactose, mannitol, glucose, sorbitol, crystalline cellulose, hydroxypropyl cellulose, starch, corn starch, silicic anhydride, magnesium aluminum silicate, collagen, polyvinyl alcohol, polyvinylpyrrolidone, carboxyvinyl polymers, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, human serum albumin, trehalose, and polysorbate.

The absorption enhancers mentioned above can be used to improve the absorption of drugs that exhibit poor absorption. Examples of absorption enhancers that may be used include surfactants such as polyoxyethylene lauryl ethers, sodium lauryl sulfate, and saponin; bile salts such as glycocholic acid, deoxycholic acid, and taurocholic acid; chelating agents such as EDTA and salicylic acids; fatty acids such as caproic acid, capric acid, lauric acid, oleic acid, linoleic acid, and mixed micelles; as well as enamine derivatives, N-acyl collagen peptides, N-acyl amino acids, cyclodextrins, chitosans, and nitric oxide donors.

The pills or tablets mentioned above may be coated with a sugar, or a gastric or enteric coating material. The injections may contain injection distilled water, physiological saline, propylene glycol, polyethylene glycol, vegetable oil, or an alcohol or the like. Furthermore, wetting agents, emulsifiers, dispersants, stabilizers, solubilizers, dissolution assistants, and preservatives and the like may also be added.

The pharmaceutical composition of the present invention may be administered in combination with other drugs or treatment methods useful for treating the diseases described above.

The dosage used when the pharmaceutical composition of the present invention is administered to mammals (for example, humans, mice, rats, guinea pigs, rabbits, dogs, horses, monkeys, pigs, or sheep), and particularly to humans, varies depending on the symptoms, the age, gender, weight and sensitivity of the patient, the administration method, the administration interval, the type of active ingredient, and the type of formulation, and although there are no particular limitations, the dosage may be, for example, within a range from 30 µg to 1,000 mg, from 100 µg to 500 mg, or from 100 µg to 100 mg, administered either once or in a plurality of separate doses. In the case of injection, a dose within a range from 1 µg/kg to 3,000 µg/kg or from 3 µg/kg to 1,000 µg/kg may be administered once or in a plurality of doses, in accordance with the weight of the patient.

### [Composition for Cell Culture]

Yet another embodiment of the present invention relates to a composition for cell culture that contains the peptide complex described above and can be used in cell culture.

The above peptide complex has Wnt signal activation capability, and therefore can also be used as a medium reagent or additive for cell culture, preferably for mammalian cells, and more preferably for human cells. The medium reagent or additive for cell culture may be a medium reagent or additive used for culturing cells for the production of cell-cultured meat.

Furthermore, the peptide complex can also be used as a reagent or additive for producing gastrointestinal organoids, and lung organoids and the like. The organoids may be produced by inducing differentiation from pluripotent stem cells into endoderm and then into cells of various tissues, although production is not limited to this method. Wnt signaling plays an important role in this differentiation induction, and therefore the peptide complex described above can be used in the step of inducing differentiation from pluripotent stem cells into endoderm and into various cells, but is not limited to this use (see Yiangou, L., et al., "Human Pluripotent Stem Cell-Derived Endoderm for Modeling Development and Clinical Applications," Cell Stem Cell, 2018, Vol. 22, No. 4, pp. 485 to 499).

There are no particular limitations on the medium, and any medium used for culturing cells or tissues may bae used. The medium may be a serum medium, but is preferably a serum-free medium or a low-serum medium.

The culture medium additive may exist in the form of a solution or a dried solid (such as a solid or powder). In the case of a solution form, the solution may be used, as is, as the culture medium, or may be diluted with a solvent, and the additives described above added to the solution, as required, before use as the culture medium. Examples of the solvent used in those cases where dilution is conducted include water, buffer solutions, physiological saline, and other media used for various cell and tissue cultures, and one of these solvents may be used alone, or a combination of two or more solvents may be used.

In those cases where the culture medium additive exists as a dried solid, a solution prepared by dissolution in, for example, a solvent such as water, a buffer solution, physiological saline, or any other medium used for various cell or tissue cultures, and then addition of the additives described above may be used as the culture medium.

The amount of the peptide complex of the present invention within the medium for culturing cells or tissues, or in a cell medium obtained therefrom, expressed, for example, as a final concentration relative to the total volume of the composition or the total volume of the medium, may be within a range from about 0.01 to about 10,000 nmol/L, preferably from about 0.1 to about 1,000 nmol/L, more preferably from about 0.5 to about 1,000 nmol/L, and even more preferably from about 1 to about 100 nmol/L.

### [Composition for Medical Use, Diagnostic Use or Research Use]

Yet another embodiment of the present invention relates to a composition for medical use, diagnostic use or research use. This composition for medical use, diagnostic use or research use according to an embodiment of the present invention is a pharmaceutical composition containing at least one substance selected from the group consisting of the peptide described above and pharmaceutically acceptable salts, esters, and solvates thereof (hereinafter, for the sake of simplicity, also referred to as simply "the peptide of the present invention"), whereas another aspect is a composition containing at least one substance selected from the group consisting of the peptide complex described above and pharmaceutically acceptable salts, esters, or solvates thereof (hereinafter, for the sake of simplicity, also referred to as simply "the peptide complex of the present invention").

### <Composition for Medical Use>

The composition for medical use according to an embodiment of the present invention (hereinafter also referred to as simply "the medical composition") contains at least one substance selected from the group consisting of the peptide and the peptide complex of the present invention.

The medical composition preferably contains the peptide of the present invention or the peptide complex of the present invention in an effective amount as an active ingredient.

Target diseases for the above medical composition include any disease caused by, exacerbated by, or otherwise associated with increases or decreases in the expression or activity of Frizzled receptors (Fzd2, Fzd7, Fzd8) and/or LRPs (LRP5, LRP6), or any disease caused by, exacerbated by, or otherwise associated with increases or decreases in Wnt signaling or any other intracellular signaling cascade activated via the above Frizzled receptors and/or the LRP. Examples of these diseases include, but are not limited to, bone metabolic diseases such as osteoporosis (see Manolagas, S.C., "Wnt signaling and osteoporosis," Maturitas, 2014, Vol. 78, No. 3, pp. 233 to 237), inflammatory bowel disease (see Moparthi, L. et al., "Wnt signaling in intestinal inflammation," Differentiation, 2019, Vol. 108, pp. 24 to 32), and cancer diseases such as breast cancer, colon cancer, lung cancer, and stomach cancer (see Park, W.-J. et al., "A New Wave of Targeting 'Undruggable' Wnt Signaling for Cancer Therapy: Challenges and Opportunities", Cells, 2023, Vol. 12, p. 1110).

There are no particular limitations on the administration form of the medical composition, and either oral administration or parenteral administration may be used. Examples of parenteral administration include administration by injection such as intramuscular injection, intravenous injection and subcutaneous injection, transdermal administration, and transmucosal administration (including nasal, oral, ocular, pulmonary, vaginal and rectal administration).

The peptide in the medical composition may be subjected to various modifications, with due consideration of the susceptibility of the peptide to metabolism and excretion. For example, polyethylene glycol (PEG) or sugar chains may be added to the polypeptide to lengthen the blood residence time and reduce antigenicity. Further, the polypeptide may be encapsulated in a biodegradable polymer such as polylactic acid glycol (PLGA), porous hydroxyapatite, liposomes, surface-modified liposomes, emulsions prepared with unsaturated fatty acids, nanoparticles, or nanospheres or the like, with this polymer functioning as a sustained-release base. In the case of transdermal administration, a weak electric current can be applied to the skin surface to enable permeation of the stratum corneum (the iontophoresis method).

The medical composition may simply employ the active ingredient as is, or may be a formulation produced by adding pharmaceutically acceptable carriers, excipients, and additives and the like. Examples of possible forms for the composition include liquids (such as injections), dispersions, suspensions, tablets, pills, powders, suppositories, powders, fine granules, granules, capsules, syrups, lozenges, inhalants, ointments, eye drops, nasal drops, ear drops, and poultices.

The formulation mentioned above can be prepared by conventional methods, using, for example, appropriate amounts of excipients, binders, disintegrants, lubricants, solubilizers, dissolution assistants, colorants, flavoring agents, stabilizers, emulsifiers, absorption enhancers, surfactants, pH adjusters, preservatives, and antioxidants and the like.

Examples of components that may be used in the above formulation include, but are not limited to, purified water, saline, phosphate buffer, pharmaceutically acceptable organic solvents such as dextrose, glycerol and ethanol, animal and vegetable oils, lactose, mannitol, glucose, sorbitol, crystalline cellulose, hydroxypropyl cellulose, starch, corn starch, silicic anhydride, magnesium aluminum silicate, collagen, polyvinyl alcohol, polyvinylpyrrolidone, carboxyvinyl polymers, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, human serum albumin, trehalose, and polysorbate.

The absorption enhancers mentioned above can be used to improve the absorption of drugs that exhibit poor absorption. Examples of absorption enhancers that may be used include surfactants such as polyoxyethylene lauryl ethers, sodium lauryl sulfate, and saponin; bile salts such as glycocholic acid, deoxycholic acid, and taurocholic acid; chelating agents such as EDTA and salicylic acids; fatty acids such as caproic acid, capric acid, lauric acid, oleic acid, linoleic acid, and mixed micelles; as well as enamine derivatives, N-acyl collagen peptides, N-acyl amino acids, cyclodextrins, chitosans, and nitric oxide donors.

The pills or tablets mentioned above may be coated with a sugar, or a gastric or enteric coating material. The injections may contain injection distilled water, physiological saline, propylene glycol, polyethylene glycol, vegetable oil, or an alcohol or the like. Furthermore, wetting agents, emulsifiers, dispersants, stabilizers, solubilizers, dissolution assistants, and preservatives and the like may also be added.

The medical composition of the present invention may be administered in combination with other drugs or treatment methods useful for treating the diseases described above.

The dosage used when the medical composition of the present invention is administered to mammals (for example, humans, mice, rats, guinea pigs, rabbits, dogs, horses, monkeys, pigs, or sheep), and particularly to humans, varies depending on the symptoms, the age, gender, weight and sensitivity of the patient, the administration method, the administration interval, the type of active ingredient, and the type of formulation, and although there are no particular limitations, the dosage may be, for example, within a range from 30 µg to 1,000 mg, from 100 µg to 500 mg, or from 100 µg to 100 mg, administered either once or in a plurality of separate doses. In the case of injection, a dose within a range from 1 µg/kg to 3,000 µg/kg or from 3 µg/kg to 1,000 µg/kg may be administered once or in a plurality of doses, in accordance with the weight of the patient.

### <Composition for Diagnostic Use>

The composition for diagnostic use according to an embodiment of the present invention (hereinafter also referred to as simply "the diagnostic composition") contains at least one substance selected from the group consisting of the peptide and the peptide complex of the present invention.

The peptides and peptide complex bind to Frizzled receptors (Fzd2, Fzd7, Fzd8) and/or LRPs (LRP5, LRP6). Accordingly, the peptide and peptide complex can also be used as diagnostic agents for detecting these Frizzled receptors and/or LRPs. The diagnostic agent may be a detection agent for detecting the expression level of a Frizzled receptor and/or LRP, and when used as a detection agent, the peptide or peptide complex of the present invention may be labeled to enable detection. In this manner, the peptide or peptide complex, or a composition containing the peptide or peptide complex, can be used as a diagnostic agent for detecting Frizzled receptors and/or LRPs.

### <Composition for Research Use>

The composition for research use according to an embodiment of the present invention (hereinafter also referred to as simply "the research composition") contains at least one substance selected from the group consisting of the peptide and the peptide complex of the present invention.

The peptide and peptide complex described above bind to Frizzled receptors (Fzd2, Fzd7, Fzd8) and/or LRPs (LRP5, LRP6). Accordingly, they can be used favorably in research involving these Frizzled receptors and/or LRPs.

The research composition of an embodiment of the present invention is preferably used in a test method for testing at least one of the following properties for the peptide or the peptide complex, and/or a complex containing a substance further bound to the peptide or the peptide complex via a linker.
a) Solubility in solvents
b) Binding ability to Frizzled receptors (Fzd2, Fzd7, Fzd8) and/or LRPs (LRP5, LRP6)
c) Toxicity to cells and/or tissues
d) Toxicity to test animals

In relation to the test methods described above, testing of the solubility in a solvent of the peptide or the peptide complex, and/or a complex containing a substance further bound to the peptide or the peptide complex via a linker, may involve measurement of the solubility. There are no particular limitations on the solvent used in the solubility measurement, and the solvent may be selected freely in accordance with the intended purpose. Furthermore, a known method for measuring the solubility may be selected appropriately in accordance with the type of solvent.

Testing the binding ability to a Frizzled receptor and/or LRP mentioned above may be a measurement of the binding ability to the Frizzled receptor and/or LRP, and known methods such as, but not limited to, surface plasmon resonance (SPR) assay, Scatchard analysis, and/or competitive binding assays such as radioimmunoassay (RIA), enzyme immunoassay (EIA), and sandwich competition assay can be used favorably.

Testing for toxicity to cells and/or tissues may employ a conventional toxicity evaluation test using cells and/or tissues, and may be implemented, for example, using an vitro method. The cells and tissues may be, but are not limited to, the types of cells and/or tissues typically used in toxicity evaluation tests for drugs.

The method used for testing toxicity to test animals may be a conventional toxicity evaluation test using test animals. There are no particular limitations on the test animals, and any typically used animal may be used, including mice, rats, guinea pigs, gerbils, hamsters, ferrets, rabbits, dogs, cats, pigs, goats, horses, cows, birds (such as chickens and quails), monkeys, and non-human primates (such as cynomolgus monkeys, marmosets, and rhesus monkeys). The above toxicity evaluation tests may be, but are not limited to, tests relating to safety that are typically conducted in non-clinical trials of drugs, and examples include general toxicity tests (single-dose toxicity tests and repeated-dose toxicity tests), genotoxicity tests (Ames tests, chromosomal aberration tests, and in vitro micronucleus tests), carcinogenicity tests, reproductive and developmental toxicity tests (ICH-I, II, and III), local irritation tests (such as eye irritation tests and skin irritation tests), other toxicity tests (skin sensitization tests, phototoxicity tests, and antigenicity tests), and chemical analyses and bioanalyses (TK and PK).

### [Nucleic Acid]

Yet another embodiment of the present invention is a nucleic acid that encodes the peptide or peptide described above.

The nucleic acid of this embodiment of the present invention may be either natural or unnatural. Further, the term "nucleic acid" includes, but is not limited to, DNA, RNA, and chimeras thereof. The nucleic acid of this embodiment can be designed and produced using conventional methods based on the amino acid sequence of the peptide or peptide complex.

### [Peptide-Drug Conjugate (PDC)]

Yet another embodiment of the present invention relates to a conjugate (peptide-drug conjugate, PDC) containing the peptide or peptide complex described above, a target substance for which delivery to a Frizzled receptor (Fzd2, Fzd7, Fzd8) and/or LRP (LRP5, LRP6) is required, and a linker for bonding the peptide or peptide complex to the substance.

The peptide binds to the Frizzled receptor or LRP. The peptide complex binds to the Frizzled receptor and the LRP. Accordingly, the peptide or peptide complex can deliver the substance to the Frizzled receptor and/or LRP.

The substance mentioned above may be any substance that a person skilled in the art requires to be delivered to the Frizzled receptor and/or the LRP.

There are no particular limitations on this substance, but examples include compounds, peptides, RI (radioisotopes), proteins, nucleic acids, and molecules used in DDS (drug delivery systems).

There are no particular limitations on the above compounds, but low molecular weight compounds or medium molecular weight compounds are preferred, and examples include known low molecular weight drugs.

There are no particular limitations on the above peptides, but peptides that bind to a target in the body and exert some form of effect are preferred, such as cyclic peptides.

There are no particular limitations on the above RI, and any compound labeled with a radioisotope, such as a low molecular weight compound, medium molecular weight compound or antibody that has been labeled with a radioisotope may be used, and examples include compounds used in PET (positron emission tomography) examinations.

There are no particular limitations on the above proteins, and any protein that exhibits a useful function in the body, such as an antibody or an enzyme may be used. Examples include enzymes used in enzyme replacement therapy.

There are no particular limitations on the above nucleic acids, which may be DNA, RNA or chimeras thereof. Examples include nucleic acid drugs.

There are no particular limitations on the above molecules used in DDS, but conventional molecules used in DDS such as liposomes or micelles are preferred. The molecule used in DDS may also contain a compound such as a drug inside the molecule.

Further, the substance to be delivered to the Frizzled receptor and/or LRP may be a complex of the substances described above.

### Examples

The present invention is described below in further detail based on a series of examples, but the present invention is not limited by the following examples. Persons skilled in the art will easily be able to add modifications or alterations based on the above description, and these modifications and alterations are also deemed to be included within the technical scope of the present invention.

### [Chemical Syntheses]

All the raw materials, building blocks, reagents, acids, bases, solid-phase resins, and solvents used in the chemical syntheses in the examples described below were either commercially available products or were able to be synthesized by a person skilled in the art using organic chemistry techniques. Unless specifically stated otherwise, commercially available products were used without modification as the amino acids containing protective groups.

Peptide chain elongation on solid-phase resins was conducted using the resins described in each of the following examples as starting materials, under typically used peptide coupling reaction conditions and Fmoc removal reaction conditions. The reactions were carried out using an automated peptide synthesizer, such as a Syro I or Syro II manufactured by Biotage Inc., or a Liberty Blue, Liberty Blue HT12 or Liberty Prime manufactured by CEM Corporation, in accordance with the manual provided by the manufacturer.

The resins used were NovaPEG Rink Amide resin, HMPB-MBHA resin, or Seiber Amide resin, with the amounts used varying within a range from 5 mg to 2 g depending on the peptide.

The reagent cocktail used for side chain deprotection and cleavage from the solid-phase resin was used in an amount within a range from 4 mL to 50 mL depending on the peptide, and was a solution with the following composition.
A: TFA/H₂O/TIS/DODT (92.5/2.5/2.5/2.5)
B: TFA/H₂O/TIS/DODT (90/2.5/2.5/5)

Examples of typical Fmoc amino acids that were used are listed in Table 4, with side chain protective groups shown in the parentheses.

**[Table 4A]**

| Table 4 (1 of 4) | |
|---|---|
| Fmoc Amino Acid | CAS Registration Number |
| Fmoc-3Py5Ph-OH | 2973755-13-2 |
| Fmoc-4Py-OH | 169555-95-7 |
| Fmoc-Ahp-OH | 1197020-22-6 |
| Fmoc-alT(tBu)-OH | 201481-37-0 |
| Fmoc-Arg(Pbf)-OH | 154445-77-9 |
| Fmoc-Asn(Trt)-OH | 132388-59-1 |
| Fmoc-Asp(Mpe)-OH | 180675-08-5 |
| Fmoc-Atb-OH | 139551-74-9 |
| Fmoc-Atp-OH | 368866-34-6 |
| Fmoc-Cbg-OH | 1391630-31-1 |
| Fmoc-Cha-OH | 135673-97-1 |
| Fmoc-Chg-OH | 161321-36-4 |
| Fmoc-Cit-OH | 133174-15-9 |
| Fmoc-Cys(Trt)-OH | 103213-32-7 |
| Fmoc-d3py-OH | 142994-45-4 |
| Fmoc-d4py-OH | 205528-30-9 |
| Fmoc-da-OH | 79990-15-1 |
| Fmoc-dcit-OH | 200344-33-8 |
| Fmoc-df3OMe-OH | 917099-04-8 |
| Fmoc-df4F-OH | 177966-64-2 |
| Fmoc-df4OMe-OH | 201335-88-8 |
| Fmoc-df-OH | 86123-10-6 |

**[Table 4B]**

| Table 4 (2 of 4) | |
|---|---|
| Fmoc Amino Acid | CAS Registration Number |
| Fmoc-ds(Trt)-OH | 212688-51-2 |
| Fmoc-dy(tBu)-OH | 118488-18-9 |
| Fmoc-F3COO(tBu)-OH | 210282-33-0 |
| Fmoc-F3Me-OH | 211637-74-0 |
| Fmoc-F4aao(tBu)-OH | 181951-92-8 |
| Fmoc-F4C-OH | 175453-08-4 |
| Fmoc-F4F-OH | 169243-86-1 |
| Fmoc-F4Me-OH | 199006-54-7 |
| Fmoc-G4pipaa(tBu)-OH | 2973753-14-7 |
| Fmoc-Gln(Trt)-OH | 132327-80-1 |
| Fmoc-Glu(tBu)-OH | 71989-18-9 |
| Fmoc-Gly(Dmb)-OH | 166881-42-1 |
| Fmoc-Gly-OH | 29022-11-5 |
| Fmoc-Gpra-OH | 198561-07-8 |
| Fmoc-Gthp-OH | 368866-31-3 |
| Fmoc-Hcit-OH | 201485-17-8 |
| Fmoc-Hgl(tBu)-OH | 159751-47-0 |
| Fmoc-Hse(Trt)-OH | 111061-55-3 |
| Fmoc-Hty(tBu)-OH | 204384-69-0 |
| Fmoc-Hw7N-OH | 2350159-31-6 |
| Fmoc-Ile-OH | 71989-23-6 |
| Fmoc-KCOpipzaa(tBu)-OH | 2680607-34-3 |
| Fmoc-Kmor-OH | 2349553-17-7 |
| Fmoc-KN3-OH | 159610-89-6 |

**[Table 4C]**

| Table 4 (3 of 4) | |
|---|---|
| Fmoc Amino Acid | CAS Registration Number |
| Fmoc-Me3Py-OH | 1979173-93-7 |
| Fmoc-Me4Py-OH | 2381854-90-4 |
| Fmoc-MeAtp-OH | 2973755-43-8 |
| Fmoc-MeChg-OH | 925240-97-7 |
| Fmoc-MeF3C-OH | 1446478-28-9 |
| Fmoc-MeF3COO(tBu)-OH | 2973754-48-0 |
| Fmoc-MeF3Me-OH | 2642331-20-0 |
| Fmoc-MeF4C-OH | 1217716-50-1 |
| Fmoc-MeF4COO(tBu)-OH | 2708283-41-2 |
| Fmoc-MeF4Me-OH | 227616-20-8 |
| Fmoc-MeF-OH | 77128-73-5 |
| Fmoc-MeN(Trt)-OH | 941296-80-6 |
| Fmoc-MeNle-OH | 112883-42-8 |
| Fmoc-MeR(Pbf)-OH | 913733-27-4 |
| Fmoc-MeV-OH | 84000-11-3 |
| Fmoc-MeW(Boc)-OH | 197632-75-0 |
| Fmoc-MeY(tBu)-OH | 133373-24-7 |
| Fmoc-Nal14N-OH | 1998513-62-4 |
| Fmoc-Nal1-OH | 96402-49-2 |
| Fmoc-Ncit-OH | 2973754-85-5 |
| Fmoc-Ndm-OH | 138585-02-1 |
| Fmoc-Nmm-OH | 149204-93-3 |
| Fmoc-OrnAc-OH | 172690-48-1 |
| Fmoc-P-OH | 71989-31-6 |

**[Table 4D]**

| Table 4 (4 of 4) | |
|---|---|
| Fmoc Amino Acid | CAS Registration Number |
| Fmoc-PEG4c-OH | 557756-85-1 |
| Fmoc-PEG8c-OH | 756526-02-0 |
| Fmoc-PEG12c-OH | 1952360-91-6 |
| Fmoc-Phe-OH | 35661-40-6 |
| Fmoc-Ser(tBu)-OH | 71989-33-8 |
| Fmoc-Ser(Trt)-OH | 111061-56-4 |
| Fmoc-Tbg-OH | 132684-60-7 |
| Fmoc-Thr(tBu)-OH | 71989-35-0 |
| Fmoc-Thr(Trt)-OH | 133180-01-5 |
| Fmoc-Tic6C-OH | N/A(US63/460619) |
| Fmoc-Tic7C-OH | 2411591-51-8 |
| Fmoc-Tic7H-OH | 178432-49-0 |
| Fmoc-Tic-OH | 136030-33-6 |
| Fmoc-TMe-OH | 928063-81-4 |
| Fmoc-Trp(Boc)-OH | 143824-78-6 |
| Fmoc-Tyr(tBu)-OH | 71989-38-3 |
| Fmoc-Val-OH | 68858-20-8 |
| Fmoc-W1aa(tBu)-OH | 1629658-33-8 |
| Fmoc-W1Me7N-OH | 1813528-10-7 |
| Fmoc-W5N(Boc)-OH | 2973753-21-6 |
| Fmoc-W6N(Boc)-OH | N/A(US63/460619) |
| Fmoc-W7N5Me-OH | N/A(US63/460619) |
| Fmoc-W7N-OH | 737007-45-3 |

Unless specifically stated otherwise, the method used for purifying each of the obtained crude peptides employed one of the reversed phase separation and purification devices listed below in A), B), C), D) or E).
A) Shimadzu prep-HPLC system (LC-20AP, SPD-M20A, CTO-20AC and CBM-20A)
B) Waters AutoPurification System
C) Waters AutoPurification System with SQD
D) Waters Preparative HPLC System
E) YMC Contichrom CUBE

Unless specifically stated otherwise, the column used was one of the columns a) to k) listed below.
a) Jeanious One -Column 20 mm I.D. × 150 mmL
b) Kinetex EVO C18 5 µm 21.2 × 150 mm
c) Waters XBridge C18 5 µm 19 × 150 mm
d) Waters XBridge C18 5 µm 30 × 150 mm
e) Waters XBridge C18 5 µm 50 × 150 mm
f) Waters XBridge C18 5 µm 50 × 250 mm
g) Waters XSelect C18 30 × 150 mm
h) Waters XSelect C18 5 µm 19 × 150 mm
i) Waters XSelect C18 5 µm 30 × 150 mm
j) Waters XSelect CSH Prep C18 5 µm OBD 50 × 250 mm
k) YMC-Triart Prep C18-S 10 µm 10 × 250 mm

Determination of the structure of each of the chemically synthesized peptides was made by using ESI-MS(+) mass spectrometry to confirm the molecular weight that had been calculated based on the amino acids used in the target sequence and other building blocks that were used as necessary. "ESI-MS(+)" refers to electrospray ionization mass spectrometry conducted in positive ion mode. Detected masses were reported in "m/z" units. Compounds with molecular weights greater than about 1,000 were frequently detected as multivalent ions.

### Basic Analytical Device and Basic Conditions

Unless specifically stated otherwise, mass spectral analyses of the peptides synthesized in the following examples were conducted using the basic analytical devices and basic conditions described below. Analyses were conducted with the gradient B (%) set to any of conditions w/x/y/z.

### (Basic Analytical Device)

AutoPurification System SQD2 single quadruple mass spectrometer, manufactured by Waters Corporation

### (Basic Conditions)

Column: Kinetex EVO C18 (1.7 µm, 2.1 × 50 mm, 100 Å)
Column temperature: 60°C
Mobile phase A: 0.025% TFA in H₂O
Mobile phase B: 0.025% TFA in MeCN
Flow rate: 0.6 mL/min
Wavelength: 220 nm
Gradient B (%): w) 5 to 95% / 2.10 min, 95 to 95% / 0.75 min

### (Basic Analytical Device)

Shimadzu LC/MS system (LC-20ADXR, CTO-20AC, SPD-M20A, SIL-20AXR, CBM-20A and LCMS-2020)

### (Basic Conditions)

Column: Kinetex EVO C18 (2.6 µm, 2.1 × 150 mm, 100 Å)
Column temperature: 60°C
Mobile phase A: 0.025% TFA in H₂O
Mobile phase B: 0.025% TFA in MeCN
Flow rate: 0.5 mL/min
Wavelength: 225 nm PDA
Gradient B (%):
   x) 5 to 45% / 7.15 min, 45 to 95% / 0.3 min, 95 to 95% / 1.55 min
   y) 20 to 60% / 7.15 min, 60 to 95% / 0.3 min, 95 to 95% / 1.55 min
   z) 20 to 60% / 54.15 min, 60 to 95% / 0.3 min, 95 to 95% / 1.55 min

### [Example 1]

Synthesis of Peptide Complex (Peptide Complex having Linker Structure Number 9 added to Peptide SEQ ID NO: 66)

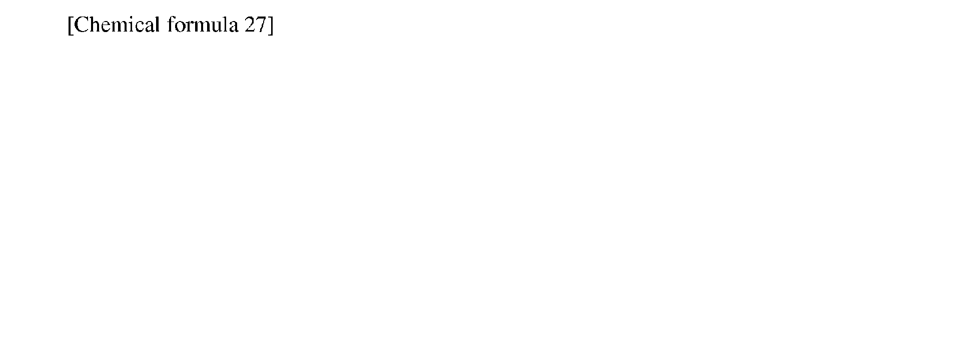

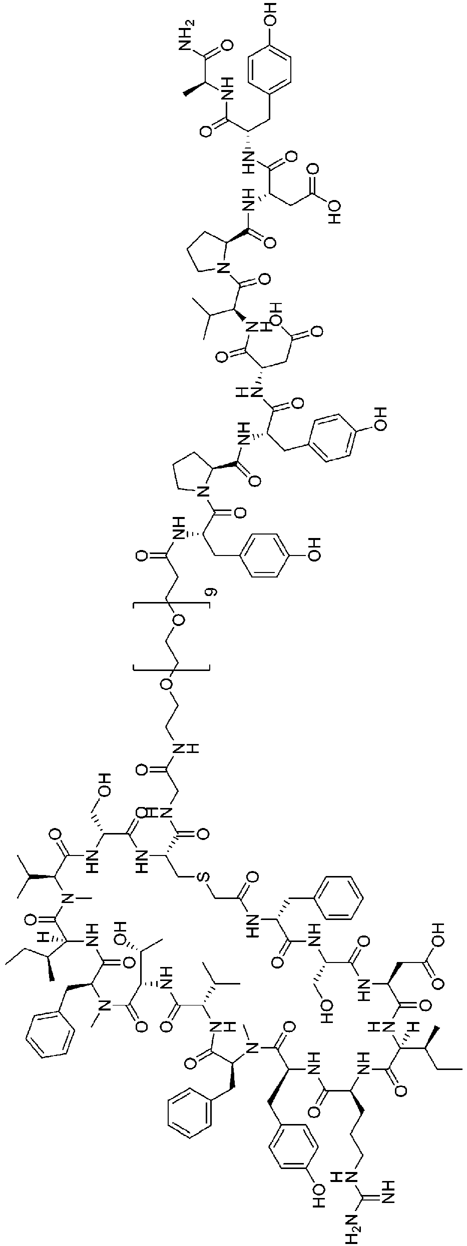

The target peptide was synthesized using Sieber amide resin, starting with removal of the Fmoc group using the general method described above. During the synthesis, a Syro II solid-phase synthesizer from Biotage Inc. was used, with the synthesis conducted in accordance with the manual provided by the manufacturer. For the introduction of each residue, Fmoc-AA/HATU/DIEA (8.4 equivalents / 7.84 equivalents / 16.8 equivalents) was used per one equivalent of resin, and the reaction was conducted twice for 20 minutes at 75°C in DMF. However, the 5th residue and the 14th residue were reacted twice for 30 minutes at 50°C. The 6th residue, 7th residue, 8th residue, 9th residue, 10th residue, 11th residue, and 12th residue were reacted three times for 20 minutes at 75°C. Introduction of the Fmoc-PEG10c of the 16th residue was achieved by a single reaction for 60 minutes at 25°C.

Removal of the Fmoc groups was achieved by reaction in a 10% pyrrolidine DMF solution at 25°C for three minutes.

Chloroacetyl group introduction was conducted by using the above method to remove the Fmoc group from the α-amino group of the Fmoc-protected peptide supported on the solid-phase resin obtained in the previous step, followed by addition of a DMF solution of C1AcOSu (5 equivalents) to the solid-phase resin and shaking at room temperature for 60 minutes.

Side chain deprotection and cleavage from the solid-phase resin was conducted by adding a reagent cocktail A (a mixture of TFA/H₂O/TIS/DODT in a volume ratio of 92.5:2.5:2.5:2.5) to the reaction vessel containing the solid-phase resin, shaking the resulting mixture vigorously and then continuing shaking at room temperature for 60 minutes. The reaction solution was collected by filtration through a frit. Addition of the filtrate to an excess of cooled diisopropyl ether yielded a cloudy white precipitate, and this mixture was centrifuged, and the solution was decanted and removed. The thus obtained solid was washed once more with a cooled diisopropyl ether/hexane (1/1) mixed solvent and then dried for 60 minutes. The resulting solid was used in the subsequent cyclization reaction.

The peptide cyclization reaction was conducted by dissolving the peptide in DMSO/H₂O (7/3) to achieve a final concentration of 1.25 mM based on the number of moles of the solid-phase resin, and then adding triethylamine (10 equivalents) and leaving the mixture to stand at room temperature overnight. The resulting reaction solution was concentrated under reduced pressure using a Genevac EZ-2 Elite, and DMSO was then added to adjust the peptide concentration to 12.5 mM.

The obtained crude product was subjected to solid-phase extraction using a column manufactured by Gilson Co., Ltd. (column: Gilson ASPEC C18, 50 mg, 1 mL).
(1) The column was washed with an extraction solution A (0.1% TFA in 95% MeCN/H₂O, 0.3 mL).
(2) The column was equilibrated with an extraction solution B (0.1% TFA in 5% MeCN/H₂O, 0.3 mL).
(3) Subsequently, 0.02 mL of the above solution was loaded onto the column.
(4) The column was washed with the extraction solution B (0.4 mL).
(5) Extraction was performed with the extraction solution A (0.4 mL). The resulting extract was concentrated under reduced pressure using an EZ-2 Elite.

The purity of one of the main peaks of the target product was calculated from the surface area ratio of the LC/MS (UV wavelength: 220 nm) chromatogram under the following analysis conditions, and revealed a purity of 55%.

### (Analysis Conditions)

Retention time: 1.52 minutes
Column: Kinetex EVO C18 (1.7 µm 2.1 × 50 mm, 100 Å)
Mobile phase A: 0.025% TFA in H₂O
Mobile phase B: 0.025% TFA in MeCN
Temperature: 60°C
Gradient (%B concentration): 5 to 95% in 2.10 minutes, then 95 to 95% for 0.75 minutes
Flow rate: 0.6 mL/min
ESI-MS(+) measured value m/z = 1144 (M+3H) 3+

### [Example 2]

Synthesis of Peptide Complex (Peptide Complex having Linker Structure Number 9 added to Peptide SEQ ID NO: 99)

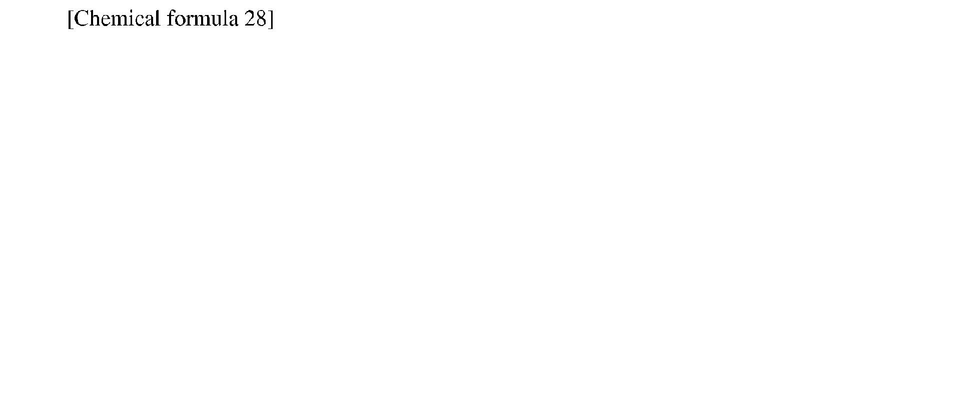

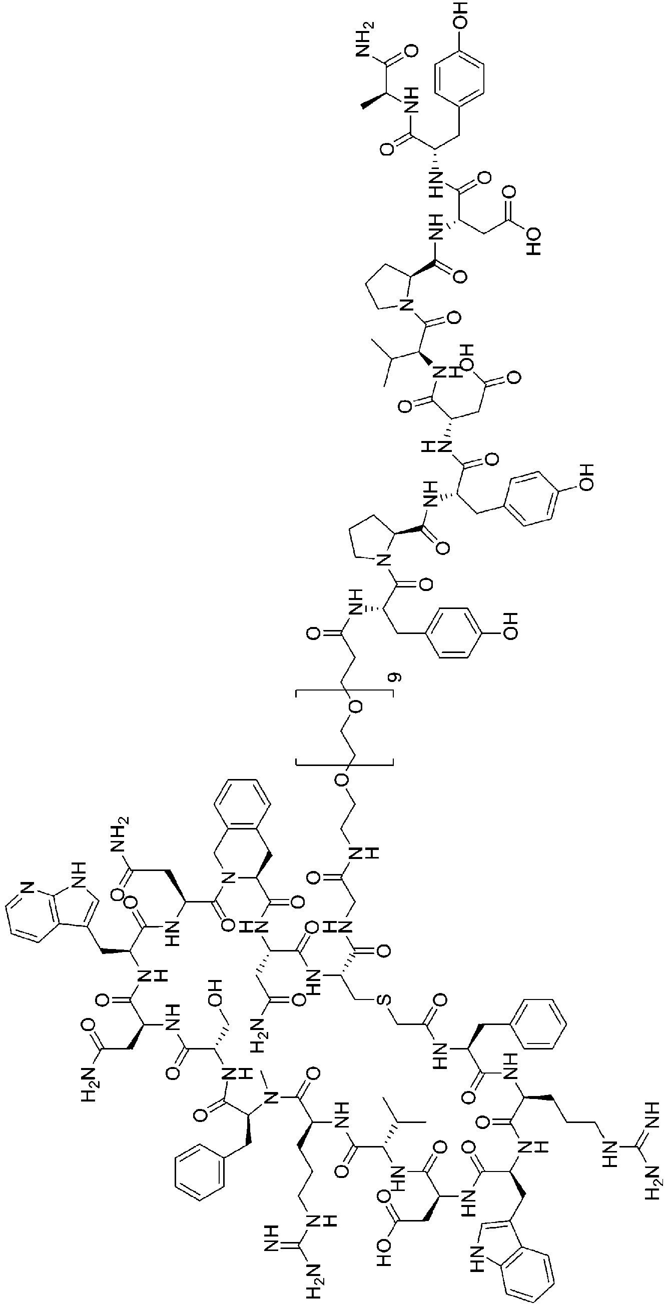

The target peptide was synthesized using Sieber amide resin, starting with removal of the Fmoc group using the general method described above. During the synthesis, a Syro II solid-phase synthesizer from Biotage Inc. was used, with the synthesis conducted in accordance with the manual provided by the manufacturer. For the introduction of each residue, Fmoc-AA/HATU/DIEA (4.2 equivalents / 3.92 equivalents / 8.4 equivalents) was used per one equivalent of resin, and the reaction was conducted twice for 20 minutes at 75°C in DMF. However, the 2nd residue and the 14th residue were reacted twice for 30 minutes at 50°C. The 6th residue was reacted twice for 60 minutes at 50°C. The 7th residue was reacted twice for 30 minutes at 75°C. Introduction of the Fmoc-PEG10c of the 16th residue was achieved by a single reaction for 60 minutes at room temperature.

Removal of the Fmoc groups was achieved by reaction in a 20% piperidine DMF solution at room temperature for five minutes, subsequent removal of the solvent, and then a second reaction in a 20% piperidine DMF solution at room temperature for 15 minutes.

Chloroacetyl group introduction was conducted by using the above method to remove the Fmoc group from the α-amino group of the Fmoc-protected peptide supported on the solid-phase resin obtained in the previous step, followed by addition of a DMF solution of ClAcOSu (5 equivalents) to the solid-phase resin and shaking at room temperature for 60 minutes.

Side chain deprotection and cleavage from the solid-phase resin was conducted by adding a reagent cocktail A (a mixture of TFA/H₂O/TIS/DODT in a volume ratio of 92.5:2.5:2.5:2.5) to the reaction vessel containing the solid-phase resin, shaking the resulting mixture vigorously and then continuing shaking at room temperature for 90 minutes. The reaction solution was collected by filtration through a frit. Addition of the filtrate to an excess of cooled diisopropyl ether yielded a cloudy white precipitate, and this mixture was centrifuged, and the solution was decanted and removed. The thus obtained solid was washed once more with a cooled diisopropyl ether/hexane (1/1) mixed solvent and then dried for 60 minutes. The resulting solid was used in the subsequent cyclization reaction.

The peptide cyclization reaction was conducted by dissolving the peptide in DMSO/H₂O (7/3) to achieve a final concentration of 2.5 mM based on the number of moles of the solid-phase resin, and then adding triethylamine (20 equivalents) and stirring before leaving the mixture to stand at room temperature overnight. The resulting reaction solution was concentrated under reduced pressure using a Genevac EZ-2 Elite to achieve a peptide concentration to 12.5 mM.

The obtained crude product was subjected to solid-phase extraction using a column manufactured by Gilson Co., Ltd. (column: Gilson ASPEC C18, 50 mg, 1 mL).
(1) The column was washed with the extraction solution A (0.1% TFA in 95% MeCN/H₂O, 0.3 mL).
(2) The column was equilibrated with the extraction solution B (0.1% TFA in 5% MeCN/H₂O, 0.3 mL).
(3) Subsequently, 0.02 mL of the above solution was loaded onto the column.
(4) The column was washed with the extraction solution B (0.4 mL).
(5) Extraction was performed with the extraction solution A (0.4 mL). The resulting extract was concentrated under reduced pressure using an EZ-2 Elite.

The purity of one of the main peaks of the target product was calculated from the surface area ratio of the LC/MS (UV wavelength: 220 nm) chromatogram under the following analysis conditions, and revealed a purity of 50%.

### (Analysis Conditions)

Retention time: 1.24 minutes
Column: Kinetex EVO C18 (1.7 µm 2.1 × 50 mm, 100 Å)
Mobile phase A: 0.025% TFA in H₂O
Mobile phase B: 0.025% TFA in MeCN
Temperature: 60°C
Gradient (%B concentration): 5 to 95% in 2.10 minutes, then 95 to 95% for 0.75 minutes
Flow rate: 0.6 mL/min
ESI-MS(+) measured value m/z = 1204 (M+3H) 3+

### [Example 3]

### Synthesis of Peptide Complex (Dimer of Dimer Structure Number 62)

### I. Synthesis of Peptide Complex (Peptide Complex having a portion of Linker Structure Number 2 added to Peptide SEQ ID NO: 74)

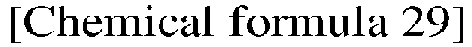

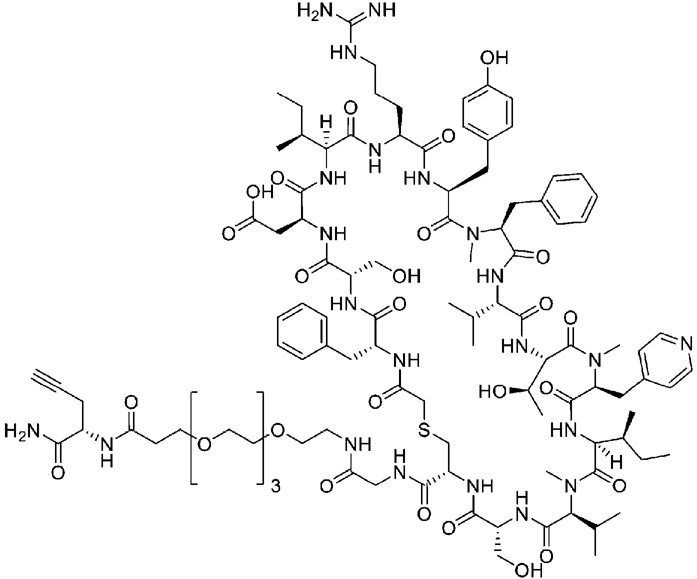

The target peptide was synthesized using Sieber amide resin (Watanabe Chemical Industries, Ltd., 0.6 mmol/g), starting with removal of the Fmoc group using the general method described above. During the synthesis, a Liberty Blue HT solid-phase synthesizer from CEM Corporation was used, with the synthesis conducted in accordance with the manual provided by the manufacturer. For the introduction of each residue, Fmoc-AA/DIPCI/Oxyma Pure (4.2 equivalents / 8 equivalents / 4 equivalents) was used per one equivalent of resin, and the reaction was conducted once for three minutes at 90°C in DMF. However, the 5th residue was reacted twice for 15 minutes at 50°C. The 6th residue and the 9th residue were reacted twice for 10 minutes at 90°C. The 11th residue was reacted twice for 30 minutes at 75°C. Introduction of the Fmoc-PEG4c of the 12th residue,13th residue, 15th residue and 16th residue, and the Fmoc-Gpra-OH of the 17th residue were achieved by a single reaction for 10 minutes at 75°C. The 14th residue was reacted once for 15 minutes at 50°C. The 10th residue was reacted once for 30 minutes at 40°C, using condensation conditions for the Fmoc-AA/HATU/DIEA (4.2 equivalents / 4 equivalents / 8 equivalents).

Fmoc removal was achieved by reaction in a 10% pyrrolidine DMF solution at 90°C for one minute, or by reaction at 50°C for 90 seconds, or by reaction at 75°C for three minutes, or by reaction at room temperature for one minute.

Chloroacetyl group introduction was conducted by adding a DMF solution of C1AcOSu (5 equivalents) to the solid-phase resin and shaking at room temperature for 60 minutes.

Side chain deprotection and cleavage from the solid-phase resin was conducted by first washing the resin obtained following the chloroacetyl group introduction step with DMF, followed by methylene chloride and then diethyl ether, drying the resin under reduced pressure, subsequently adding the reagent cocktail A (a mixture of TFA/H₂O/TIS/DODT in a volume ratio of 92.5:2.5:2.5:2.5) to the reaction vessel containing the solid-phase resin, and then shaking the resulting mixture at room temperature for 60 minutes. The reaction solution was collected by filtration through a frit. Addition of the filtrate to an excess of a mixed solvent of cooled diisopropyl ether/hexane (1/1) yielded a cloudy white precipitate, and this mixture was centrifuged, and the solution was decanted and removed. The thus obtained solid was washed once more with cooled diethyl ether, and then dried under reduced pressure. The resulting solid was used in the subsequent cyclization reaction.

The peptide cyclization reaction was conducted by dissolving the peptide in MeCN/H₂O (1/1) to achieve a final concentration of 3.1 mM based on the number of moles of the solid-phase resin, and then adding triethylamine (10 equivalents), shaking the mixture at room temperature for one hour, and then adding acetic acid. The resulting reaction solution was then concentrated using a Genevac HT-12.

The obtained crude product was purified under the conditions described below.

### (Purification Conditions)

Column: Waters XBridge C18 (5 µm, 50 × 150 mm)
Mobile phases:
   A) 0.1% TFA in H₂O
   B): 0.1% TFA in MeCN
Temperature: 40°C
Gradient (%B conc.): 5% for 2 minutes, 5 to 28% in one minute, 28 to 33% in 8 minutes, and then 33 to 60% in one minute
Flow rate: 20 mL/min for one minute, 20 mL/min increased to 120 mL/min in one minute, then held at 120 mL/min.

Following freeze drying, a peptide complex (190 mg) having a portion of linker structure number 2 added to peptide SEQ ID NO: 74 was obtained.

### II. Synthesis of Peptide Complex (Peptide Complex having a portion of Linker Structure Number 2 added to Peptide SEQ ID NO: 162)

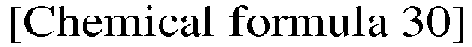

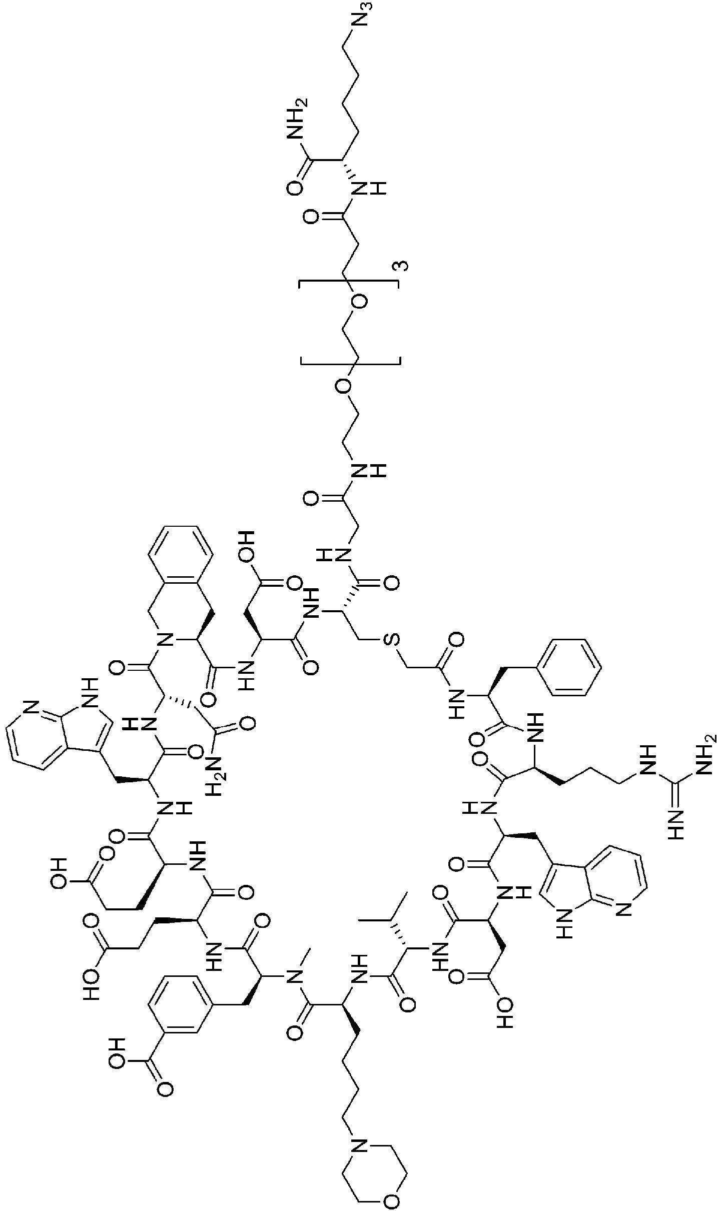

The target peptide was synthesized using Sieber amide resin (Watanabe Chemical Industries, Ltd., 0.54 mmol/g), starting with removal of the Fmoc group using the general method described above. During the synthesis, a Liberty Blue solid-phase synthesizer from CEM Corporation was used, with the synthesis conducted in accordance with the manual provided by the manufacturer. For the introduction of each residue, Fmoc-AA/DIPCI/Oxyma Pure (4.2 equivalents / 8 equivalents / 4 equivalents) was used per one equivalent of resin, and the reaction was conducted once for three minutes at 90°C in DMF. However, the 2nd residue was reacted twice for 15 minutes at 50°C. The 11th residue and the 12th residue were reacted twice for 10 minutes at 90°C. The 14th residue was reacted twice for 15 minutes at 50°C. The 6th residue was reacted twice for 60 minutes at room temperature, using condensation conditions for the Fmoc-AA/HATU/DIEA (4.2 equivalents / 4 equivalents / 8 equivalents). Further, when introducing Fmoc-W7N-OH, Fmoc-Kmor-OH and Fmoc-KN3-OH, solutions prepared by dissolving these amino acids in NMP were used. Fmoc removal was achieved by reaction in a 10% pyrrolidine DMF solution at 90°C for one minute, or by reaction at 50°C for 90 seconds, or by two repetitions of reaction at room temperature for one minute.

Chloroacetyl group introduction was conducted by adding a DMF/DCM (1/1) solution of C1AcOSu (5 equivalents) to the solid-phase resin and shaking at room temperature for 30 minutes.

Side chain deprotection and cleavage from the solid-phase resin was conducted by first washing the resin obtained following the chloroacetyl group introduction step with DMF, followed by methylene chloride and then diethyl ether, drying the resin under reduced pressure, subsequently adding the reagent cocktail A (a mixture of TFA/H₂O/TIS/DODT in a volume ratio of 92.5:2.5:2.5:2.5) to the reaction vessel containing the solid-phase resin, and then shaking the resulting mixture at room temperature for 60 minutes. The reaction solution was collected by filtration through a frit. Addition of the filtrate to an excess of a mixed solvent of cooled diisopropyl ether/hexane (1/1) yielded a cloudy white precipitate, and this mixture was centrifuged, and the solution was decanted and removed. The thus obtained solid was washed once more with cooled diethyl ether, and then dried under reduced pressure. The resulting solid was used in the subsequent cyclization reaction.

The peptide cyclization reaction was conducted by dissolving the peptide in MeCN/H₂O (1/1) to achieve a final concentration 5 mM based on the number of moles of the solid-phase resin, and then adding triethylamine (10 equivalents), shaking the mixture at room temperature for one hour, and then adding acetic acid. The resulting reaction solution was then concentrated using a Genevac HT-12.

The obtained crude product was purified under the conditions described below.

### (Purification Conditions)

Column: Waters XBridge C18 (5 µm, 50 × 150 mm)
Mobile phases:
   A) 0.1% TFA in H₂O
   B): 0.1% TFA in MeCN
Temperature: 40°C
Gradient (%B conc.): 5% for 2 minutes, 5 to 21% in one minute, 21 to 26% in 8 minutes, and then 26 to 60% in one minute
Flow rate: 20 mL/min for one minute, 20 mL/min increased to 120 mL/min in one minute, then held at 120 mL/min.

Following freeze drying, a peptide complex (161 mg) having a portion of linker structure number 2 added to peptide SEQ ID NO: 162 was obtained.

### III. Synthesis of Peptide Complex (Dimer of Dimer Structure Number 62)

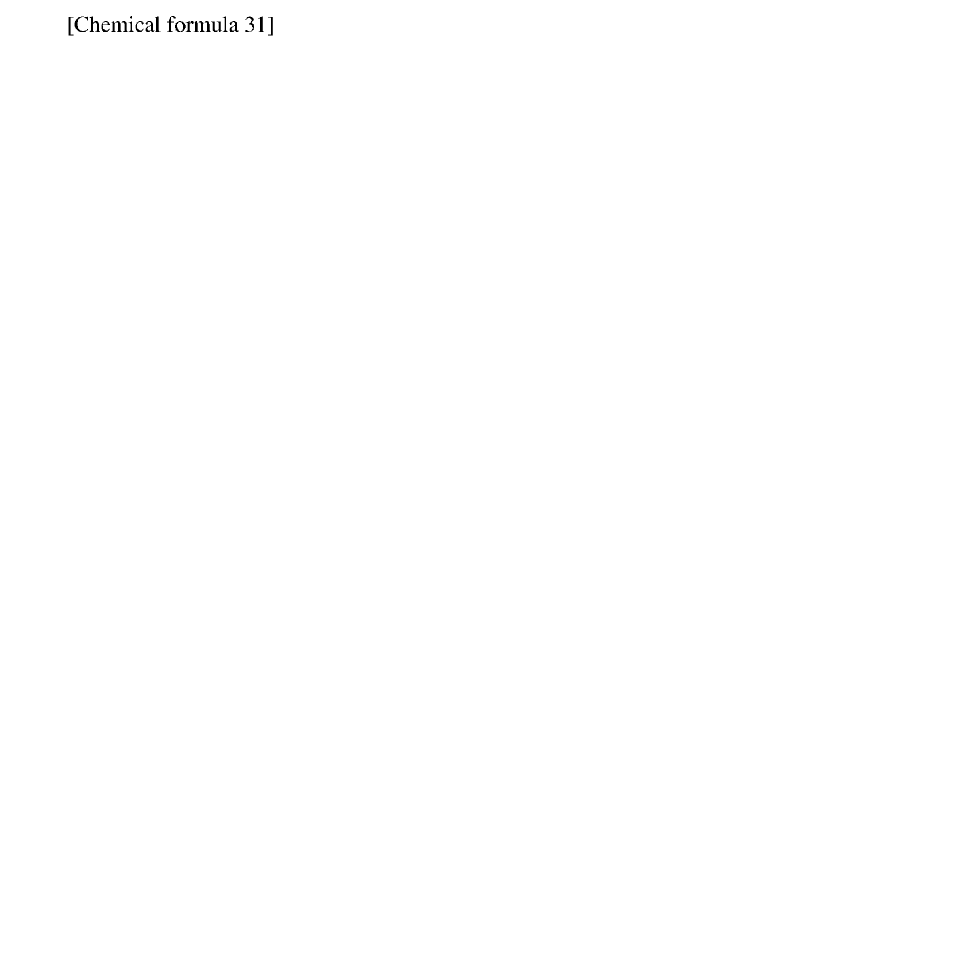

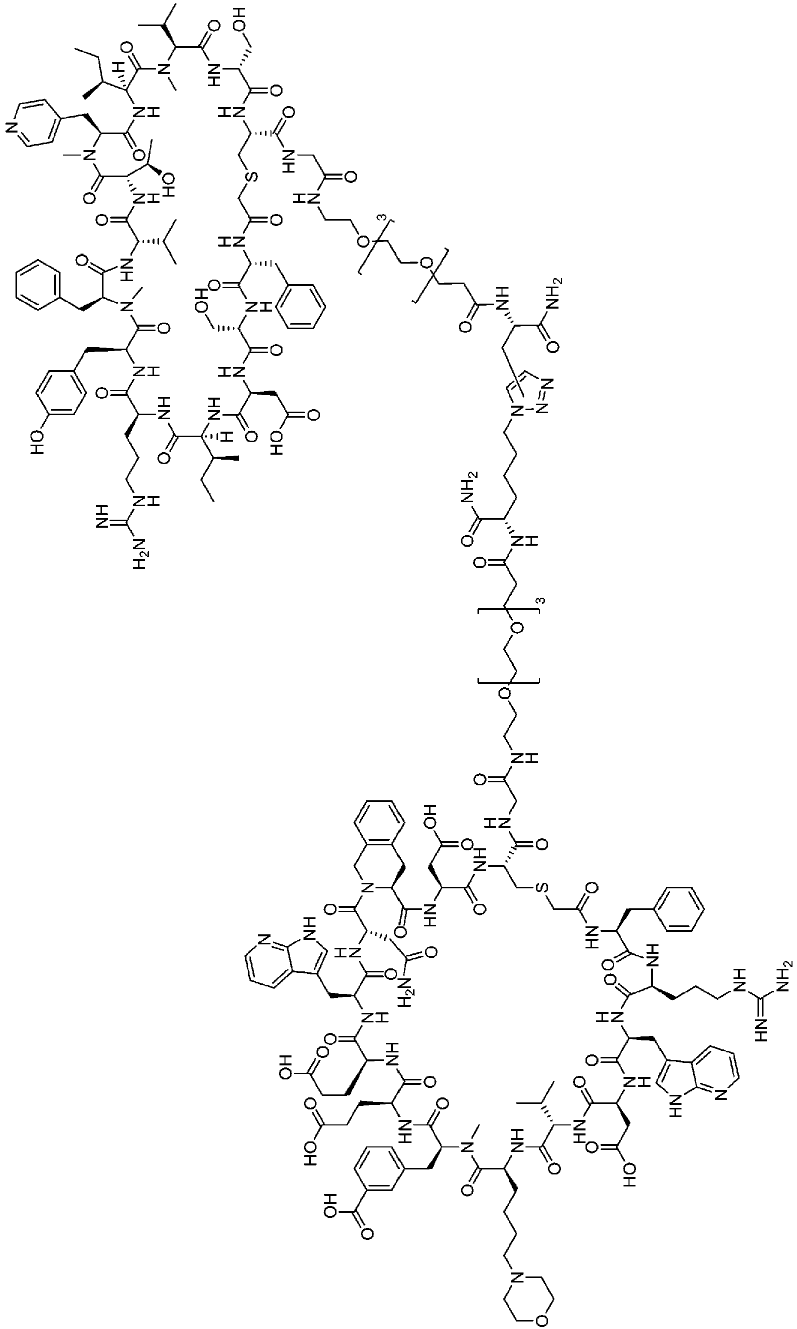

Synthesis of the peptide complex was conducted by dissolving the peptide complex (the peptide complex having a portion of linker structure number 2 added to peptide SEQ ID NO: 162) (30 mg) in DMF/H₂O (3/1), adding the peptide complex (the peptide complex having a portion of linker structure number 2 added to peptide SEQ ID NO: 74) (27 mg, 1.1 equivalents) and [Cu(CH₃CN)₄]PF₆ (4 equivalents), and then stirring the resulting mixture at room temperature for 30 minutes.

The obtained crude product was purified under the conditions described below.

### (Purification Conditions)

Column: Waters XSelect CSH Prep C18 (5 µm, OBD, 50 × 250 mm)
Mobile phases:
   A) 1% AcOH in H₂O
   B): 1% AcOH in MeCN
   C): 0.2 M TEAA in H₂O
   D): MeCN
Temperature: 50°C
Main Pump Gradients
(%A conc.): 0.1% for 5.0 minutes, then 0.1 to 100% in 0.1 minutes, with 100%-%B from 5.1 minutes onward
(%B conc.): 0% for 5.1 minutes, then 0 to 4.2% in 1.9 minutes, 4.2 to 50% in 20 minutes, and then 50 to 90% in four minutes
(%C conc.): 99.9% for 5.0 minutes, then 99.9 to 0% in 0.1 minutes, with 0% from 5.1 minutes onward
(%D conc.): 0%
Flow rate: 18 mL/min for 5.1 minutes, 18 mL/min increased to 118 mL/min in 1.9 minutes, then held at 118 mL/min
At-column-dilution pump 1% AcOH in H₂O/MeCN (1/1)
Flow rate: 2 mL/min

The product purity was calculated from the surface area ratio of the LC/MS (UV wavelength: 225 nm) chromatogram under the following analysis conditions, and revealed a purity of 95.06%.

### (Analysis Conditions)

Retention time: 5.80 minutes
Column: Kinetex EVO C18 (2.6 µm, 2.1 × 150 mm, 100 Å)
Mobile phases:
   A) 0.025% TFA in H₂O
   B): 0.025% TFA in MeCN
Temperature: 60°C
Gradient (%B concentration): 5 to 45% in 7.15 minutes, then 45 to 95% in 0.30 minutes, and subsequently 95 to 95% for 1.55 minutes
Flow rate: 0.5 mL/min
ESI-MS(+) measured value m/z = 1186 (M+4H) 4+

### [Example 4]

### Synthesis of Peptide Complex (Dimer of Dimer Structure Number 46)

### I. Synthesis of Peptide Complex (Peptide Complex having a portion of Linker Structure Number 8 added to Peptide SEQ ID NO: 35)

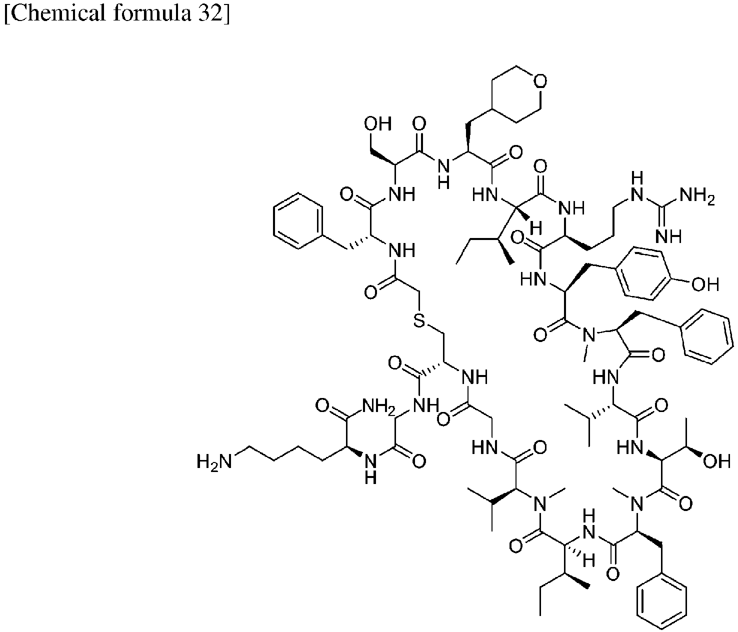

The target peptide was synthesized using Sieber amide resin (Watanabe Chemical Industries, Ltd., 0.54 mmol/g), starting with removal of the Fmoc group using the general method described above. During the synthesis, a Liberty PRIME solid-phase synthesizer from CEM Corporation was used, with the synthesis conducted in accordance with the manual provided by the manufacturer. For the introduction of each residue, Fmoc-AA/DIPCI/Oxyma Pure (4.2 equivalents / 8 equivalents / 4 equivalents) was used per one equivalent of resin, and the reaction was conducted once for two minutes at 105°C in DMF. However, the 5th residue was reacted twice for 15 minutes at 50°C. The 6th residue, the 9th residue and the 11th residue were reacted twice for 30 minutes at 75°C. The 14th residue was reacted once for 15 minutes at 50°C.

Fmoc removal was conducted under the basic condition of reaction with an 83 mM Oxyma pure 4% pyrrolidine solution in DMF at 110°C for 90 seconds, but also under the condition of conducting two consecutive reactions with a 10% pyrrolidine solution in DMF at room temperature for one minute.

Chloroacetyl group introduction was conducted by adding a DMF solution of C1AcOSu (5 equivalents) to the solid-phase resin and shaking at room temperature for 60 minutes.

Side chain deprotection and cleavage from the solid-phase resin was conducted by first washing the resin obtained following the chloroacetyl group introduction step with DMF, followed by methylene chloride and then diethyl ether, drying the resin under reduced pressure, subsequently adding the reagent cocktail A (a mixture of TFA/H₂O/TIS/DODT in a volume ratio of 92.5:2.5:2.5:2.5) to the reaction vessel containing the solid-phase resin, and then shaking the resulting mixture at room temperature for 60 minutes. The reaction solution was collected by filtration through a frit. Addition of the filtrate to an excess of a mixed solvent of cooled diisopropyl ether/hexane (1/1) yielded a cloudy white precipitate, and this mixture was centrifuged, and the solution was decanted and removed. The thus obtained solid was washed once more with cooled diethyl ether, and then dried under reduced pressure. The resulting solid was used in the subsequent cyclization reaction.

The peptide cyclization reaction was conducted by dissolving the peptide in MeCN/H₂O (1/1) to achieve a final concentration of 2.5 mM based on the number of moles of the solid-phase resin, and then adding triethylamine (10 equivalents), shaking the mixture at room temperature for four hours, and then adding acetic acid. The resulting reaction solution was then concentrated using a Genevac EZ-2 Elite. The obtained crude product was purified under the conditions described below, yielding a peptide complex (a peptide complex having a portion of linker structure number 8 added to peptide SEQ ID NO: 35).

### (Purification Conditions)

Column: Waters XBridge C18 (5 µm, 50 × 150 mm)
Mobile phases:
   A) 0.1% TFA in H₂O
   B): 0.1% TFA in MeCN
Temperature: 40°C
Gradient (%B conc.): 9% for 2 minutes, 9 to 34% in one minute, 34 to 39% in 8 minutes, and then 39 to 60% in one minute
Flow rate: 20 mL/min for one minute, 20 mL/min increased to 120 mL/min in one minute, then held at 120 mL/min.

The purity of the target product was calculated from the surface area ratio of the LC/MS (UV wavelength: 225 nm) chromatogram under the following analysis conditions, and revealed a purity of 97.80%.

### (Analysis Conditions)

Retention time: 4.25 minutes
Column: Kinetex EVO C18 (2.6 µm, 2.1 × 150 mm, 100 Å)
Mobile phases:
   A) 0.025% TFA in H₂O
   B): 0.025% TFA in MeCN
Temperature: 60°C
Gradient (%B concentration): 20 to 60% in 7.15 minutes, 60 to 95% in 0.30 minutes, then 95 to 95% for 1.55 minutes
Flow rate: 0.5 mL/min
ESI-MS(+) measured value m/z = 988 (M+2H) 2+

### II. Synthesis of Peptide Complex (Peptide Complex having a portion of Linker Structure Number 8 added to Peptide SEQ ID NO: 79)

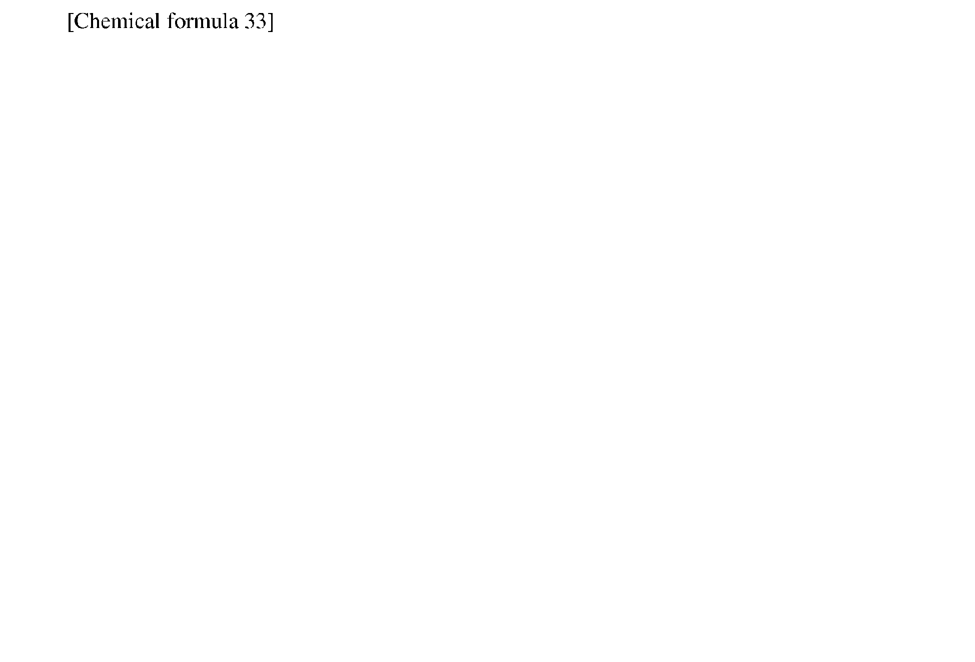

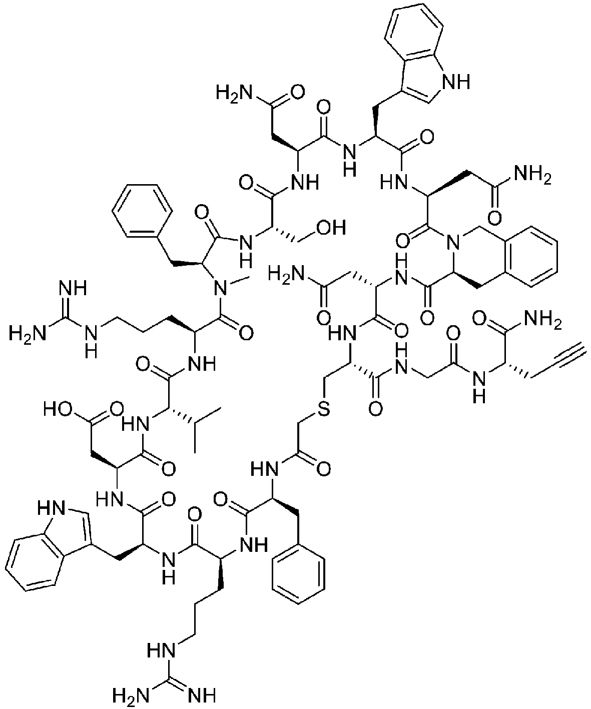

The target peptide was synthesized using Sieber amide resin (Watanabe Chemical Industries, Ltd., 0.54 mmol/g), starting with removal of the Fmoc group using the general method described above. During the synthesis, a Liberty PRIME solid-phase synthesizer from CEM Corporation was used, with the synthesis conducted in accordance with the manual provided by the manufacturer. For the introduction of each residue, Fmoc-AA/DIPCI/Oxyma Pure (4.2 equivalents / 8 equivalents / 4 equivalents) was used per one equivalent of resin, and the reaction was conducted once for two minutes at 105°C in DMF. However, the 2nd residue and the 6th residue were reacted twice for 15 minutes at 50°C. The 11th residue and the 12th residue were reacted twice for three minutes at 105°C. The 14th residue was reacted once for 15 minutes at 50°C.

Fmoc removal was conducted under the basic condition of reaction with an 83 mM Oxyma pure 4% pyrrolidine solution in DMF at **110°C** for 90 seconds, but also under either the condition of conducting two consecutive reactions with a 10% pyrrolidine solution in DMF at room temperature for one minute, or the condition of reaction with a 10% pyrrolidine solution in DMF at 50°C for 90 seconds.

Chloroacetyl group introduction was conducted by adding a DMF solution of C1AcOSu (5 equivalents) to the solid-phase resin and shaking at room temperature for 60 minutes.

Side chain deprotection and cleavage from the solid-phase resin was conducted by first washing the resin obtained following the chloroacetyl group introduction step with DMF, followed by methylene chloride and then diethyl ether, drying the resin under reduced pressure, subsequently adding the reagent cocktail A (a mixture of TFA/H₂O/TIS/DODT in a volume ratio of 92.5:2.5:2.5:2.5) to the reaction vessel containing the solid-phase resin, and then shaking the resulting mixture at room temperature for 60 minutes. The reaction solution was collected by filtration through a frit. Addition of the filtrate to an excess of a mixed solvent of cooled diisopropyl ether/hexane (1/1) yielded a cloudy white precipitate, and this mixture was centrifuged, and the solution was decanted and removed. The thus obtained solid was washed once more with cooled diethyl ether, and then dried under reduced pressure. The resulting solid was used in the subsequent cyclization reaction.

The peptide cyclization reaction was conducted by dissolving the peptide in MeCN/H₂O (1/1) to achieve a final concentration of 2.5 mM based on the number of moles of the solid-phase resin, and then adding triethylamine (10 equivalents), shaking the mixture at room temperature for four hours, and then adding acetic acid. The resulting reaction solution was then concentrated using a Genevac EZ-2 Elite. The obtained crude product was purified under the conditions described below, yielding a peptide complex (a peptide complex having a portion of linker structure number 8 added to peptide SEQ ID NO: 79).

### (Purification Conditions)

Column: Waters XBridge C18 (5 µm, 50 × 150 mm)
Mobile phases:
   A) 0.1% TFA in H₂O
   B): 0.1% TFA in MeCN
Temperature: 40°C
Gradient (%B conc.): 5% for 2 minutes, 5 to 27% in one minute, 27 to 32% in 8 minutes, and then 32 to 60% in one minute
Flow rate: 20 mL/min for one minute, 20 mL/min increased to 120 mL/min in one minute, then held at 120 mL/min.

The purity of the target product was calculated from the surface area ratio of the LC/MS (UV wavelength: 225 nm) chromatogram under the following analysis conditions, and revealed a purity of 95.95%.

### (Analysis Conditions)

Retention time: 5.85 minutes
Column: Kinetex EVO C18 (2.6 µm, 2.1 × 150 mm, 100 Å)
Mobile phases:
   A) 0.025% TFA in H₂O
   B): 0.025% TFA in MeCN
Temperature: 60°C
Gradient (%B concentration): 5 to 45% in 7.15 minutes, 45 to 95% in 0.30 minutes, then 95 to 95% for 1.55 minutes
Flow rate: 0.5 mL/min
ESI-MS(+) measured value m/z = 1055 (M+2H) 2+

### III. Synthesis of Peptide Complex (Dimer of Dimer Structure Number 46)

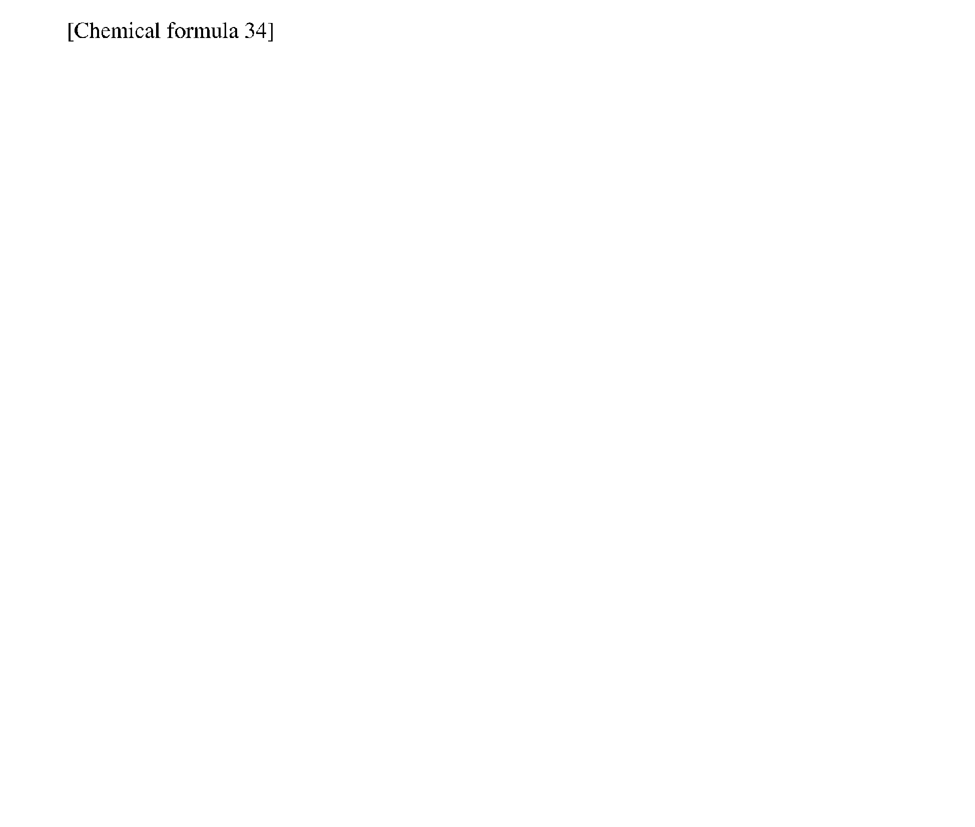

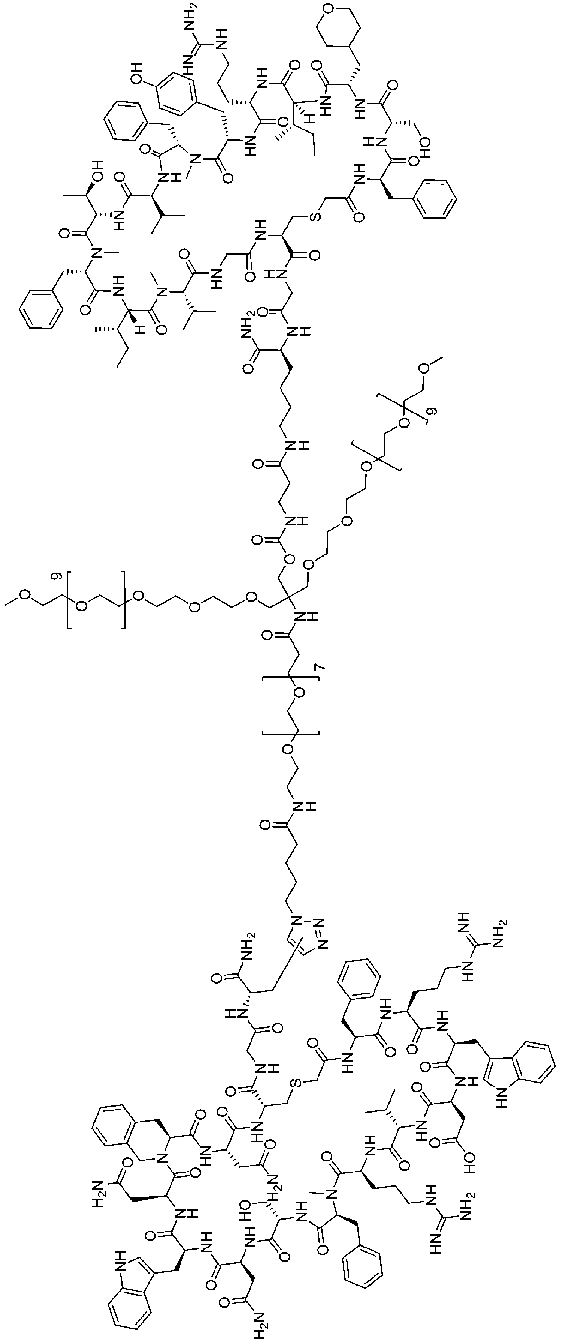

Synthesis of the peptide complex was conducted by adding a DMA solution of 0.0175 M AZ(L08)-P12-PS (0.36 mL) and DIPEA (0.012 mL) to the peptide complex (the peptide complex having a portion of linker structure number 8 added to peptide SEQ ID NO: 35) (20 mg) at 40°C, stirring the resulting mixture at the same temperature for two hours, adding an additional amount of the DMA solution of 0.0175 M AZ(L08)-P12-PS (0.13 mL), and stirring at the same temperature for a further one hour. Water (0.36 ml), a DMA solution (0.36 mL) of the peptide complex (the peptide complex having a portion of linker structure number 8 added to peptide SEQ ID NO: 79) (20.05 mg), TBTA (7.2 mg) and [Cu(CH₃CN)₄]PF₆ (3.4 mg) were added to the reaction solution, and following stirring at room temperature for one hour, acetic acid was added. The thus obtained crude product was purified under the conditions described below, yielding the target product (17 mg).

### (Purification Conditions)

Column: Waters XBridge C18 (5 µm, 19 × 150 mm)
Mobile phases:
   A) 0.1% TFA in H₂O
   B): 0.1% TFA in MeCN
Temperature: 40°C
Gradient (%B conc.): 14 to 39% in 3 minutes, 39 to 44% in 8 minutes, and then 44 to 60% in one minute
Flow rate: 17 mL/min.

The purity of the target product was calculated from the surface area ratio of the LC/MS (UV wavelength: 225 nm) chromatogram under the following analysis conditions, and revealed a purity of 94.64%.

### (Analysis Conditions)

Retention time: 5.40 minutes
Column: Kinetex EVO C18 (2.6 µm, 2.1 × 150 mm, 100 Å)
Mobile phases:
   A) 0.025% TFA in H₂O
   B): 0.025% TFA in MeCN
Temperature: 60°C
Gradient (%B concentration): 20 to 60% in 7.15 minutes, 60 to 95% in 0.30 minutes, then 95 to 95% for 1.55 minutes
Flow rate: 0.5 mL/min
ESI-MS(+) measured value m/z = 1484 (M+4H) 4+

### [Example 5]

### Synthesis of Peptide Complex (Dimer of Dimer Structure Number 63)

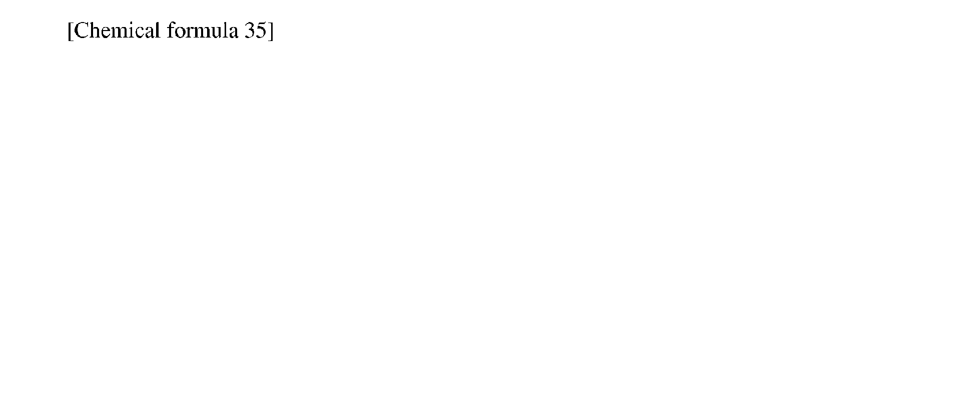

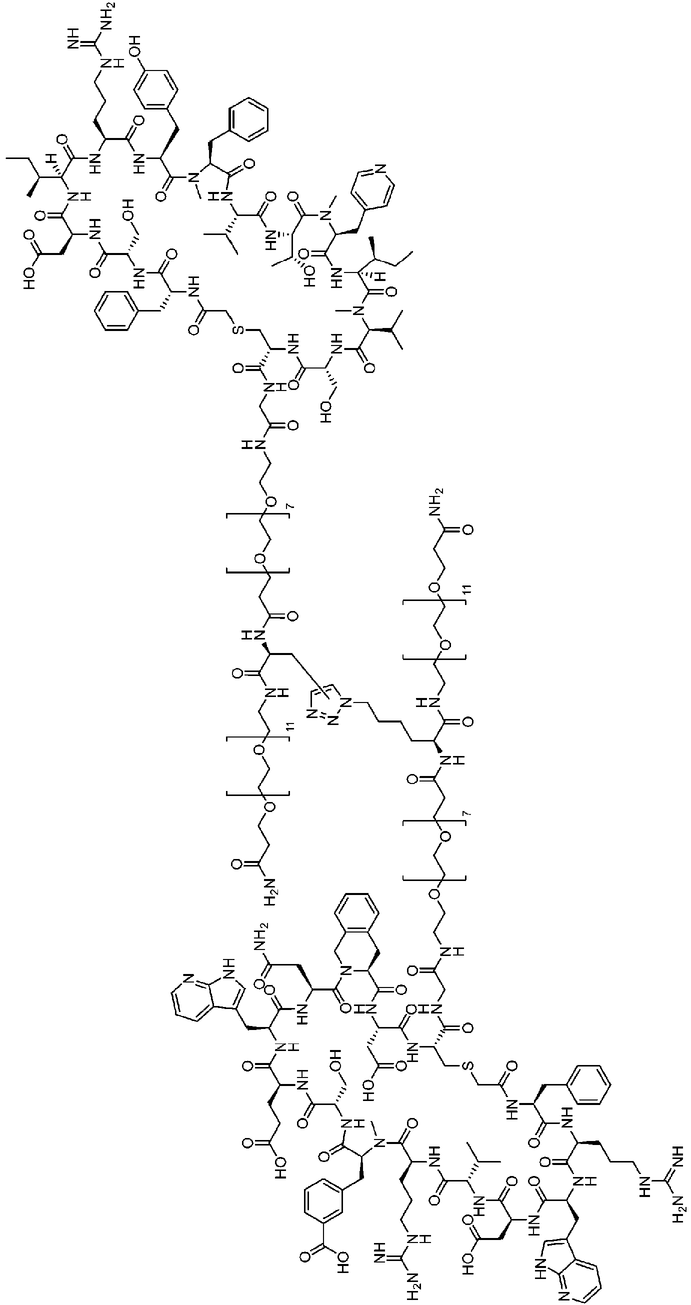

The target product was obtained by conducting synthesis using the same method as Example 3.

The purity of the target product was calculated from the surface area ratio of the LC/MS (UV wavelength: 225 nm) chromatogram under the following analysis conditions, and revealed a purity of 97.49%.

### (Analysis Conditions)

Retention time: 4.10 minutes
Column: Kinetex EVO C18 (2.6 µm, 2.1 × 150 mm, 100 Å)
Mobile phases:
   A) 0.025% TFA in H₂O
   B): 0.025% TFA in MeCN
Temperature: 60°C
Gradient (%B concentration): 20 to 60% in 7.15 minutes, 60 to 95% in 0.30 minutes, then 95 to 95% for 1.55 minutes
Flow rate: 0.5 mL/min
ESI-MS(+) measured value m/z = 1242 (M+5H) 5+

### [Example 6]

### Synthesis of Peptide Complex (Dimer of Dimer Structure Number 66)

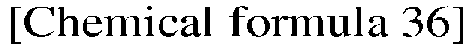

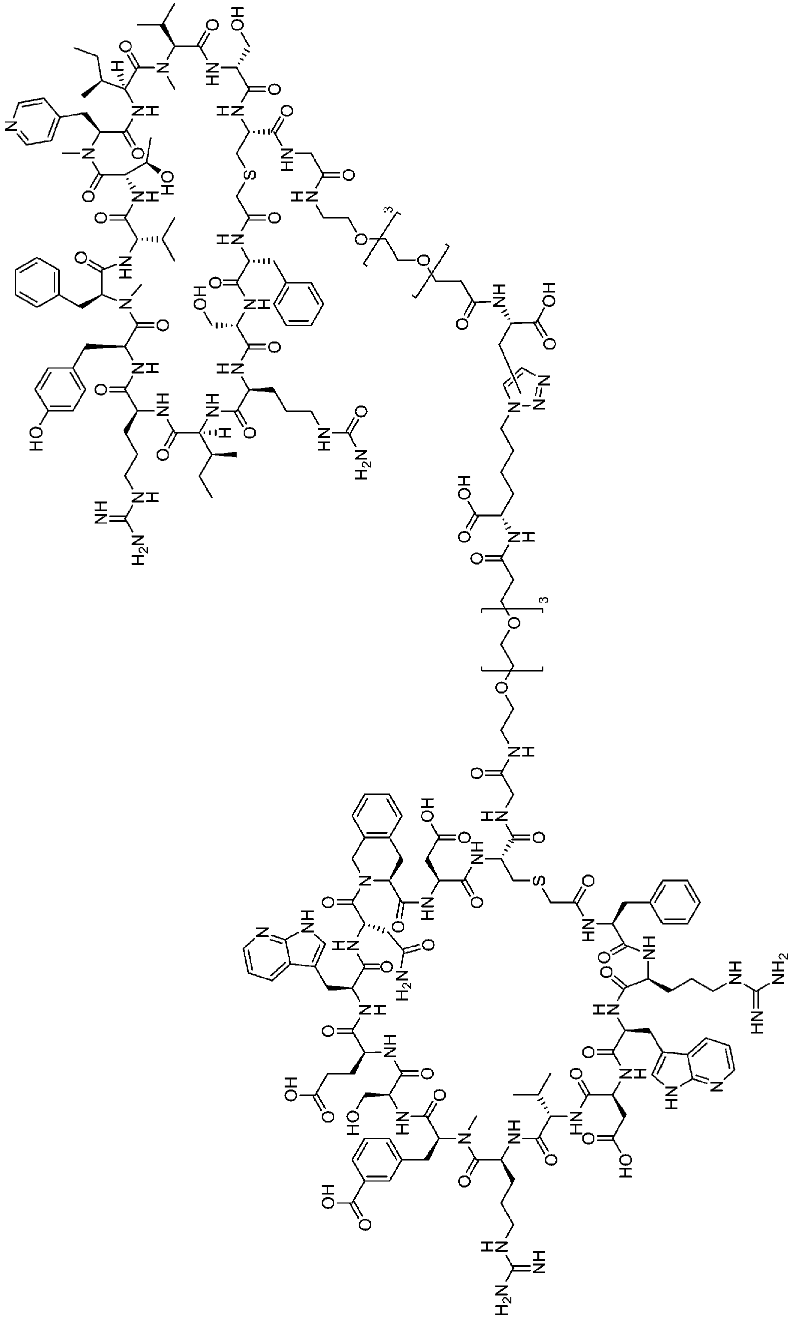

Using a peptide complex (a peptide complex having a portion of linker structure number 3 added to peptide SEQ ID NO: 163) synthesized in the same manner as Example 3 using an Fmoc-Gpra-HMPB-MBHA resin that was synthesized by conducting a single reaction for one hour at room temperature in DMF/DCM (1/2) of Fmoc-Gpra-OH/DIC/DMAP (4 equivalents / 4 equivalents / 0.5 equivalents) per one equivalent of an HMPB-MBHA resin (from Novabiochem Ltd., 0.69 mmol/g), and a peptide complex (a peptide complex having a portion of linker structure number 3 added to peptide SEQ ID NO: 76) synthesized in the same manner as Example 3 using an Fmoc-KN3-HMPB-MBHA resin that was synthesized by conducting a single reaction for one hour at room temperature in DMF/DCM (1/2) of Fmoc-KN3-OH/DIC/DMAP (4 equivalents / 4 equivalents / 0.5 equivalents) per one equivalent of the HMPB-MBHA resin (from Novabiochem Ltd., 0.69 mmol/g), a target product was obtained using the same method as Example 3.

The purity of the target product was calculated from the surface area ratio of the LC/MS (UV wavelength: 225 nm) chromatogram under the following analysis conditions, and revealed a purity of 95.04%.

### (Analysis Conditions)

Retention time: 2.80 minutes
Column: Kinetex EVO C18 (2.6 µm, 2.1 × 150 mm, 100 Å)
Mobile phases:
   A) 0.025% TFA in H₂O
   B): 0.025% TFA in MeCN
Temperature: 60°C
Gradient (%B concentration): 20 to 60% in 7.15 minutes, 60 to 95% in 0.30 minutes, then 95 to 95% for 1.55 minutes
Flow rate: 0.5 mL/min
ESI-MS(+) measured value m/z = 1176 (M+4H) 4+

### [Example 7]

### Synthesis of Various Peptides

In this example, various peptides were chemically synthesized in the same manner as described above in Example 1 to Example 6. The amino acid sequences of the synthesized cyclic peptides are shown in Table 5 and Table 6, the linker structures are shown in the formulas below, and the peptide complexes produced by dimerizing the cyclic peptides via a linker are shown in Table 7.

The synthesized peptide complexes were each analyzed using the analysis conditions described above in any one of Examples 1 to 6, and the structure was confirmed by ESI-MS(+) mass spectrometry. For each peptide complex, the obtained ESI-MS(+) measured value, the retention time, the valence, and the concentration gradient (%) for the mobile phase B used during analysis are shown in Table 5, Table 6, and Table 7.

The analysis results shown in Tables 5 and 6 are values obtained as a result of analyzing the peptide complex in which a linker represented by linker structural number 9 has been added to the peptide indicated by the shown peptide sequence number.

### [Table 5A]

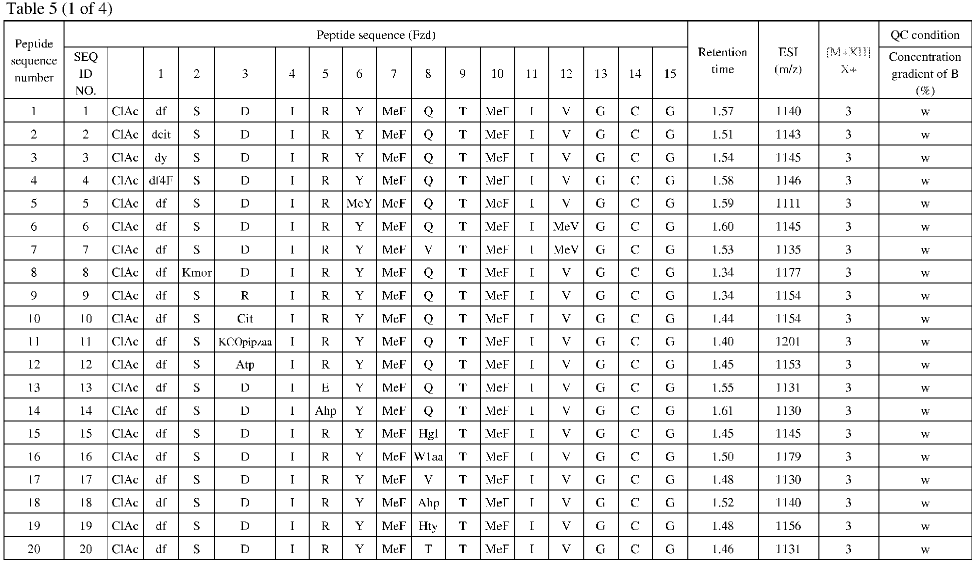

### [Table 5B]

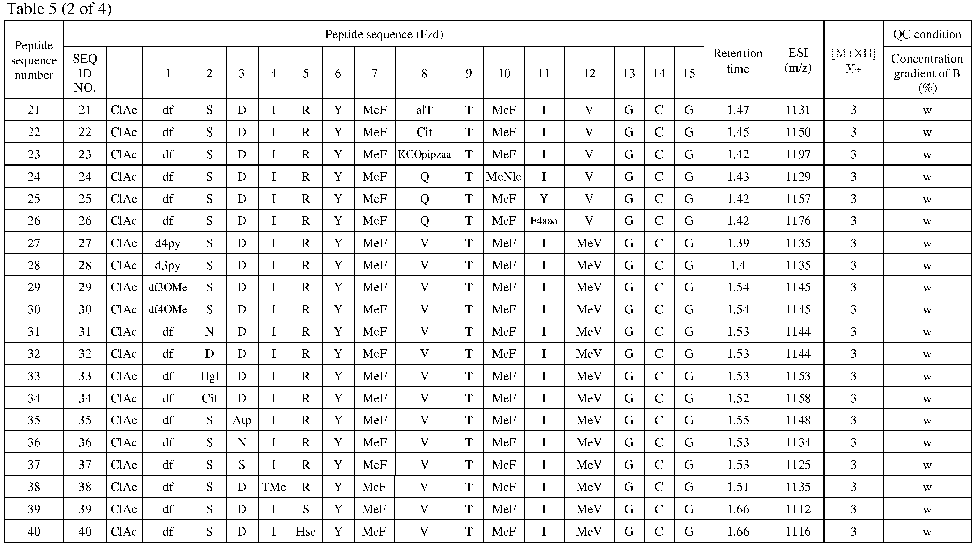

### [Table 5C]

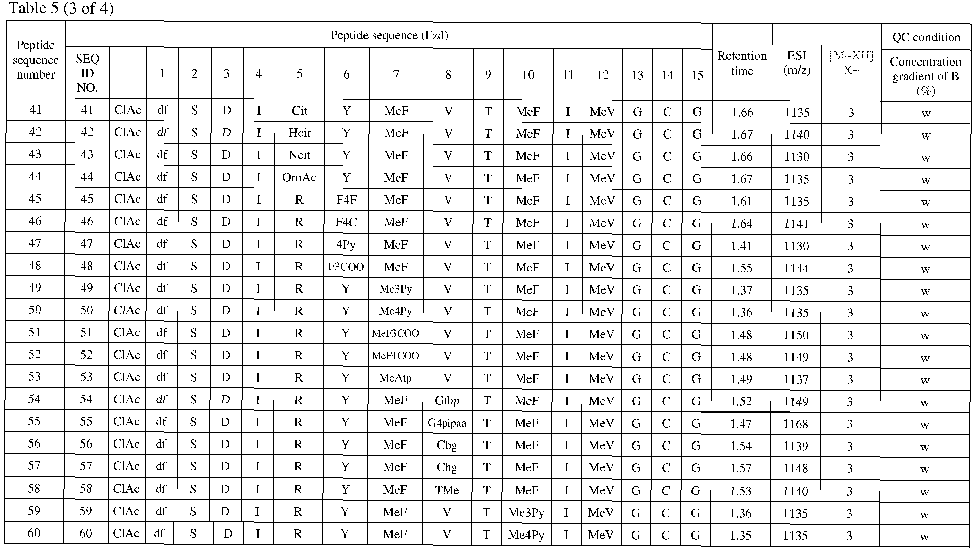

### [Table 5D]

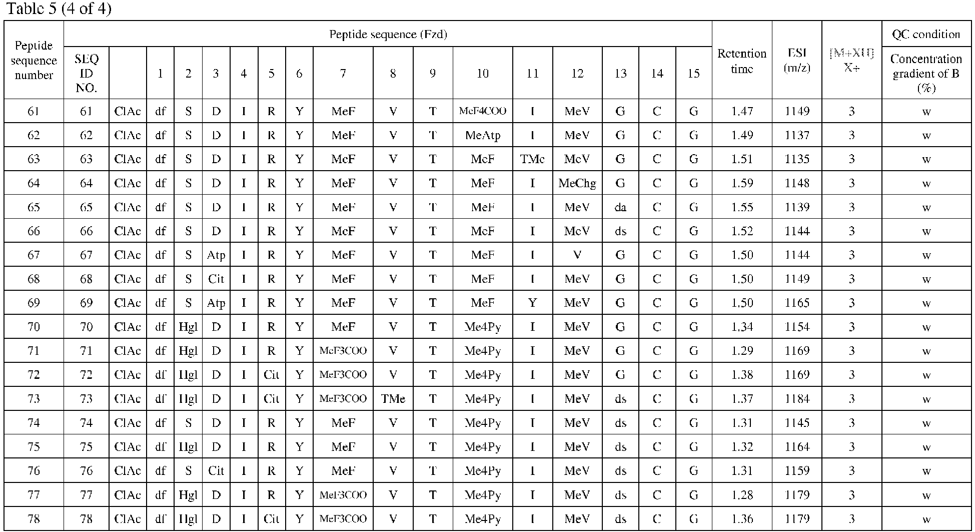

### [Table 6A]

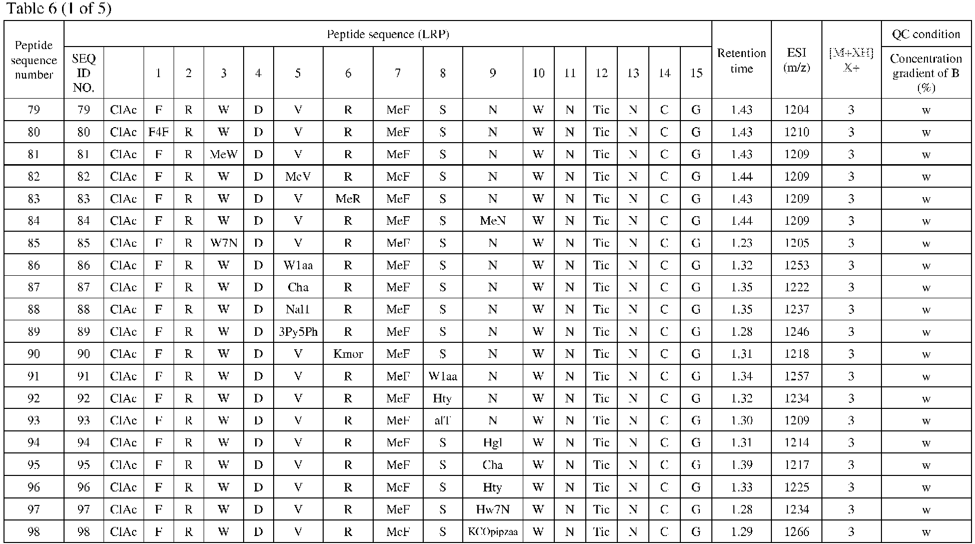

### [Table 6B]

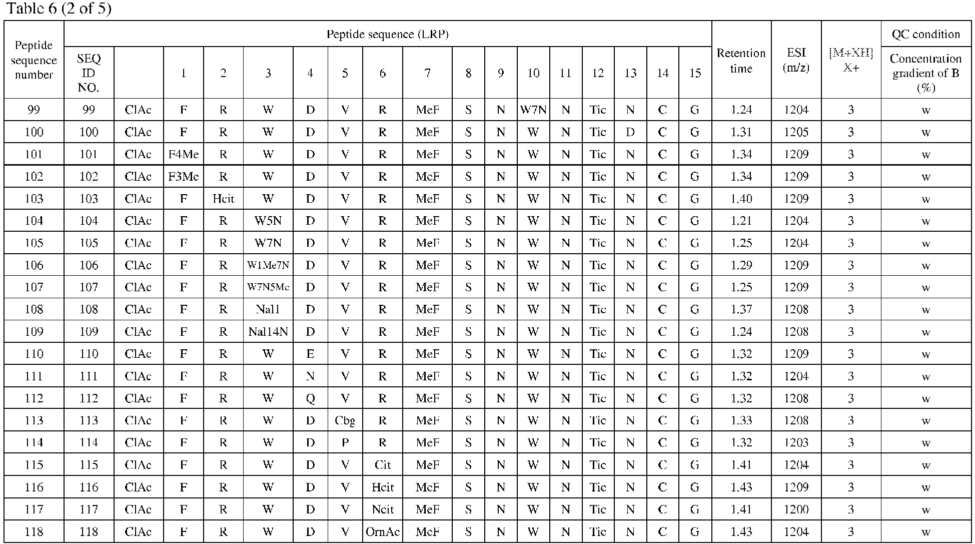

### [Table 6C]

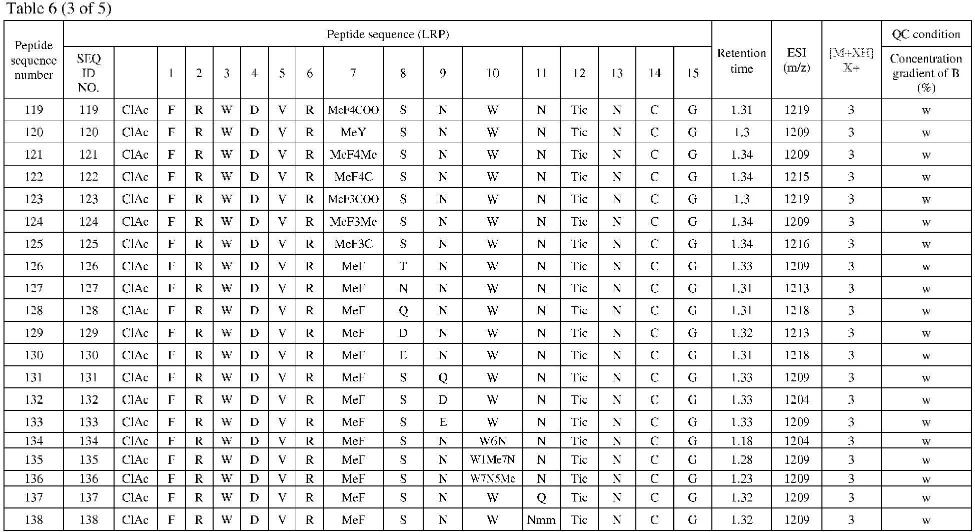

### [Table 6D]

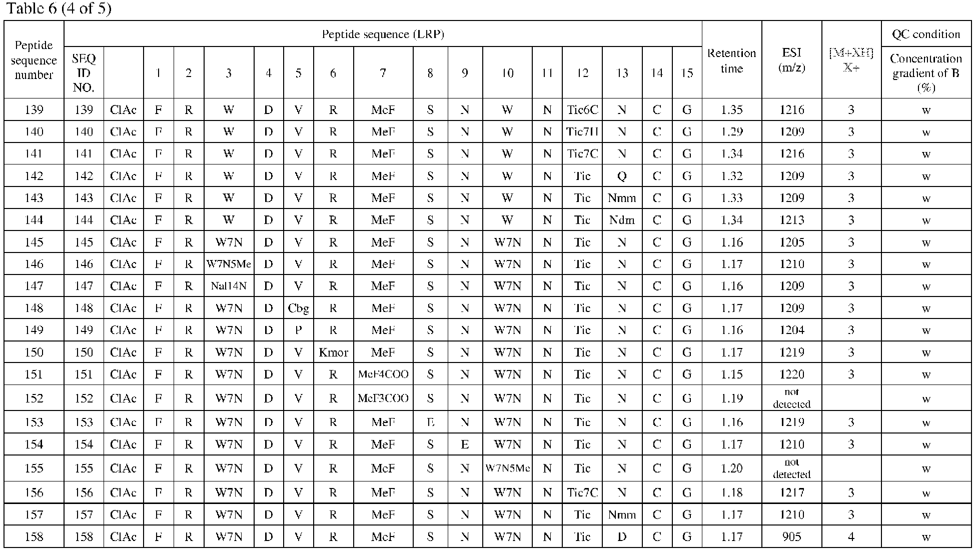

### [Table 6E]

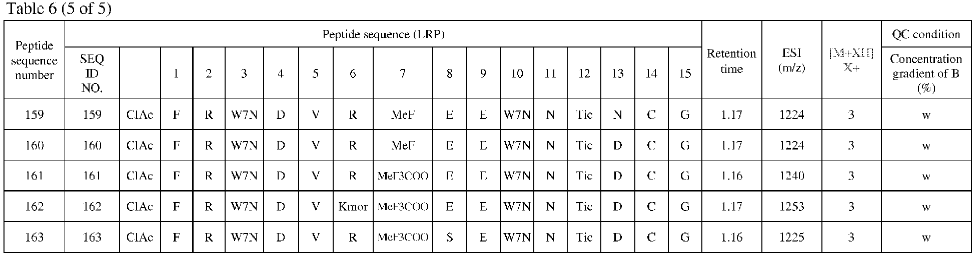

### [Chemical formula 37A]

| Linker Structure Number | Linker Name | Structure |
|---|---|---|
| 1 | P08K_d | 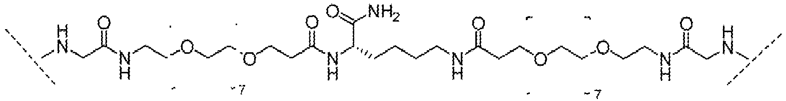 |
| 2 | click_P04K/c_P04K | 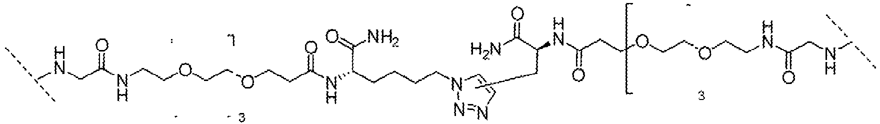 |
| 3 | click_P04K_0H | 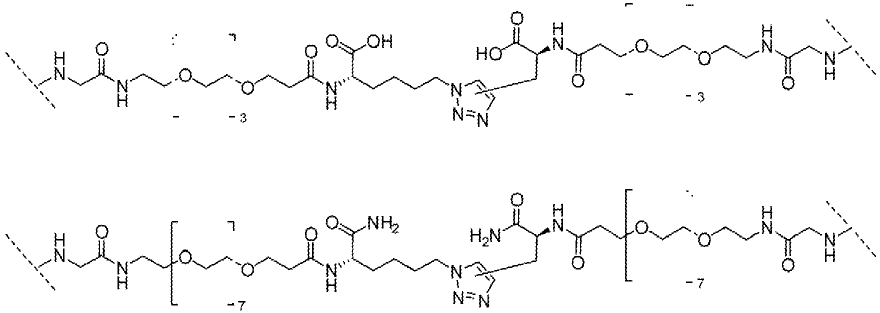 |
| 4 | click_P08K | 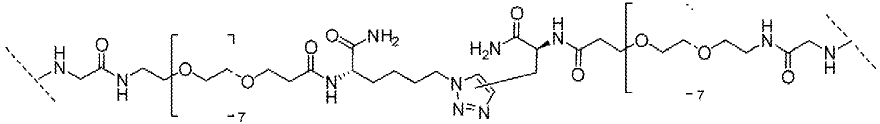 |

### [Chemical formula 37B]

| Linker Structure Number | Linker Name | Structure |
|---|---|---|
| 5 | click_P12K | 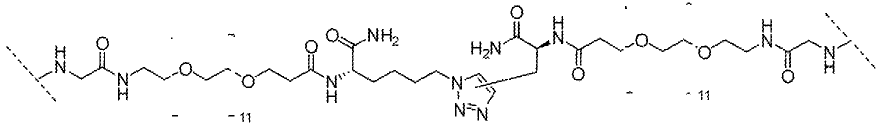 |
| 6 | click_P04K_branch | 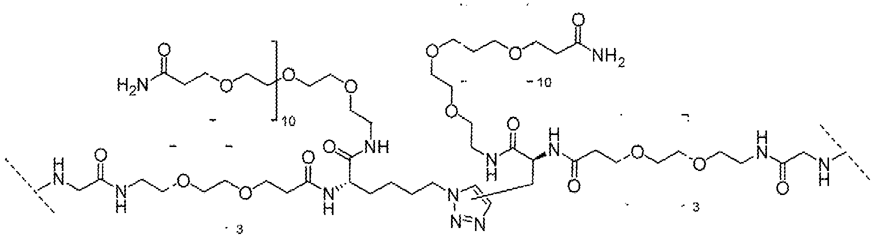 |
| 7 | click_P0BK_branch | 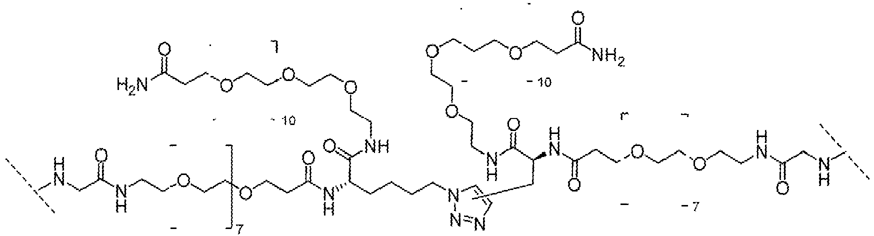 |

### [Chemical formula 37C]

| Linker Structure Number | Linker Name | Structure |
|---|---|---|
| 8 | NOF | 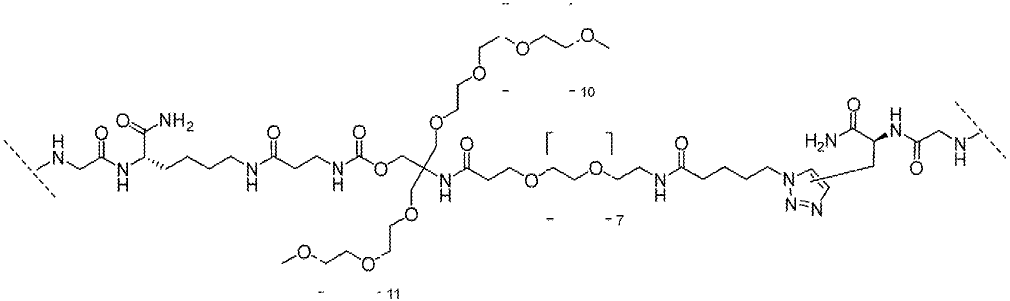 |
| 9 | tag | 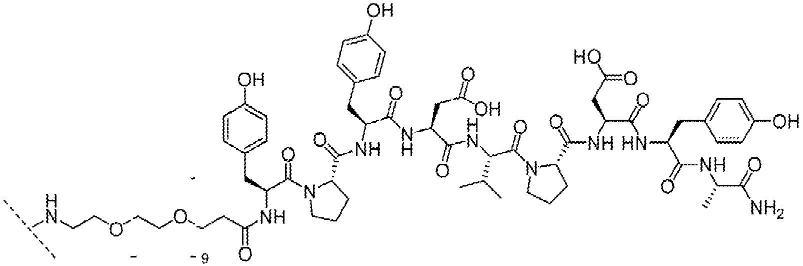 |

**[Table 7A]**

| Table 7 (1 of 3) | | | | | | | |
|---|---|---|---|---|---|---|---|
| Dimer structure number | Peptide sequence number | Linker structure number | Peptide sequence number | Retention time | **ESI** (m/z) | [M+XH] X+ | QC condition |
| | | | | | | | Concentration gradient of B (%) |
| 1 | 1 | 1 | 79 | 4.44 | 1199 | 4 | x |
| 2 | 1 | 2 | 79 | 4.23 | 1146 | 4 | x |
| 3 | 1 | 4 | 79 | 4.37 | 1234 | 4 | x |
| 4 | 1 | 5 | 79 | 4.51 | 1322 | 4 | x |
| 5 | 12 | 2 | 79 | 4.90 | 1155 | 4 | x |
| 6 | 17 | 2 | 79 | 4.72 | 1138 | 4 | x |
| 7 | 19 | 2 | 79 | 4.70 | 1157 | 4 | x |
| 8 | 20 | 2 | 79 | 4.61 | 1138 | 4 | x |
| 9 | 22 | 2 | 79 | 4.88 | 1152 | 4 | x |
| 10 | 6 | 2 | 79 | 4.59 | 1149 | 4 | x |
| 11 | 67 | 2 | 79 | 4.80 | 1148 | 4 | x |
| 12 | 7 | 2 | 79 | 4.86 | 1141 | 4 | x |
| 13 | 35 | 2 | 79 | 4.94 | 1151 | 4 | x |
| 14 | 17 | 4 | 79 | 4.76 | 1226 | 4 | x |
| 15 | 35 | 2 | 85 | 4.20 | 1152 | 4 | x |
| 16 | 35 | 2 | 99 | 4.12 | 1152 | 4 | x |
| 17 | 35 | 2 | 145 | 3.84 | 1152 | 4 | x |
| 18 | 35 | 2 | 90 | 4.60 | 1162 | 4 | x |
| 19 | 68 | 2 | 79 | 4.56 | 1152 | 4 | x |
| 20 | 35 | 2 | 98 | 4.50 | 1198 | 4 | x |
| 21 | 35 | 2 | 100 | 4.74 | 1152 | 4 | x |
| 22 | 69 | 2 | 79 | 4.54 | 1164 | 4 | x |
| 23 | 33 | 2 | 145 | 4.06 | 1156 | 4 | x |
| 24 | 41 | 2 | 145 | 4.39 | 1142 | 4 | x |
| 25 | 51 | 2 | 145 | 3.75 | 1153 | 4 | x |
| 26 | 70 | 2 | 145 | 3.18 | 1156 | 4 | x |
| 27 | 71 | 2 | 145 | 2.93 | 1167 | 4 | x |
| 28 | 72 | 2 | 145 | 3.16 | 1167 | 4 | x |
| 29 | 77 | 2 | 145 | 5.59 | 1175 | 4 | y |
| 30 | 78 | 2 | 145 | 5.79 | 1175 | 4 | y |

### [Table 7B]

| Table 7 (2 of 3) | | | | | | | |
|---|---|---|---|---|---|---|---|
| Dimer structure number | Peptide sequence number | Linker structure number | Peptide sequence number | Retention time | ESI (m/z) | [M+XH] X+ | QC condition |
| | | | | | | | Concentration gradient of B (%) |
| 31 | 73 | 2 | 145 | 5.84 | 1179 | 4 | y |
| 32 | 7 | 2 | 150 | 3.98 | 1152 | 4 | x |
| 33 | 7 | 2 | 152 | 3.88 | 1153 | 4 | x |
| 34 | 7 | 2 | 154 | 3.97 | 764 | 6 | x |
| 35 | 7 | 2 | 159 | 3.98 | 1156 | 4 | x |
| 36 | 7 | 2 | 160 | 4.01 | 1156 | 4 | x |
| 37 | 7 | 2 | 161 | 3.75 | 1168 | 4 | x |
| 38 | 7 | 2 | 162 | 3.78 | 1178 | 4 | x |
| 39 | 74 | 2 | 161 | 3.26 | 1168 | 4 | x |
| 40 | 75 | 2 | 161 | 3.22 | 1189 | 4 | x |
| 41 | 66 | 2 | 161 | 3.65 | 1175 | 4 | x |
| 42 | 66 | 2 | 162 | 4.98 | 1185 | 4 | x |
| 43 | 66 | 2 | 163 | 4.97 | 1164 | 4 | x |
| 44 | 35 | 6 | 79 | 5.04 | 1451 | 4 | x |
| 45 | 35 | 7 | 79 | 5.16 | 1232 | 5 | x |
| 46 | 35 | 8 | 79 | 5.40 | 1484 | 4 | x |
| 47 | 35 | 8 | 79 | 5.42 | 1484 | 4 | x |
| 48 | 1 | 6 | 79 | 4.73 | 1445 | 4 | x |
| 49 | 1 | 7 | 79 | 4.86 | 1227 | 5 | x |
| 50 | 7 | 2 | 145 | 4.07 | 1142 | 4 | x |
| 51 | 60 | 2 | 145 | 2.86 | 1142 | 4 | x |
| 52 | 60 | 7 | 161 | 4.16 | 1245 | 5 | x |
| 53 | 75 | 7 | 161 | 4.14 | 1262 | 5 | x |
| 54 | 66 | 7 | 161 | 4.76 | 1251 | 5 | x |
| 55 | 66 | 7 | 162 | 4.88 | 1259 | 5 | x |
| 56 | 66 | 7 | 163 | 4.86 | 1242 | 5 | x |
| 57 | 74 | 7 | 161 | 4.09 | 1251 | 5 | x |
| 58 | 74 | 2 | 161 | 5.43 | 1175 | 4 | y |
| 59 | 76 | 7 | 161 | 4.06 | 1259 | 5 | x |
| 60 | 76 | 2 | 161 | 5.40 | 1186 | 4 | y |

**[Table 7C]**

| Table 7 (1 of 3) | | | | | | | |
|---|---|---|---|---|---|---|---|
| Dimer structure number | Peptide sequence number | Linker structure number | Peptide sequence number | Retention time | ESI (m/z) | [M+XH] X+ | QC condition |
| | | | | | | | Concentration gradient of B (%) |
| 61 | 74 | 7 | 162 | 4.12 | 1259 | 5 | z |
| 62 | 74 | 2 | 162 | 5.80 | 1186 | 4 | y |
| 63 | 74 | 7 | 163 | 4.10 | 1242 | 5 | x |
| 64 | 74 | 2 | 163 | 5.44 | 1165 | 4 | y |
| 65 | 76 | 2 | 163 | 3.01 | 1175 | 4 | x |
| 66 | 76 | 3 | 163 | 2.80 | 1176 | 4 | x |
| 67 | 76 | 7 | 163 | 3.58 | 1251 | 5 | x |

### [Example 8]

### Evaluation of Binding Activity to Frizzled using ELISA

In order to evaluate the binding activity of the synthesized peptide complexes (peptide complexes having the linker structure number 9 added to peptide sequence numbers 1 to 78) to Frizzled, a binding activity evaluation using the ELISA method was conducted using the steps described below.

Biotin-labeled polyclonal anti-human IgG (eBioscience; 13-4998) was added to a streptavidin-coated 96-well plate (Thermo; 436014) in an amount of 200 µg per well, and left to stand for 30 minutes.

Following washing three times with PBS-T, 3 pmol of Fc-tagged Frizzled-2 (R&D systems; 1307-FZ), Frizzled-7 (R&D systems; 6178-FZ), or Frizzled-8 (R&D systems; 6129-FZ) was added per well and left to stand for 30 minutes to immobilize each Frizzled on the plate.

Following washing three times with PBS-T, the peptide complex diluted to 10 nM was added to Frizzled-2 and Frizzled-7, the peptide complex diluted to 500 nM was added to Frizzled-8, and the plate was left to stand for one hour.

Following washing three times with PBS-T, Anti-HA-tag mAb-HRP-Direct T (MBL; M180-7) that had been diluted 5000-fold with PBS-T was added and left to stand for 30 minutes.

Following washing three times with PBS-T, SureBlue TMB I-Component Microwell Peroxidase Substrate (SeraCare; 5120-0077) was added and left to stand for 10 minutes, and the reaction was then halted by adding an equal volume of TMB Stop Solution (SeraCare; 5150-0021).

Using an Infinite M Nano* (TECAN), the absorbance was measured at 450 nm. The binding signal for each peptide complex was calculated by subtracting the absorbance in those wells containing no added peptide complex from the absorbance in those wells containing the added peptide.

The results are shown in Table 8. The synthesized peptide complexes were shown to have binding activity to Frizzled-2 (FZD2), Frizzled-7 (FZD7), and Frizzled-8 (FZD8).

**[Table 8A]**

| Table 8 (1 of 3) | | | |
|---|---|---|---|
| Peptide sequence number | ELISA signal | | |
| | Frizzled-2 (FZD2) | Frizzled-7 (FZD7) | Frizzled-8 (FZD8) |
| 1 | 1.5 | 1.4 | 0.7 |
| 2 | 0.0 | 0.0 | 0.4 |
| 3 | 0.3 | 0.3 | 0.1 |
| 4 | 0.3 | 0.3 | 0.0 |
| 5 | 0.3 | 0.3 | 0.1 |
| 6 | 1.7 | 1.7 | 1.5 |
| 7 | 1.7 | 1.6 | 1.3 |
| 8 | 1.2 | 1.1 | 0.5 |
| 9 | 1.2 | 1.2 | 0.1 |
| 10 | 1.3 | 1.2 | 0.4 |
| 11 | 1.2 | 1.1 | 0.4 |
| 12 | 1.2 | 1.2 | 0.2 |
| 13 | 0.2 | 0.1 | 0.5 |
| 14 | 1.2 | 0.7 | 0.5 |
| 15 | 1.2 | 1.1 | 0.4 |
| 16 | 1.4 | 1.4 | 0.4 |
| 17 | 1.3 | 1.3 | 0.7 |
| 18 | 1.4 | 1.4 | 0.2 |
| 19 | 1.6 | 1.5 | 0.3 |
| 20 | 1.3 | 1.3 | 0.5 |
| 21 | 1.2 | 1.1 | 0.4 |
| 22 | 1.2 | 1.2 | 0.7 |
| 23 | 1.2 | 1.2 | 0.5 |
| 24 | 1.0 | 0.8 | 0.8 |
| 25 | 1.1 | 1.1 | 0.5 |
| 26 | 1.0 | 1.0 | 0.7 |
| 27 | 1.4 | 1.3 | 1.0 |
| 28 | 1.3 | 1.3 | 1.1 |
| 29 | 1.5 | 1.5 | 1.3 |
| 30 | 1.6 | 1.5 | 1.3 |

**[Table 8B]**

| Table 8 (2 of 3) | | | |
|---|---|---|---|
| Peptide sequence number | ELISA signal | | |
| | Frizzled-2 (FZD2) | Frizzled-7 (FZD7) | Frizzled-8 (FZD8) |
| 31 | 1.5 | 1.5 | 1.4 |
| 32 | 1.6 | 1.5 | 1.4 |
| 33 | 1.3 | 1.4 | 1.5 |
| 34 | 1.5 | 1.5 | 1.1 |
| 35 | 1.4 | 1.4 | 0.6 |
| 36 | 1.5 | 1.5 | 0.7 |
| 37 | 1.4 | 1.5 | 0.7 |
| 38 | 0.2 | 0.2 | 0.8 |
| 39 | 1.3 | 1.3 | 1.2 |
| 40 | 1.3 | 1.3 | 1.2 |
| 41 | 1.5 | 1.4 | 1.2 |
| 42 | 1.4 | 1.4 | 1.2 |
| 43 | 1.3 | 1.3 | 1.2 |
| 44 | 1.4 | 1.4 | 1.3 |
| 45 | 0.6 | 0.6 | 0.9 |
| 46 | 0.2 | 0.2 | 0.4 |
| 47 | 0.4 | 0.3 | 0.6 |
| 48 | 0.4 | 0.2 | 0.7 |
| 49 | 1.2 | 1.1 | 0.6 |
| 50 | 1.1 | 1.1 | 0.9 |
| 51 | 1.4 | 1.4 | 1.0 |
| 52 | 0.7 | 0.4 | 1.0 |
| 53 | 0.2 | 0.2 | 1.2 |
| 54 | 1.5 | 1.5 | 1.1 |
| 55 | 1.4 | 1.4 | 0.8 |
| 56 | 1.6 | 1.6 | 1.2 |
| 57 | 1.5 | 1.5 | 1.1 |
| 58 | 1.5 | 1.5 | 1.3 |
| 59 | 1.4 | 1.1 | 1.1 |
| 60 | 1.5 | 1.3 | 1.2 |

**[Table 8C]**

| Table 8 (3 of 3) | | | |
|---|---|---|---|
| Peptide sequence number | ELISA signal | | |
| | Frizzled-2 (FZD2) | Frizzled-7 (FZD7) | Frizzled-8 (FZD8) |
| 61 | 0.0 | 0.0 | 0.8 |
| 62 | 1.4 | 1.3 | 1.2 |
| 63 | 1.5 | 1.5 | 1.4 |
| 64 | 0.8 | 0.8 | 1.0 |
| 65 | 1.4 | 1.5 | 1.4 |
| 66 | 1.4 | 1.5 | 1.3 |
| 67 | 1.3 | 1.3 | 0.3 |
| 68 | 1.3 | 1.4 | 0.4 |
| 69 | 1.4 | 1.4 | 0.4 |
| 70 | 1.3 | 1.1 | 0.5 |
| 71 | 1.0 | 0.9 | 0.4 |
| 72 | 0.8 | 0.7 | 0.4 |
| 73 | 1.2 | 1.1 | 0.7 |
| 74 | 1.4 | 1.3 | 0.8 |
| 75 | 1.3 | 1.2 | 0.7 |
| 76 | 1.4 | 1.4 | 0.5 |
| 77 | 1.3 | 1.2 | 0.7 |
| 78 | 1.2 | 1.1 | 0.5 |

### [Example 9]

### Evaluation of Binding Activity to LRP using ELISA

In order to evaluate the binding activity of the synthesized peptide complexes (peptide complexes having the linker structure number 9 added to peptide sequence numbers 79 to 163) to LRP, a binding activity evaluation using the ELISA method was conducted using the steps described below.

### I. Evaluation of Peptide Complex Binding Activity to LRP5

Biotin-labeled LRP5 (ACROBiosystems; LR5-H82E6) was added to a streptavidin-coated 96-well plate (Thermo; 436014) in an amount of 3 pmol per well, and left to stand for 30 minutes to immobilize the LRP5 on the plate.

Following washing three times with PBS-T, the peptide complex diluted to 10 nM was added and left to stand for one hour.

Following washing three times with PBS-T, Anti-HA-tag mAb-HRP-Direct T (MBL; M180-7) that had been diluted 5000-fold with PBS-T was added and left to stand for 30 minutes.

Following washing three times with PBS-T, the detection reagent SureBlue TMB I-Component Microwell Peroxidase Substrate (SeraCare; 5120-0077) was added and left to stand for 10 minutes, and the reaction was then halted by adding an equal volume of TMB Stop Solution (SeraCare; 5150-0021).

Using an Infinite M Nano* (TECAN), the absorbance was measured at 450 nm. The binding signal for each peptide complex was calculated by subtracting the absorbance in those wells containing no added peptide complex from the absorbance in those wells containing the added peptide.

### II. Evaluation of Peptide Complex Binding Activity to LRP6

Biotin-labeled polyclonal anti-human IgG (eBioscience; 13-4998) was added to a streptavidin-coated 96-well plate (Thermo; 436014) in an amount of 200 µg per well, and left to stand for 30 minutes.

Following washing three times with PBS-T, 3 pmol of Fc-tagged LRP6 (R&D systems; 1505-LR) was added per well to immobilize the LRP6 on the plate.

Following washing three times with PBS-T, the peptide complex diluted to 100 nM was added, and the plate was left to stand for one hour. Thereafter, an antibody reaction and absorbance detection were conducted in the same manner as that described above in "I. Evaluation of Peptide Complex Binding Activity to LRP5."

The results are shown in Table 9. The synthesized peptide complexes were shown to have binding activity to LRP5 and LRP6.

**[Table 9A]**

| Table 9 (1 of 3) | | |
|---|---|---|
| Peptide sequence number | ELISA signal | |
| | LRP5 | LRP6 |
| 79 | 1.9 | 0.6 |
| 80 | 1.6 | 0.0 |
| 81 | 1.8 | 0.7 |
| 82 | 0.9 | 0.0 |
| 83 | 1.7 | 0.3 |
| 84 | 0.5 | 0.0 |
| 85 | 2.0 | 0.9 |
| 86 | 1.9 | 0.8 |
| 87 | 1.9 | 0.9 |
| 88 | 1.6 | 0.6 |
| 89 | 1.6 | 0.5 |
| 90 | 1.6 | 0.8 |
| 91 | 1.9 | 0.6 |
| 92 | 1.8 | 0.5 |
| 93 | 2.1 | 0.8 |
| 94 | 1.7 | 0.9 |
| 95 | 1.8 | 0.5 |
| 96 | 1.8 | 0.7 |
| 97 | 1.9 | 0.7 |
| 98 | 1.8 | 0.9 |
| 99 | 2.0 | 1.1 |
| 100 | 1.9 | 0.8 |
| 101 | 1.6 | 0.2 |
| 102 | 1.1 | 0.0 |
| 103 | 1.0 | 0.2 |
| 104 | 1.6 | 0.0 |
| 105 | 2.1 | 0.7 |
| 106 | 1.8 | 0.5 |
| 107 | 1.5 | 1.3 |
| 108 | 1.5 | 0.8 |
| 109 | 1.4 | 1.0 |
| 110 | 1.8 | 0.8 |

**[Table 9B]**

| Table 9 (2 of 3) | | |
|---|---|---|
| Peptide sequence number | ELISA signal | |
| | LRP5 | LRP6 |
| 111 | 1.8 | 0.4 |
| 112 | 1.5 | 0.1 |
| 113 | 1.8 | 0.7 |
| 114 | 1.7 | 0.2 |
| 115 | 1.4 | 0.2 |
| 116 | 1.3 | 0.1 |
| 117 | 1.4 | 0.1 |
| 118 | 1.1 | 0.1 |
| 119 | 2.0 | 0.9 |
| 120 | 2.2 | 1.2 |
| 121 | 2.2 | 1.1 |
| 122 | 2.3 | 1.2 |
| 123 | 2.3 | 0.9 |
| 124 | 2.1 | 0.9 |
| 125 | 2.1 | 1.0 |
| 126 | 2.1 | 0.7 |
| 127 | 1.9 | 0.6 |
| 128 | 2.0 | 0.7 |
| 129 | 1.8 | 0.5 |
| 130 | 2.2 | 0.7 |
| 131 | 2.2 | 0.9 |
| 132 | 1.9 | 0.8 |
| 133 | 2.1 | 0.7 |
| 134 | 1.1 | 0.2 |
| 135 | 2.2 | 0.5 |
| 136 | 2.1 | 1.0 |
| 137 | 1.1 | 0.1 |
| 138 | 1.9 | 0.1 |
| 139 | 1.7 | 0.2 |
| 140 | 2.1 | 0.5 |

**[Table 9C]**

| Table 9 (3 of 3) | | |
|---|---|---|
| Peptide sequence number | ELISA signal | |
| | LRP5 | LRP6 |
| 141 | 2.0 | 1.0 |
| 142 | 1.1 | 0.0 |
| 143 | 1.7 | 0.6 |
| 144 | 1.3 | 0.3 |
| 145 | 1.9 | 1.1 |
| 146 | 1.9 | 1.2 |
| 147 | 1.9 | 1.2 |
| 148 | 1.8 | 1.2 |
| 149 | 2.1 | 0.9 |
| 150 | 1.8 | 1.2 |
| 151 | 1.8 | 1.2 |
| 152 | 0.1 | 0.6 |
| 153 | 2.0 | 1.0 |
| 154 | 1.9 | 1.3 |
| 155 | 0.2 | 0.7 |
| 156 | 2.0 | 1.3 |
| 157 | 1.9 | 1.2 |
| 158 | 0.5 | 1.0 |
| 159 | 1.9 | 1.2 |
| 160 | 1.8 | 1.2 |
| 161 | 0.1 | 0.7 |
| 162 | 1.8 | 1.5 |
| 163 | 2.0 | 1.4 |

### [Example 10]

### Evaluation of Wnt3a Inhibition Activity

In order to evaluate the Wnt3a inhibition activity of the synthesized peptides (peptide sequence numbers 79, 81, 83, 85 to 96, and 98 to 100), inhibition of transcriptional activity was assessed using a TCF/LEF reporter.

TCF/LEF Reporter-HEK 293 cells (BPS Bioscience) were cultured in EMEM (Fuji FILM) containing 10% FBS (Thermo Fisher Scientific), 50 µg/mL Gentamicin (Nacalai Tesque), and 400 µg/mL Geneticin (Thermo Fisher Scientific).

The cells were detached using TrypLE (Thermo Fisher Scientific) and then suspended in EMEM containing 10% FBS, 50 µg/mL Gentamicin, and 10 mM LiCl (Sigma).

The cells were seeded into a white 96-well plate (Thermo Fisher Scientific) for cell adhesion and luminescence detection at a rate of 35,000 cells per well, and were then cultured overnight.

Following addition of the peptide, 1 nM Recombinant Human R-spondin 1 (R&D Systems) and 10 nM Wnt3a were added. Stimulation was conducted for 5 hours and 30 minutes in a CO₂ incubator at 37°C.

The ONE-Glo Luciferase Assay System (Peomega) was added and shaken for 4 minutes, and the resulting luminescent signals were detected using a SpectraMax Paradigm multimode microplate reader (Molecular Devices). The thus obtained signals were analyzed using a GraphPad Prism, and the percentage inhibition of each peptide was calculated with the signal value induced by Wnt3a and R-spondin deemed to be 0% inhibition and the signal value with no addition of Wnt3a or R-spondin deemed to be 100% inhibition.

Tests were conducted with the added peptide concentration set to 1 nM, 10 nM, and 100 nM.

Regarding the percentage inhibition values, peptides that exhibited 50% or more inhibition activity when 1 nM of the peptide was added were designated 1-a, peptides that exhibited 50% or more inhibition activity when 10 nM of the peptide was added were designated 1-b, peptides that exhibited 50% or more inhibition activity when 100 nM of the peptide was added were designated 1-c, and peptides that exhibited at least 1% but less than 50% inhibition activity when 100 nM of the peptide was added were designated 1-d.

The results are shown in Table 10. The synthesized peptides were shown to have Wnt3a inhibition activity.

**[Table 10]**

| Peptide sequence number | TCF/LEF Reporter Assay Result |
|---|---|
| 79 | 1-b |
| 81 | 1-b |
| 83 | 1-b |
| 85 | 1-b |
| 86 | 1-b |
| 87 | 1-b |
| 88 | 1-b |
| 89 | 1-b |
| 90 | 1-b |
| 91 | 1-b |
| 92 | 1-c |
| 93 | 1-b |
| 94 | 1-b |
| 95 | 1-b |
| 96 | 1-b |
| 98 | 1-a |
| 99 | 1-a |
| 100 | 1-b |

### [Example 11]

### Evaluation of Wnt Signal Activation Capability

In order to evaluate the Wnt signal activation capability of each of the synthesized peptide complexes (dime structure numbers 1 to 67), transcriptional activity was evaluated using a TCF/LEF reporter.

TCF/LEF Reporter-HEK 293 cells (BPS Bioscience) were cultured in EMEM (Fuji FILM) containing 10% FBS (Thermo Fisher Scientific), 50 µg/mL Gentamicin (Nacalai Tesque), and 400 µg/mL Geneticin (Thermo Fisher Scientific).

The cells were detached using TrypLE (Thermo Fisher Scientific) and then suspended in EMEM containing 10% FBS, 50 µg/mL Gentamicin, and 10 mM LiCl (Sigma).

The cells were seeded into a white 96-well plate (Thermo Fisher Scientific) for cell adhesion and luminescence detection at a rate of 35,000 cells per well, and were then cultured overnight.

Subsequently, Recombinant Human Wnt-3a Protein (R&D systems) or a peptide complex was added in the presence of 1 nM Recombinant Human R-spondin 1 (R&D systems), and stimulation was conducted for 5 hours and 30 minutes in a CO₂ incubator at 37°C.

The ONE-Glo Luciferase Assay System (Peomega) was added and shaken for 4 minutes, and the resulting luminescent signals were detected using a SpectraMax Paradigm multimode microplate reader (Molecular Devices).

The thus obtained signals were analyzed using a GraphPad Prism, and the percentage activity was calculated with the maximum value of the signal value induced by Wnt3a deemed to be 100% and the signal value with no stimulus deemed to be 0%. Evaluations were conducted with the Wnt concentration within a range from 0.03 to 30 mM to determine the 100% value.

For each dimer structure number, tests were conducted using the peptide complex addition concentrations described below.
a) For dimer structure number 1, 1 nM, 10 nM, 100 nM, and 1,000 nM
b) For dimer structure numbers 2 to 4, 1 nM, 10 nM, and 100 nM
c) For dimer structure numbers 5 to 14, 0.1 nM, 1 nM, 10 nM, and 100 nM
d) For dimer structure numbers 15 to 43, 0.1 nM, 1 nM, and 10 nM
e) For dimer strength numbers 44 to 56, six points starting at 30 nM and then reducing by a common ratio of 1/4 (0.03 nM, 0.12 nM, 0.47 nM, 1.9 nM, 7.5 nM and 30 nM)
f) For dimer strength numbers 57 to 67, six points starting at 30 nM and then reducing by a common ratio of 1/5 (0.0096, 0.048, 0.24, 1.2, 6.0, and 30 nM)

Regarding the percentage activity values, peptide complexes that exhibited 50% or more activity when 0.1 nM of the peptide complex was added were designated 2-A, peptide complexes that exhibited 50% or more activity when 1 nM of the peptide complex was added were designated 2-B, peptide complexes that exhibited 50% or more activity when 10 nM of the peptide complex was added were designated 2-C, peptide complexes that exhibited 50% or more activity when 100 nM of the peptide complex was added were designated 2-D, and peptide complexes that exhibited at least 1% but less than 50% activity when 100 nM of the peptide complex was added were designated 2-E.

Peptide complexes that exhibited 50% or more activity when 0.03 nM of the peptide complex was added were designated 3-A, peptide complexes that exhibited 50% or more activity when 0.12 nM of the peptide complex was added were designated 3-B, peptide complexes that exhibited 50% or more activity when 0.47 nM of the peptide complex was added were designated 3-C, peptide complexes that exhibited 50% or more activity when 1.9 nM of the peptide complex was added were designated 3-D, peptide complexes that exhibited 50% or more activity when 7.5 nM of the peptide complex was added were designated 3-E, peptide complexes that exhibited 50% or more activity when 30 nM of the peptide complex was added were designated 3-F, and peptide complexes that exhibited at least 1% but less than 50% activity when 30 nM of the peptide complex was added were designated 3-G.

Peptide complexes that exhibited 50% or more activity when 0.0096 nM of the peptide complex was added were designated 4-A, peptide complexes that exhibited 50% or more activity when 0.048 nM of the peptide complex was added were designated 4-B, peptide complexes that exhibited 50% or more activity when 0.24 nM of the peptide complex was added were designated 4-C, peptide complexes that exhibited 50% or more activity when 1.2 nM of the peptide complex was added were designated 4-D, peptide complexes that exhibited 50% or more activity when 6 nM of the peptide complex was added were designated 4-E, peptide complexes that exhibited 50% or more activity when 30 nM of the peptide complex was added were designated 4-F, and peptide complexes that exhibited at least 1% but less than 50% activity when 30 nM of the peptide complex was added were designated 4-G.

The results are shown in Table 11. The synthesized peptides were shown to have Wnt signal activation capabilities, similar to the recombinant protein Wnt3a.

**[Table 11A]**

| Table 11 (1 of 3) | |
|---|---|
| Dimer structure number | TCF/LEF Reporter Assay Result |
| 1 | 2-C |
| 2 | 2-C |
| 3 | 2-C |
| 4 | 2-C |
| 5 | 2-C |
| 6 | 2-B |
| 7 | 2-C |
| 8 | 2-C |
| 9 | 2-C |
| 10 | 2-B |
| 11 | 2-B |
| 12 | 2-B |
| 13 | 2-B |
| 14 | 2-B |
| 15 | 2-B |
| 16 | 2-A |
| 17 | 2-B |
| 18 | 2-B |
| 19 | 2-B |
| 20 | 2-B |
| 21 | 2-B |
| 22 | 2-B |
| 23 | 2-B |
| 24 | 2-B |
| 25 | 2-B |
| 26 | 2-B |
| 27 | 2-B |
| 28 | 2-B |
| 29 | 2-B |
| 30 | 2-B |

**[Table 11B]**

| Table 11 (2 of 3) | |
|---|---|
| Dimer structure number | TCF/LEF Reporter Assay Result |
| 31 | 2-B |
| 32 | 2-B |
| 33 | 2-B |
| 34 | 2-B |
| 35 | 2-B |
| 36 | 2-B |
| 37 | 2-B |
| 38 | 2-A |
| 39 | 2-B |
| 40 | 2-B |
| 41 | 2-B |
| 42 | 2-A |
| 43 | 2-A |
| 44 | 3-D |
| 45 | 3-D |
| 46 | 3-D |
| 47 | 3-D |
| 48 | 3-F |
| 49 | 3-F |
| 50 | 3-D |
| 51 | 3-D |
| 52 | 3-C |
| 53 | 3-C |
| 54 | 3-C |
| 55 | 3-C |
| 56 | 3-B |
| 57 | 4-D |
| 58 | 4-D |
| 59 | 4-D |
| 60 | 4-D |

**[Table 11C]**

| Table 11 (3 of 3) | |
|---|---|
| Dimer structure number | TCF/LEF Reporter Assay Result |
| 61 | 4-D |
| 62 | 4-C |
| 63 | 4-C |
| 64 | 4-C |
| 65 | 4-C |
| 66 | 4-C |
| 67 | 4-C |

### Industrial Applicability

The peptide complex of the present invention binds to a Frizzled receptor and a co-receptor LRP5 and/or LRP6 to activate the Wnt signaling pathway. Consequently, the peptide complex of the present invention is useful for producing various organoids of the small intestine, large intestine, stomach, pancreas, and lung and the like. Further, the peptide of the present invention binds to LRP5 and/or LRP6 and exhibits Wnt inhibition activity. Accordingly, the peptide of the present invention can be used as a drug for inhibiting the Wnt signaling pathway.

## Claims

1. A peptide comprising an amino acid sequence represented by formula A1, or an amino acid sequence in which one or a plurality of amino acid residues have been substituted, deleted, added or inserted within an amino acid sequence represented by formula A1:
A1: X1-X2-X3-X4-X5-X6-X7-X8-X9-X10-X11-X12-X13-X14
wherein
X1 represents any D-amino acid residue,
X2 and X3 each independently represent any amino acid residue,
X4 and X5 each independently represent an amino acid residue having an aliphatic hydrocarbon group which may be substituted in a side chain,
X6 represents an amino acid residue having an aryl group which may be substituted in a side chain,
X7 represents an N-alkyl amino acid residue having an aryl group which may be substituted or a cycloalkyl group which may be substituted in a side chain,
X8 represents any amino acid residue,
X9 represents an L-threonine (T) residue,
X10 represents any N-alkylated amino acid residue,
X11 represents any amino acid residue,
X12 represents an amino acid residue having an aliphatic hydrocarbon group in a side chain,
X13 represents an amino acid residue having an aliphatic hydrocarbon group which may be substituted in a side chain,
X14 represents an L-cysteine (C) residue, and
in formula A1, the amino acid sequence is described from an N-terminal side to a C-terminal side.

2. The peptide according to Claim 1, wherein in formula A1:
X1 represents a D-phenylalanine (df) residue, (R)-2-amino-3-(pyridin-4-yl)propanoic acid (d4py) residue, (R)-2-amino-3-(pyridin-3-yl)propanoic acid (d3py) residue, (R)-2-amino-3-(3-methoxyphenyl)propanoic acid (df3OMe) residue, (R)-2-amino-3-(4-methoxyphenyl)propanoic acid (df4OMe) residue, (R)-2-amino-5-ureidopentanoic acid (dicit) residue, D-tyrosine (dy) residue, or (R)-2-amino-3-(4-fluorophenyl)propanoic acid (df4F) residue,
X2 represents an L-serine (S) residue, L-asparagine (N) residue, L-aspartic acid (D) residue, L-2-aminoadipic acid (Hgl) residue, L-citrulline (Cit) residue, or (S)-2-amino-6-morpholinohexanoic acid (Kmor) residue,
X3 represents an L-aspartic acid (D) residue, L-arginine (R) residue, L-citrulline (Cit) residue, N6-(4-(carboxymethyl)piperazine-1-carbonyl)-L-lysine (KCOpipzaa) residue, (2S)-2-amino-3-(oxan-4-yl)propanoic acid (Atp) residue, L-asparagine (N) residue, or L-serine (S) residue,
X4 represents an L-isoleucine (I) residue or O-methyl-L-threonine (TMe) residue,
X5 represents an L-arginine (R) residue, L-glutamic acid (E) residue, (S)-2-aminoheptanoic acid (Ahp) residue, L-serine (S) residue, L-homoserine (Hse) residue, L-citrulline (Cit) residue, N6-carbamoyl-L-lysine (Hcit) residue, (S)-2-amino-4-ureidobutanoic acid (Ncit) residue, or (S)-5-acetamido-2-aminopentanoic acid (OrnAc) residue,
X6 represents an L-tyrosine (Y) residue, methyl-L-tyrosine (MeY) residue, (S)-2-amino-3-(4-fluorophenyl)propanoic acid (F4F) residue, (S)-2-amino-3-(4-chlorophenyl)propanoic acid (F4C) residue, (S)-2-amino-3-(pyridin-4-yl)propanoic acid (4Py) residue, or (S)-3-(2-amino-2-carboxyethyl)benzoic acid (F3COO) residue,
X7 represents a methyl-L-phenylalanine (MeF) residue, (S)-2-(methylamino)-3-(pyridin-3-yl)propanoic acid (Me3Py) residue, (S)-2-(methylamino)-3-(pyridin-4-yl)propanoic acid (Me4Py) residue, (S)-3-(2-carboxy-2-(methylamino)ethyl)benzoic acid (MeF3COO) residue, (S)-4-(2-carboxy-2-(methylamino)ethyl)benzoic acid (MeF4COO) residue, or (S)-2-(methylamino)-3-(tetrahydro-2H-pyran-4-yl)propanoic acid (MeAtp) residue,
X8 represents an L-glutamine (Q) residue, L-valine (V) residue, L-2-aminoadipic acid (Hgl) residue, 1-(carboxymethyl)-L-tryptophan (W1aa) residue, (S)-2-aminoheptanoic acid (Ahp) residue, homo-L-tyrosine (Hty) residue, L-threonine (T) residue, allothreonine (alT) residue, L-citrulline (Cit) residue, N6-(4-(carboxymethyl)piperazine-1-carbonyl) -L-lysine (KCOpipzaa) residue, (S)-2-amino-2-(tetrahydro-2H-pyran-4-yl)acetic acid (Gthp) residue, (S)-2-amino-2-(1-(carboxymethyl)piperidin-4-yl)acetic acid (G4pipaa) residue, (S)-2-amino-2-cyclobutylacetic acid (Cbg) residue, (S)-2-amino-2-cyclohexylacetic acid (Chg) residue, or O-methyl-threonine (TMe) residue,
X10 represents a methyl-L-phenylalanine (MeF) residue, (S)-2-(methylamino)hexanoic acid (MeNle) residue, (S)-2-(methylamino)-3-(pyridin-3-yl)propanoic acid (Me3Py) residue, (S)-2-(methylamino)-3-(pyridin-4-yl)propanoic acid (Me4Py) residue, (S)-4-(2-carboxy-2-(methylamino)ethyl)benzoic acid (MeF4COO) residue, or (S)-2-(methylamino)-3-(tetrahydro-2H-pyran-4-yl)propanoic acid (MeAtp) residue,
X11 represents an L-isoleucine (I) residue, L-tyrosine (Y) residue, (S)-2-amino-3-(4-(carboxymethoxy)phenyl)propanoic acid (F4aao) residue, or O-methyl-L-threonine (TMe) residue,
X12 represents an L-valine (V) residue, methyl-L-valine (MeV) residue, or N-methyl-L-cyclohexylglycine (MeChg) residue, and
X13 represents a glycine (G) residue, D-alanine (da) residue, or D-serine (ds) residue.

3. A peptide comprising an amino acid sequence represented by formula A2, or an amino acid sequence in which at least one amino acid residue among 1 to 12 amino acid residues selected from the group consisting of a 1st, 2nd, 3rd, 4th, 5th, 6th, 7th, 8th, 10th, 11th, 12th and 13th amino acid residues in an amino acid sequence represented by formula A2 has been substituted, deleted, added or inserted:
A2: df-S-D-I-R-Y-MeF-Q-T-MeF-I-V-G-C (SEQ ID NO: 164)
wherein
df represents a D-phenylalanine residue,
S represents an L-serine residue,
D represents an L-aspartic acid residue,
I represents an L-isoleucine residue,
R represents an L-arginine residue,
Y represents an L-tyrosine residue,
MeF represents a methyl-L-phenylalanine residue,
Q represents an L-glutamine residue,
T represents an L-threonine residue,
V represents an L-valine residue,
G represents a glycine residue, and
C represents an L-cysteine residue.

4. The peptide according to any one of Claims 1 to 3, further having a glycine residue at a C-terminal.

5. The peptide according to any one of Claims 1 to 3, wherein the peptide is a cyclic peptide.

6. The peptide according to Claim 4, wherein the peptide is a cyclic peptide

7. The peptide according to any one of Claims 1 to 3, having a cyclic structure in which an amino acid residue derived from a chloroacetylated amino acid and an L-cysteine residue contained within the peptide are bonded together.

8. The peptide according to any one of Claims 1 to 3, further comprising an additional amino acid residue.

9. The peptide according to any one of Claims 1 to 3, having binding properties to an Fzd receptor.

10. The peptide according to Claim 9, having the ability to bind to at least one receptor selected from the group consisting of an Fzd receptor 2, Fzd receptor 7 and Fzd receptor 8.

11. The peptide according to any one of Claims 1 to 3, wherein the peptide has an amino acid sequence represented by formula A3:
A3: ClAc-X1-X2-X3-X4-X5-X6-X7-X8-X9-X10-X11-X12-X13-X14
wherein
ClAc represents a chloroacetyl group, X1, X2, X3, X4, X5, X6, X7, X8, X9, X10, X11, X12, X13, and X14 represent amino acid residues shown in Table 1, and in Table 1,
df represents a D-phenylalanine residue,
d4py represents an (R)-2-amino-3-(pyridin-4-yl)propanoic acid residue,
d3py represents an (R)-2-amino-3-(pyridin-3-yl)propanoic acid residue,
df-3OMe represents an (R)-2-amino-3-(3-methoxyphenyl)propanoic acid residue,
df4OMe represents an (R)-2-amino-3-(4-methoxyphenyl)propanoic acid residue,
dcit represents an (R)-2-amino-5-ureidopentanoic acid residue,
dy represents a D-tyrosine residue,
df4F represents an (R)-2-amino-3-(4-fluorophenyl)propanoic acid residue,
S represents an L-serine residue,
N represents an L-asparagine residue,
D represents an L-aspartic acid residue,
Hgl represents an L-2-aminoadipic acid residue,
Cit represents an L-citrulline residue,
Kmor represents an (S)-2-amino-6-morpholinohexanoic acid residue,
R represents an L-arginine residue,
KCOpipzaa represents an N6-(4-(carboxymethyl)piperazine-1-carbonyl)-L-lysine residue,
Atp represents a (2S)-2-amino-3-(oxan-4-yl)propanoic acid residue,
I represents an L-isoleucine residue,
TMe represents an O-methyl-L-threonine residue,
E represents an L-glutamic acid residue,
Ahp represents an (S)-2-aminoheptanoic acid residue,
Hse represents an L-homoserine residue,
Hcit represents an N6-carbamoyl-L-lysine residue,
Neit represents an (S)-2-amino-4-ureidobutanoic acid residue,
OrnAc represents an (S)-5-acetamido-2-aminopentanoic acid residue,
Y represents an L-tyrosine residue,
MeY represents a methyl-L-tyrosine residue,
F4F represents an (S)-2-amino-3-(4-fluorophenyl)propanoic acid residue,
F4C represents an (S)-2-amino-3-(4-chlorophenyl)propanoic acid residue,
4Py represents an (S)-2-amino-3-(pyridin-4-yl)propanoic acid residue,
F3COO represents an (S)-3-(2-amino-2-carboxyethyl)benzoic acid residue,
MeF represents a methyl-L-phenylalanine residue,
Me3Py represents an (S)-2-(methylamino)-3-(pyridin-3-yl)propanoic acid residue,
Me4Py represents an (S)-2-(methylamino)-3-(pyridin-4-yl) ) propanoic acid residue,
MeF3COO represents an (S)-3-(2-carboxy-2-(methylamino)ethyl)benzoic acid residue,
MeF4COO represents an (S)-4-(2-carboxy-2-(methylamino)ethyl)benzoic acid residue,
MeAtp represents an (S)-2-(methylamino)-3-(tetrahydro-2H-pyran-4-yl)propanoic acid residue,
Q represents an L-glutamine residue,
V represents an L-valine residue,
W1aa represents a 1-(carboxymethyl)-L-tryptophan residue,
Hty represents a homo-L-tyrosine residue,
T represents an L-threonine residue,
alT represents an allothreonine residue,
Gthp represents an (S)-2-amino-2-(tetrahydro-2H-pyran-4-yl)acetic acid residue,
G4pipaa represents an (S)-2-amino-2-(1-(carboxymethyl)piperidin-4-yl)acetic acid residue,
Cbg represents an (S)-2-amino-2-cyclobutylacetic acid residue,
Chg represents an (S)-2-amino-2-cyclohexylacetic acid residue,
MeNle represents an (S)-2-(methylamino)hexanoic acid residue,
F4aao represents an (S)-2-amino-3-(4-(carboxymethoxy)phenyl)propanoic acid residue,
MeV represents a methyl-L-valine residue,
MeChg represents an N-methyl-L-cyclohexylglycine residue,
G represents a glycine residue, and
C represents a L-cysteine residue.
**[Table 1A]**
| Table 1 (1 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 1 | df | S | D | I | R | Y | MeF | Q | T | MeF | I | C | G | C | G |
| 2 | dcit | S | D | I | R | Y | MeF | Q | T | MeF | I | V | G | C | G |
| 3 | dy | S | D | I | R | Y | MeF | Q | T | MeF | I | V | G | C | G |
| 4 | df4F | S | D | I | R | Y | MeF | Q | T | MeF | I | V | G | C | G |
| 5 | df | S | D | I | R | MeY | MeF | Q | T | MeF | I | V | G | C | G |
| 6 | df | S | D | I | R | Y | MeF | Q | T | MeF | I | MeV | G | C | G |
| 7 | df | S | D | I | R | Y | MeF | V | T | MeF | I | MeV | G | C | G |
| 8 | df | Kmor | D | I | R | Y | MeF | Q | T | MeF | I | V | G | C | G |
| 9 | df | S | R | I | R | Y | MeF | Q | T | MeF | I | V | G | C | G |
| 10 | df | S | Cit | I | R | Y | MeF | Q | T | MeF | I | v | G | C | G |
| 11 | df | S | KCOpipzaa | I | R | Y | MeF | Q | T | MeF | I | V | G | C | G |
| 12 | df | S | Atp | I | R | Y | MeF | Q | T | MeF | I | V | G | C | G |
| 13 | df | S | D | I | E | Y | MeF | Q | T | MeF | I | V | G | C | G |
| 14 | df | S | D | I | Ahp | Y | MeF | Q | T | MeF | I | V | G | C | G |
| 15 | df | S | D | I | R | Y | MeF | Hgl | T | MeF | I | V | G | C | G |
| 16 | df | S | D | I | R | Y | MeF | W1aa | T | MeF | I | V | G | C | G |
| 17 | df | S | D | I | R | Y | MeF | V | T | MeF | I | V | G | C | G |
| 18 | df | S | D | I | R | Y | MeF | Ahp | T | MeF | I | V | G | C | G |
| 19 | df | S | D | I | R | Y | MeF | Hty | T | MeF | I | V | G | C | G |
| 20 | df | S | D | I | R | Y | MeF | T | T | MeF | I | V | G | C | G |
**[Table 1B]**
| Table 1 (2 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 21 | df | S | D | I | R | Y | MeF | a1T | T | MeF | I | V | G | C | G |
| 22 | df | S | D | I | R | Y | MeF | Cit | T | MeF | I | V | G | C | G |
| 23 | df | S | D | I | R | Y | MeF | KCOpipzaa | T | MeF | I | V | G | C | G |
| 24 | df | S | D | I | R | Y | MeF | Q | T | MeNle | I | V | G | C | G |
| 25 | df | S | D | I | R | Y | MeF | Q | T | MeF | Y | V | G | C | G |
| 26 | df | S | D | I | R | Y | MeF | Q | T | MeF | F4aao | V | G | C | G |
| 27 | d4py | S | D | I | R | Y | MeF | V | T | MeF | I | MeV | G | C | G |
| 28 | d3py | S | D | I | R | Y | MeF | V | T | MeF | I | MeV | G | C | G |
| 29 | df30Me | S | D | I | R | Y | MeF | V | T | MeF | I | MeV | G | C | G |
| 30 | df4OMe | S | D | I | R | Y | MeF | V | T | MeF | I | MeV | G | C | G |
| 31 | df | N | D | I | R | Y | MeF | V | T | MeF | I | MeV | G | C | G |
| 32 | df | D | D | I | R | Y | MeF | V | T | MeF | I | MeV | G | C | G |
| 33 | df | Hgl | D | I | R | Y | MeF | V | T | MeF | I | MeV | G | C | G |
| 34 | df | Cit | D | I | R | Y | MeF | V | T | MeF | I | MeV | G | C | G |
| 35 | df | S | Atp | I | R | Y | MeF | V | T | MeF | I | MeV | G | C | G |
| 36 | df | S | N | I | R | Y | MeF | V | T | MeF | I | MeV | G | C | G |
| 37 | df | S | S | I | R | Y | MeF | V | T | MeF | I | MeV | G | C | G |
| 38 | df | S | D | TMe | R | Y | MeF | V | T | MeF | I | MeV | G | C | G |
| 39 | df | S | D | I | S | Y | MeF | V | T | MeF | I | MeV | G | C | G |
| 40 | df | S | D | I | Hse | Y | MeF | V | T | MeF | I | MeV | G | C | G |
**[Table 1C]**
| Table 1 (3 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 41 | df | s | D | I | Cit | Y | MeF | V | T | MeF | I | MeV | G | C | G |
| 42 | df | s | D | I | Hcit | Y | MeF | V | T | MeF | I | MeV | G | C | G |
| 43 | df | s | D | I | Ncit | Y | MeF | V | T | MeF | I | MeV | G | C | G |
| 44 | df | S | D | I | OrnAc | Y | MeF | V | T | MeF | I | MeV | G | C | G |
| 45 | df | S | D | I | R | F4F | MeF | v | T | MeF | I | MeV | G | C | G |
| 46 | df | S | D | I | R | F4C | MeF | V | T | MeF | I | MeV | G | C | G |
| 47 | df | S | D | I | R | 4Py | MeF | v | T | MeF | I | MeV | G | C | G |
| 48 | df | S | D | I | R | F3COO | MeF | V | T | MeF | I | MeV | G | C | G |
| 49 | df | S | D | I | R | Y | Me3Py | v | T | MeF | I | MeV | G | C | G |
| 50 | df | S | D | I | R | Y | Me4Py | v | T | MeF | I | MeV | G | C | G |
| 51 | df | S | D | I | R | Y | MeF3COO | V | T | MeF | I | MeV | G | C | G |
| 52 | df | S | D | I | R | Y | MeF4COO | v | T | MeF | I | MeV | G | C | G |
| 53 | df | S | D | I | R | Y | MeAtp | V | T | MeF | I | MeV | G | C | G |
| 54 | df | S | D | I | R | Y | MeF | Gthp | T | MeF | I | MeV | G | C | G |
| 55 | df | S | D | I | R | Y | MeF | G4pipaa | T | MeF | I | MeV | G | C | G |
| 56 | df | S | D | I | R | Y | MeF | Cbg | T | MeF | I | MeV | G | C | G |
| 57 | df | S | D | I | R | Y | MeF | Chg | T | MeF | I | MeV | G | C | G |
| 58 | df | S | D | I | R | Y | MeF | TMe | T | MeF | I | MeV | G | C | G |
| 59 | df | S | D | I | R | Y | MeF | V | T | Me3Py | I | MeV | G | C | G |
| 60 | df | S | D | I | R | Y | MeF | V | T | Me4Py | I | MeV | G | C | G |
**[Table 1D]**
| Table 1 (4 of 4) | | | | | | | | | | | | | | | |
|---|---|---|---|---|---|---|---|---|---|---|---|---|---|---|---|
| SEQ ID NO: | X1 | X2 | X3 | X4 | X5 | X6 | X7 | X8 | X9 | X10 | X11 | X12 | X13 | X14 | X15 |
| 61 | df | S | D | I | R | Y | MeF | V | T | MeF4COO | I | MeV | G | C | G |
| 62 | df | S | D | I | R | Y | MeF | V | T | MeAtp | I | MeV | G | c | G |
| 63 | df | S | D | I | R | Y | MeF | V | T | MeF | TMe | MeV | G | c | G |
| 64 | df | S | D | I | R | Y | MeF | v | T | MeF | I | MeChg | G | C | G |
| 65 | df | S | D | I | R | Y | MeF | v | T | MeF | I | MeV | da | c | G |
| 66 | df | S | D | I | R | Y | MeF | V | T | MeF | I | MeV | ds | C | G |
| 67 | df | S | Atp | I | R | Y | MeF | V | T | MeF | I | V | G | C | G |
| 68 | df | S | Cit | I | R | Y | MeF | V | T | MeF | I | MeV | G | C | G |
| 69 | df | S | Atp | I | R | Y | MeF | V | T | MeF | Y | MeV | G | c | G |
| 70 | df | Hgl | D | I | R | Y | MeF | v | T | Me4Py | I | MeV | G | C | G |
| 71 | df | Hgl | D | I | R | Y | MeF3COO | V | T | Me4Py | I | MeV | G | C | G |
| 72 | df | Hgl | D | I | Cit | Y | MeF3COO | V | T | Me4Py | I | MeV | G | C | G |
| 73 | df | Hgl | D | I | Cit | Y | MeF3COO | TMe | T | Me4Py | I | MeV | ds | C | G |
| 74 | df | S | D | I | R | Y | MeF | V | T | Me4Py | I | MeV | ds | c | G |
| 75 | df | Hgl | D | I | R | Y | MeF | V | T | Me4Py | I | MeV | ds | C | G |
| 76 | df | S | Cit | I | R | Y | MeF | V | T | Me4Py | I | MeV | ds | c | G |
| 77 | df | Hgl | D | I | R | Y | MeF3COO | V | T | Me4Py | I | MeV | ds | C | G |
| 78 | df | Hgl | D | I | Cit | Y | MeF3COO | V | T | Me4Py | I | MeV | ds | C | G |

12. A peptide comprising an amino acid sequence represented by formula B1, or an amino acid sequence in which one or a plurality of amino acid residues have been substituted, deleted, added or inserted within an amino acid sequence represented by formula B1:
B1: Y1-Y2-Y3-Y4-Y5-Y6-Y7-Y8-Y9-Y10-Y11-Y12-Y13-Y14
wherein
Y1 represents an L-phenylalanine (F) residue which may be substituted,
Y2 represents an L-arginine (R) residue which may be substituted,
Y3 represents an L-tryptophan (W) residue which may be substituted or an (S)-2-amino-3-(naphthalen-1-yl)propanoic acid (Nall) residue which may be substituted,
Y4 represents an acidic amino acid residue or a neutral amino acid residue,
Y5 represents an aromatic amino acid residue which may be substituted, an aliphatic amino acid residue, or an L-proline (P) residue,
Y6 represents an aliphatic amino acid residue which may be substituted,
Y7 represents an N-alkylated aromatic amino acid residue,
Y8 represents an amino acid residue derived from an amino acid having a hydroxy group, carboxy group or amide group in the side chain,
Y9 represents any amino acid residue,
Y10 represents an L-tryptophan (W) residue which may be substituted,
Y11 represents an L-asparagine (N) residue which may be substituted or a glutamine (Q) residue,
Y12 represents an (S)-1,2,3,4-tetrahydroisoquinoline-3-carboxylic acid (Tic) residue which may be substituted,
Y13 represents a neutral amino acid residue or acidic amino acid residue which may be substituted,
Y14 represents an L-cysteine (C) residue, and
in formula B1, the amino acid sequence is described from an N-terminal side to a C-terminal side.

13. The peptide according to Claim 12, wherein in formula B1:
Y1 represents an L-phenylalanine (F) residue, (S)-2-amino-3-(4-fluorophenyl)propanoic acid (F4F) residue, (S)-2-amino-3-(p-toluyl)propanoic acid (F4Me) residue, or (S)-2-amino-3-(m-toluyl)propanoic acid (F3Me) residue,
Y2 represents an L-arginine (R) residue or N6-carbamoyl-L-lysine (Hcit) residue,
Y3 represents an L-tryptophan (W) residue, methyl-L-tryptophan (MeW) residue, (S)-2-amino-3-(1H-pyrrolo[2,3-b]pyridin-3-yl)propanoic acid (W7N) residue, (S)-2-amino-3-(1H-pyrrolo[3,2-c]pyridin-3-yl)propanoic acid (W5N) residue, (S)-2-amino-3-(1-methyl-1H-pyrrolo[2,3-b]pyridin-3-yl)propanoic acid (W1Me7N) residue, (S)-2-amino-3-(5-methyl-1H-pyrrolo[2,3-b]pyridin-3-yl)propanoic acid (W7N5Me) residue, (S)-2-amino-3-(naphthalen-1-yl)propanoic acid (Nall) residue, or (S)-2-amino-3-(quinolin-4-yl)propanoic acid (Nal14N) residue,
Y4 represents an L-aspartic acid (D) residue, L-glutamic acid (E) residue, L-asparagine (N) residue, or L-glutamine (Q) residue,
Y5 represents an L-valine (V) residue, methyl-L-valine (MeV) residue, 1-(carboxymethyl)-L-tryptophan (W1aa) residue, (S)-2-amino-3-cyclohexylpropanoic acid (Cha) residue, (S)-2-amino-3-(naphthalen-1-yl)propanoic acid (Nall) residue, (S)-2-amino-3-(5-phenylpyridin-3-yl)propanoic acid (3Py5Ph) residue, (S)-2-amino-2-cyclobutylacetic acid (Cbg) residue, or L-proline (P) residue,
Y6 represents an L-arginine (R) residue, methyl-L-arginine (MeR) residue, (S)-2-amino-6-morpholinohexanoic acid (Kmor) residue, L-citrulline (Cit) residue, N6-carbamoyl-L-lysine (Hcit) residue, (S)-2-amino-4-ureidobutanoic acid (Ncit) residue, or (S)-5-acetamido-2-aminopentanoic acid (OrnAc) residue,
Y7 represents a methyl-L-phenylalanine (MeF) residue, (S)-4-(2-carboxy-2-(methylamino)ethyl)benzoic acid (MeF4COO) residue, methyl-L-tyrosine (MeY) residue, (S)-2-(methylamino)-3-(p-toluyl)propanoic acid (MeF4Me) residue, (S)-3-(4-chlorophenyl)-2-(methylamino)propanoic acid (MeF4C) residue, (S)-3-(2-carboxy-2-(methylamino)ethyl)benzoic acid (MeF3COO) residue, (S)-2-(methylamino)-3-(m-toluyl)propanoic acid (MeF3Me) residue, or (S)-3-(3-chlorophenyl)-2-(methylamino)propanoic acid (MeF3C) residue,
Y8 represents an L-serine (S) residue, 1-(carboxymethyl)-L-tryptophan (W1aa) residue, homo-L-tyrosine (Hty) residue, allothreonine (alT) residue, L-threonine (T) residue, L-asparagine (N) residue, L-glutamine (Q) residue, L-aspartic acid (D) residue, or L-glutamic acid (E) residue,
Y9 represents an L-asparagine (N) residue, methyl-L-asparagine (MeN) residue, L-2-aminoadipic acid (Hgl) residue, (S)-2-amino-3-cyclohexylpropanoic acid (Cha) residue, homo-L-tyrosine (Hty) residue, (S)-2-amino-4-(1H-pyrrolo[2,3-b]pyridin-3-yl)butanoic acid (Hw7N) residue, N6-(4-(carboxymethyl)piperazine-1-carbonyl)-L-lysine (KCOpipzaa) residue, L-glutamine (Q) residue, L-aspartic acid (D) residue, or L-glutamic acid (E) residue,
Y10 represents an L-tryptophan (W) residue, (S)-2-amino-3-(1H-pyrrolo[2,3-b]pyridin-3-yl)propanoic acid (W7N) residue, (S)-2-amino-3-(1H-pyrrolo[2,3-c]pyridin-3-yl)propanoic acid (W6N) residue, (S)-2-amino-3-(1-methyl-1H-pyrrolo[2,3-b]pyridin-3-yl)propanoic acid (W1Me7N) residue, or (S)-2-amino-3-(5-methyl-1H-pyrrolo[2,3-b]pyridin-3-yl)propanoic acid (W7N5Me) residue,
Y11 represents an L-asparagine (N) residue, L-glutamine (Q) residue, or (2S)-2-amino-4-(methylamino)-4-oxobutanoic acid (Nmm) residue,
Y12 represents an (S)-1,2,3,4-tetrahydroisoquinoline-3-carboxylic acid (Tic) residue, (S)-6-chloro-1,2,3,4-tetrahydroisoquinoline-3-carboxylic acid (Tic6C) residue, (S)-7-hydroxy-1,2,3,4-tetrahydroisoquinoline-3-carboxylic acid (Tic7H) residue, or (S)-7-chloro-1,2,3,4-tetrahydroisoquinoline-3-carboxylic acid (Tic7C) residue, and
Y13 represents an L-asparagine (N) residue, L-aspartic acid (D) residue, L-glutamine (Q) residue, (2S)-2-amino-4-(methylamino)-4-oxobutanoic acid (Nmm) residue, or N4,N4-dimethyl-L-asparagine (Ndm) residue.

14. A peptide comprising an amino acid sequence represented by formula B2, or an amino acid sequence in which at least one amino acid residue among 1 to 13 amino acid residues selected from the group consisting of a 1st, 2nd, 3rd, 4th, 5th, 6th, 7th, 8th, 9th, 10th, 11th, 12th and 13th amino acid residues in an amino acid sequence represented by formula B2 has been substituted, added, deleted or inserted:
B2: F-R-W-D-V-R-MeF-S-N-W-N-Tic-N-C (SEQ ID NO: 165)
wherein
F represents an L-phenylalanine residue,
R represents an L-arginine residue,
W represents an L-tryptophan residue,
D represents an L-aspartic acid residue,
V represents an L-valine residue,
MeF represents a methyl-L-phenylalanine residue,
S represents an L-serine residue,
Tic represents an (S)-1,2,3,4-tetrahydroisoquinoline-3-carboxylic acid residue,
N represents an L-asparagine residue, and
C represents an L-cysteine residue.

15. The peptide according to any one of Claims 12 to 14, further having a glycine residue at a C-terminal.

16. The peptide according to any one of Claims 12 to 14, wherein the peptide is a cyclic peptide.

17. The peptide according to Claim 15, wherein the peptide is a cyclic peptide.

18. The peptide according to any one of Claims 12 to 14, having a cyclic structure in which an amino acid residue derived from a chloroacetylated amino acid and an L-cysteine residue contained within the peptide are bonded together.

19. The peptide according to any one of Claims 12 to 14, further comprising an additional amino acid residue.

20. The peptide according to any one of Claims 12 to 14, having binding properties to LRP.

21. The peptide according to Claim 20, having an ability to bind to at least one receptor selected from the group consisting of LRP5 and LRP6.

22. The peptide according to any one of Claims 12 to 14, having DKKl-like activity.

23. The peptide according to any one of Claims 12 to 14, wherein the peptide has an amino acid sequence represented by formula B3:
B3: ClAc-Y1-Y2-Y3-Y4-Y5-Y6-Y7-Y8-Y9-Y10-Y11-Y12-Y13-Y14
wherein
ClAc represents a chloroacetyl group, Y1, Y2, Y3, Y4, Y5, Y6, Y7, Y8, Y9, Y10, Y11, Y12, Y13 and Y14 represent amino acid residues shown in Table 2, and in Table 2,
F represents an L-phenylalanine residue,
F4F represents an (S)-2-amino-3-(4-fluorophenyl)propanoic acid residue,
F4Me represents an (S)-2-amino-3-(p-toluyl)propanoic acid residue,
F3Me represents an (S)-2-amino-3-(m-toluyl)propanoic acid residue,
R represents an L-arginine residue,
Hcit represents an N6-carbamoyl-L-lysine residue,
W represents an L-tryptophan residue,
MeW represents a methyl-L-tryptophan residue,
W7N represents an (S)-2-amino-3-(1H-pyrrolo[2,3-b]pyridin-3-yl)propanoic acid residue,
W5N represents an (S)-2-amino- 3-(1H-pyrrolo[3,2-c]pyridin-3-yl)propanoic acid residue,
W1Me7N represents an (S)-2-amino-3-(1-methyl-1H-pyrrolo[2,3-b]pyridin-3-yl)propanoic acid residue,
W7N5Me represents an (S)-2-amino-3-(5-methyl-1H-pyrrolo[2,3-b]pyridin-3-yl)propanoic acid residue
Nal1 represents an (S)-2-amino-3-(naphthalen-1-yl)propanoic acid residue,
Nal14N represents an (S)-2-amino-3-(quinolin-4-yl)propanoic acid residue,
D represents an L-aspartic acid residue,
E represents an L-glutamic acid residue,
N represents an L-asparagine residue,
Q represents an L-glutamine residue,
V represents an L-valine residue,
MeV represents a methyl-L-valine residue,
W1aa represents a 1-(carboxymethyl)-L-tryptophan residue,
Cha represents an (S)-2-amino-3-cyclohexylpropanoic acid residue,
3Py5Ph represents an (S)-2-amino-3-(5-phenylpyridin-3-yl)propanoic acid residue,
Cbg represents an (S)-2-amino-2-cyclobutylacetic acid residue,
P represents an L-proline residue,
MeR represents a methyl-L-arginine residue,
Kmor represents an (S)-2-amino-6-morpholinohexanoic acid residue,
Cit represents an L-citrulline residue,
Ncit represents an (S)-2-amino-4-ureidobutanoic acid residue,
OrnAc represents an (S)-5-acetamido-2-aminopentanoic acid residue,
MeF represents a methyl-L-phenylalanine residue,
MeF4COO represents an (S)-4-(2-carboxy-2-(methylamino)ethyl)benzoic acid residue,
MeY represents a methyl-L-tyrosine residue,
MeF4Me represents an (S)-2-(methylamino)-3-(p-toluyl)propanoic acid residue,
MeF4C represents an (S)-3-(4-chlorophenyl)-2-(methylamino)propanoic acid residue,
MeF3COO represents an (S)-3-(2-carboxy-2-(methylamino)ethyl)benzoic acid residue,
MeF3Me represents an (S)-2-(methylamino)-3-(m-toluyl)propanoic acid residue,
MeF3C represents an (S)-3-(3-chlorophenyl)-2-(methylamino)propanoic acid residue,
S represents an L-serine residue,
Hty represents a homo-L-tyrosine residue,
alT represents an allothreonine residue,
T represents an L-threonine residue,
MeN represents a methyl-L-asparagine residue,
Hgl represents an L-2-aminoadipic acid residue,
Hw7N represents an (S)-2-amino-4-(1H-pyrrolo[2,3-b]pyridin-3-yl)butanoic acid residue,
KCOpipzaa represents an N6-(4-(carboxymethyl)piperazine-1-carbonyl)-L-lysine residue,
W6N represents an (S)-2-amino-3-(1H-pyrrolo[2,3-c]pyridin-3-yl)propanoic acid residue,
Nmm represents a (2S)-2-amino-4-(methylamino)-4-oxobutanoic acid residue,
Tic represents an (S)-1,2,3,4-tetrahydroisoquinoline-3-carboxylic acid residue,
Tic6C represents an (S)-6-chloro-1,2,3,4-tetrahydroisoquinoline-3-carboxylic acid residue,
Tic7H represents an (S)-7-hydroxy-1,2,3,4-tetrahydroisoquinoline-3-carboxylic acid residue,
Tic7C represents an (S)-7-chloro-1,2,3,4-tetrahydroisoquinoline-3-carboxylic acid residue,
Ndm represents an N4,N4-dimethyl-L-asparagine residue,
C represents an L-cysteine residue, and
G represents a glycine residue.
**[Table 2A]**
| Table 2 (1 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 79 | F | R | W | D | V | R | MeF | S | N | W | N | Tic | N | C | G |
| 80 | F4F | R | W | D | V | R | MeF | S | N | W | N | Tic | N | C | G |
| 81 | F | R | MeW | D | V | R | MeF | S | N | W | N | Tic | N | C | G |
| 82 | F | R | W | D | MeV | R | MeF | S | N | W | N | Tic | N | C | G |
| 83 | F | R | W | D | V | MeR | MeF | S | N | W | N | Tic | N | C | G |
| 84 | F | R | W | D | V | R | MeF | S | MeN | W | N | Tic | N | C | G |
| 85 | F | R | W7N | D | V | R | MeF | S | N | W | N | Tic | N | C | G |
| 86 | F | R | W | D | W1aa | R | MeF | S | N | W | N | Tic | N | C | G |
| 87 | F | R | W | D | Cha | R | MeF | S | N | W | N | Tic | N | C | G |
| 88 | F | R | W | D | Nal1 | R | MeF | S | N | W | N | Tic | N | C | G |
| 89 | F | R | W | D | 3Py5Ph | R | MeF | S | N | W | N | Tic | N | C | G |
| 90 | F | R | W | D | V | Kmor | MeF | S | N | W | N | Tic | N | C | G |
| 91 | F | R | W | D | V | R | MeF | W1aa | N | W | N | Tic | N | C | G |
| 92 | F | R | W | D | V | R | MeF | Hty | N | W | N | Tic | N | C | G |
| 93 | F | R | W | D | V | R | MeF | alT | N | W | N | Tic | N | C | G |
| 94 | F | R | W | D | V | R | MeF | S | Hgl | W | N | Tic | N | C | G |
| 95 | F | R | W | D | V | R | MeF | S | Cha | W | N | Tic | N | C | G |
| 96 | F | R | W | D | V | R | MeF | S | Hty | W | N | Tic | N | C | G |
| 97 | F | R | W | D | V | R | MeF | S | Hw7N | W | N | Tic | N | C | G |
| 98 | F | R | W | D | V | R | MeF | S | KCOpipzaa | W | N | Tic | N | C | G |
**[Table 2B]**
| Table 2 (2 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 99 | F | R | W | D | V | R | MeF | S | N | W7N | N | Tic | N | C | G |
| 100 | F | R | W | D | V | R | MeF | S | N | W | N | Tic | D | C | G |
| 101 | F4Me | R | W | D | V | R | MeF | S | N | W | N | Tic | N | C | G |
| 102 | F3Me | R | W | D | V | R | MeF | S | N | W | N | Tic | N | C | G |
| 103 | F | Hcit | W | D | V | R | MeF | S | N | W | N | Tic | N | C | G |
| 104 | F | R | W5N | D | V | R | MeF | S | N | W | N | Tic | N | C | G |
| 105 | F | R | W7N | D | V | R | MeF | S | N | W | N | Tic | N | C | G |
| 106 | F | R | W1Me7N | D | v | R | MeF | S | N | W | N | Tic | N | C | G |
| 107 | F | R | W7N5Me | D | v | R | MeF | S | N | W | N | Tic | N | C | G |
| 108 | F | R | Nal1 | D | V | R | MeF | S | N | W | N | Tic | N | C | G |
| 109 | F | R | Nal14N | D | V | R | MeF | S | N | W | N | Tic | N | C | G |
| 110 | F | R | W | E | V | R | MeF | S | N | W | N | Tic | N | C | G |
| 111 | F | R | W | N | V | R | MeF | S | N | W | N | Tic | N | C | G |
| 112 | F | R | W | Q | V | R | MeF | S | N | W | N | Tic | N | C | G |
| 113 | F | R | W | D | Cbg | R | MeF | S | N | W | N | Tic | N | C | G |
| 114 | F | R | W | D | P | R | MeF | S | N | W | N | Tic | N | C | G |
| 115 | F | R | W | D | V | Cit | MeF | S | N | W | N | Tic | N | C | G |
| 116 | F | R | W | D | V | Hcit | MeF | S | N | W | N | Tic | N | C | G |
| 117 | F | R | W | D | V | Ncit | MeF | S | N | W | N | Tic | N | C | G |
| 118 | F | R | W | D | V | OrnAc | MeF | S | N | W | N | Tic | N | C | G |
**[Table 2C]**
| Table 2 (3 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 119 | F | R | W | D | V | R | MeF4COO | S | N | W | N | Tic | N | C | G |
| 120 | F | R | W | D | V | R | MeY | S | N | W | N | Tic | N | C | G |
| 121 | F | R | W | D | V | R | MeF4Me | S | N | W | N | Tic | N | C | G |
| 122 | F | R | W | D | V | R | MeF4C | S | N | W | N | Tic | N | C | G |
| 123 | F | R | W | D | V | R | MeF3COO | S | N | W | N | Tic | N | C | G |
| 124 | F | R | W | D | V | R | MeF3Me | S | N | W | N | Tic | N | C | G |
| 125 | F | R | W | D | V | R | MeF3C | S | N | W | N | Tic | N | C | G |
| 126 | F | R | W | D | V | R | MeF | T | N | W | N | Tic | N | C | G |
| 127 | F | R | W | D | V | R | MeF | N | N | W | N | Tic | N | C | G |
| 128 | F | R | W | D | V | R | MeF | Q | N | W | N | Tic | N | C | G |
| 129 | F | R | W | D | V | R | MeF | D | N | W | N | Tic | N | C | G |
| 130 | F | R | W | D | V | R | MeF | E | N | W | N | Tic | N | C | G |
| 131 | F | R | W | D | V | R | MeF | S | Q | W | N | Tic | N | C | G |
| 132 | F | R | W | D | V | R | MeF | S | D | W | N | Tic | N | C | G |
| 133 | F | R | W | D | V | R | MeF | S | E | W | N | Tic | N | C | G |
| 134 | F | R | W | D | V | R | MeF | S | N | W6N | N | Tic | N | C | G |
| 135 | F | R | W | D | V | R | MeF | S | N | W1Me7N | N | Tic | N | C | G |
| 136 | F | R | W | D | V | R | MeF | S | N | W7N5Me | N | Tic | N | C | G |
| 137 | F | R | W | D | V | R | MeF | S | N | W | Q | Tic | N | C | G |
| 138 | F | R | W | D | V | R | MeF | S | N | W | Nmm | Tic | N | C | G |
**[Table 2D]**
| Table 2 (4 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 139 | F | R | W | D | V | R | MeF | S | N | W | N | Tic6C | N | C | G |
| 140 | F | R | W | D | V | R | MeF | S | N | W | N | Tic7H | N | C | G |
| 141 | F | R | W | D | V | R | MeF | S | N | W | N | Tic7C | N | C | G |
| 142 | F | R | W | D | V | R | MeF | S | N | W | N | Tic | Q | C | G |
| 143 | F | R | W | D | V | R | MeF | S | N | W | N | Tic | Nmm | C | G |
| 144 | F | R | W | D | V | R | MeF | S | N | W | N | Tic | Ndm | C | G |
| 145 | F | R | W7N | D | V | R | MeF | S | N | W7N | N | Tic | N | C | G |
| 146 | F | R | W7N5Me | D | v | R | MeF | S | N | W7N | N | Tic | N | C | G |
| 147 | F | R | Nal14N | D | v | R | MeF | S | N | W7N | N | Tic | N | C | G |
| 148 | F | R | W7N | D | Cbg | R | MeF | S | N | W7N | N | Tic | N | C | G |
| 149 | F | R | W7N | D | P | R | MeF | S | N | W7N | N | Tic | N | C | G |
| 150 | F | R | W7N | D | v | Kmor | MeF | S | N | W7N | N | Tic | N | C | G |
| 151 | F | R | W7N | D | V | R | MeF4COO | S | N | W7N | N | Tic | N | C | G |
| 152 | F | R | W7N | D | V | R | MeF3COO | S | N | W7N | N | Tic | N | C | G |
| 153 | F | R | W7N | D | V | R | MeF | E | N | W7N | N | Tic | N | C | G |
| 154 | F | R | W7N | D | V | R | MeF | S | E | W7N | N | Tic | N | C | G |
| 155 | F | R | W7N | D | V | R | MeF | S | N | W7N5Me | N | Tic | N | C | G |
| 156 | F | R | W7N | D | V | R | MeF | S | N | W7N | N | Tic7C | N | C | G |
| 157 | F | R | W7N | D | V | R | MeF | S | N | W7N | N | Tic | Nmm | C | G |
| 158 | F | R | W7N | D | V | R | MeF | S | N | W7N | N | Tic | D | C | G |
**[Table 2E]**
| Table 2 (5 of 5) | | | | | | | | | | | | | | | |
|---|---|---|---|---|---|---|---|---|---|---|---|---|---|---|---|
| SEQ ID NO: | Y1 | Y2 | Y3 | Y4 | Y5 | Y6 | Y7 | Y8 | Y9 | Y10 | Y11 | Y12 | Y13 | Y14 | Y15 |
| 159 | F | R | W7N | D | V | R | MeF | E | E | W7N | N | Tic | N | C | G |
| 160 | F | R | W7N | D | V | R | MeF | E | E | W7N | N | Tic | D | C | G |
| 161 | F | R | W7N | D | V | R | MeF3COO | E | E | W7N | N | Tic | D | C | G |
| 162 | F | R | W7N | D | V | Kmor | MeF3COO | E | E | W7N | N | Tic | D | C | G |
| 163 | F | R | W7N | D | V | R | MeF3COO | S | E | W7N | N | Tic | D | C | G |

24. A peptide complex comprising a first peptide and a second peptide, wherein
the first peptide is the peptide according to Claim 1, and
the second peptide is the peptide according to Claim 12.

25. The peptide complex according to Claim 24, wherein the peptide complex comprises the first peptide, the second peptide, and a linker connecting the first peptide and the second peptide.

26. The peptide complex according to Claim 25, wherein a C-terminal of the first peptide and a C-terminal of the second peptide are bonded via the linker.

27. The peptide complex according to Claim 25, wherein the linker is selected from among linkers shown in Table 3, and
in Table 3, -click- represents a bond formed by click chemistry.
[Table 3]
**Table 3**
| |
|---|
| P08K_d |
| click_P04K / c_P04K |
| click_P04K_OH |
| click_P08K |
| click_P12K |
| click_P04K_branch |
| click_P08K_branch |
| NOF |
| tag |

28. The peptide complex according to Claim 25, wherein
the first peptide is a peptide comprising an amino acid sequence represented by formula A1, and
the second peptide is a peptide comprising an amino acid sequence represented by formula B1:
A1: X1-X2-X3-X4-X5-X6-X7-X8-X9-X10-X11-X12-X13-X14
B1: Y1-Y2-Y3-Y4-Y5-Y6-Y7-Y8-Y9-Y10-Y11-Y12-Y13-Y14
wherein in formula A1:
X1 represents a D-phenylalanine (df) residue,
X2 represents an L-serine (S) residue or an L-2-aminoadipic acid (Hgl) residue,
X3 represents an L-aspartic acid (D) residue, L-citrulline (Cit) residue, or (2S)-2-amino-3-(oxan-4-yl)propanoic acid (Atp) residue,
X4 represents an L-isoleucine (I) residue,
X5 represents an L-arginine (R) residue or an L-citrulline (Cit) residue,
X6 represents an L-tyrosine (Y) residue,
X7 represents a methyl-L-phenylalanine (MeF) residue or an (S)-3-(2-carboxy-2-(methylamino)ethyl)benzoic acid (MeF3COO) residue,
X8 represents an L-glutamine (Q) residue, L-valine (V) residue, homo-L-tyrosine (Hty) residue, L-threonine (T) residue, L-citrulline (Cit) residue, or O-methyl-L-threonine (TMe) residue,
X10 represents a methyl-L-phenylalanine (MeF) residue or an (S)-2-(methylamino)-3-(pyridin-4-yl)propanoic acid (Me4Py) residue,
X11 represents an L-isoleucine (I) residue or an L-tyrosine (Y) residue,
X12 represents an L-valine (V) residue or a methyl-L-valine (MeV) residue, and
X13 represents a glycine (G) residue or a D-serine (ds) residue, and
in formula B1:
Y1 represents an L-phenylalanine (F) residue,
Y2 represents an L-arginine (R) residue,
Y3 represents an L-tryptophan (W) residue or an (S)-2-amino-3-(1H-pyrrolo[2,3-b]pyridin-3-yl)propanoic acid (W7N) residue,
Y4 represents an L-aspartic acid (D) residue,
Y5 represents an L-valine (V) residue,
Y6 represents an L-arginine (R) residue or an (S)-2-amino-6-morpholinohexanoic acid (Kmor) residue,
Y7 represents a methyl-L-phenylalanine (MeF) residue or an (S)-3-(2-carboxy-2-(methylamino)ethyl)benzoic acid (MeF3COO) residue,
Y8 represents an L-serine (S) residue or an L-glutamic acid (E) residue,
Y9 represents an L-asparagine (N) residue, N6-(4-(carboxymethyl)piperazine-1-carbonyl)-L-lysine (KCOpipzaa) residue, or L-glutamic acid (E) residue,
Y10 represents an L-tryptophan (W) residue or an (S)-2-amino-3-(1H-pyrrolo[2,3-b]pyridin-3-yl)propanoic acid (W7N) residue,
Y11 represents an L-asparagine (N) residue,
Y12 represents an (S)-1,2,3,4-tetrahydroisoquinoline-3-carboxylic acid (Tic) residue, and
Y13 represents an L-asparagine (N) residue or an L-aspartic acid (D) residue.

29. The peptide complex according to Claim 25, wherein the peptide complex activates Wnt signaling.

30. A pharmaceutical composition, comprising the peptide according to any one of Claims 1 to 3 and 12 to 14.

31. A pharmaceutical composition, comprising the peptide complex according to Claim 25.

32. A composition for cell culture, comprising the peptide complex according to Claim 25, and used for cell culture.

33. A composition for medical use, diagnostic use or research use, comprising the peptide complex according to Claim 25.

34. A composition for medical use, diagnostic use or research use, comprising the peptide according to any one of Claims 1 to 3 and 12 to 14.
